(19) Europäisches Patentamt
European Patent Office
Office européen des brevets

(11) EP 1 447 412 A1

(12) EUROPEAN PATENT APPLICATION

(43) Date of publication:
**18.08.2004 Bulletin 2004/34**

(51) Int Cl.[7]: **C07K 14/47**, G01N 27/447

(21) Application number: **03075460.0**

(22) Date of filing: **17.02.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(71) Applicant: **Universiteit Utrecht Holding B.V.**
**3584 CK Utrecht (NL)**

(72) Inventors:
- **Braakman, Lina Jantina**
  **3572 KN Utrecht (NL)**
- **van Anken, Eelco**
  **3514 EE Utrecht (NL)**

(74) Representative:
**Prins, Adrianus Willem, Mr. Ir. et al**
**Vereenigde,**
**Nieuwe Parklaan 97**
**2587 BN Den Haag (NL)**

Remarks:
The sequence listing, which is published as annex to the application documents, was filed after the date of filing. The applicant has declared that it does not include matter which goes beyond the content of the application as filed.

# (54) Method for the characterization of proteins, isolated proteins and uses therefore

(57) The invention provides a novel method of analysing the proteome of a cell. The method allows the correlation of expression of proteins with the activity of compartments in the cell and the association of thus far unidentified proteins with(in) such compartments. This gives the artisan a powerful tool in the elucidation of the function of proteins thus identified. According to this method a new family of proteins was identified and the function thereof determined and confirmed by experiments. The family termed smERp belongs to the oxidoreductase proteins, but is distinct therefrom in their activity and internal structure. The invention further identifies several other proteins and their function, together with methods wherein the identified proteins can be manipulated to obtain, or gain further functionality. The invention also provides methods wherein proteins identified in the present invention are used for the production of proteins of interest.

EP 1 447 412 A1

## Description

The invention relates to the field of biotechnology. In particular the invention relates to the identification of proteins and their function and uses of the identified proteins.

The human genome project has made an enormous amount of information available on the sequence of DNA in the cell. The project results in information on the organisation of genes and their primary structure. The knowledge about the function of the various proteins encoded by this nucleic acid does not keep the same pace. In part, this is due to the fact that proteins usually do not work alone, neither do they work all the time nor only in particular types of cells or cell organelles. The complexity with which proteins perform their function in the cell is seen as the next hurdle in the understanding of the biology of the cell. The characterization of proteins and their function in a cell is often referred to as the field of Proteomics, whereas the set of proteins expressed by a cell is referred to as the proteome. Many methods have been devised to gain access to the proteome of a cell. The challenge lies amongst others in dealing with information on a large number of different proteins in a limited time span and with limited resources. Many approaches are based on separation of the proteins of a cell in one or two dimensions and studying differences in the patterns thus generated between different types of cells. In the present invention a new way to characterize proteins in a cell has been developed. This method offers a new way of looking at proteins and their expression in a cell and through this new perspective opens a route to the identification proteins in a cell and their function. In one aspect the invention provides a method for identifying a protein from a collection of proteins

- providing at least two protein samples wherein said protein samples are derived from related cells that differ in their activation or differentiation state,
- subjecting each of said samples to a protein separation step,
- selecting a protein that displays an altered relative abundance and/or migration between said samples in said separation step,
- and identifying said protein. The protein can be identified by MALDI peptide mass mapping and peptide sequencing through for instance nanoES tandem mass spectrometry. Many alternative methods for the identification of proteins or parts thereof are, however, known in the art. The samples are derived from cells. The samples typically comprise all proteins of the cells. However, the sample may also contain certain fractions of the cells, such as a nuclear fraction or a fraction containing predominantly endoplasmic reticulum (ER) or mitochondria. It is important that samples contain the same derivative of the cells, so that for instance endoplasmic reticulum proteins in one sample are compared with endoplasmic reticulum proteins in another sample. By comparing the same type of samples from related cells it is possible to observe alterations in the relative abundance and/or migration of proteins between the samples, and coupling these alterations to the differences in activation and/or differentiation state of the cells of which the samples where derived. Through this coupling it is possible to associate proteins with an altered abundance and or migration with the differences between the related cells and thereby obtain information on cellular processes such proteins are involved in. There are several ways of looking at these differences. By studying differences between two activation states or two differentiation states one may already obtain much information. Thus a method preferably further comprises the clustering of signals of proteins on the basis of similar differences in the relative abundance and/or migration between said samples. Proteins of such a cluster are expected to be involved in the difference in activation/differentiation states of the cells in the samples. The full force of the method of the invention comes from cells that are progressing from one state to another, and comparing samples that are taken at different stages of activation/differentiation. This is easy to achieve when, for instance, samples are taken at various times during the progression from one activation/differentiation state to the next. The relationship between the cells of the different samples is thus preferably as direct as possible. A collection of cells is divided into several groups, wherein subsequently the various groups are treated differently to allow the cells time to respond and allow differences in the abundance or the migration to become apparent between the groups. Information on the relative abundance and/or migration of proteins in a separation step in this setting allows a direct correlation with the state of the groups of cells. In this approach it is possible to identify clusters of proteins that have responded in a similar way to the different state. When several time points are analysed during the transition from one activation/differentiation state to another it is possible to identify clusters that (dis)appear early and cluster that (dis) appear later in time. By correlating these clusters with activities and compartmental location in the cell it is possible to correlate identified proteins with a function in the correlated compartment. Correlations can be made in many different ways. A preferred way is to identify known proteins in the cluster. Knowledge of the compartmentalization of the known protein can subsequently be used as an indication for the compartmentalization of an identified as yet unknown protein. The identification of two or more known proteins in a cluster and the correlation of them to the same compartment of course strengthens the indication even further. Thus a method of the invention preferably further comprises correlating said clusters with an activity of a compartment of a cell. Preferred compartments entail the endoplasmic reticulum with or without the nuclear envelope, the nuclear envelope, the cytosol, the Golgi complex, and/or peroxisomes, however, the nucleus and the mitochondria are other compartments of interest.

As mentioned above, the cells of the different samples should be related to each other. The term "related" means that one sample contains a first collection of cells whereas said one or more other samples contain collections of cells that are directly derived thereof. This can be achieved by splitting a culture of cells into several fractions and producing a first sample from one fraction, while the other fraction or fractions are allowed to reach a different activation/differentiation state prior to generating a sample from these fractions. There are of course also other ways in which to generate fractions of related cells. Important is that the cells share the same origin prior to inducing a different activation/differentiation state. Cells of the hemopoietic system are preferred in the present invention since these cells can be easily purified (stem cells) and differentiated in vitro. Moreover many hemopoietic cells exhibit various specific and clearly identified activation states. Activation states are often also referred to as maturation states. Though the hemopoietic cell lineage is preferred, other cell lineages can also be used, for instance colon cells, thyrocytes, skin cells etc all sharing the feature that primitive cells form more differentiated specialized cells. In a particularly preferred embodiment the cells are derived from the B-cell lineage.

A key question in cell biology is how cells control the equilibrium of the various organelles to maintain their shape. Even more challenging is how they adjust their architecture to comply with new functions. A spectacular example of such a transformation is the differentiation of B-lymphocytes to plasma cells. B-lymphocytes express immunoglobulins (Ig) on their surface as clonal antigen receptors but do not secrete antibodies. These cells have a small cytoplasm, with scarce endoplasmic reticulum (ER) cisternae. Upon binding of specific antigen to the B cell receptor, or by stimulation with mitogens, B cells proliferate and differentiate into plasma cells, each of which secretes thousands of antibodies per second. In IgM secreting plasma cells the components of secreted polymers are the secretory form of μ heavy chains (μs), light (λ) and J chains (Reddy and Corley, 1999). This massive Ig production correlates with a highly developed secretory machinery, most being ER (Wiest et al., 1990), like in other professional secretory cells. Many resident ER proteins have been implicated in the folding and assembly of antibody: BiP (Haas and Wabl, 1983) and GRP94 sequentially associate with Ig heavy chains (Melnick et al., 1994), GRP170 was found to bind Ig (Lin et al., 1993), and PDI, ERp72 and many other as yet unidentified microsomal oxidoreductases interact with IgM subunits in a thiol-mediated fashion (Reddy et al., 1996).

Considering the interest in B-lymphocyte differentiation much of the present invention is illustrated using this system. Although these cells are preferred in the invention, the invention is not limited to these types of cells. As a model for the present invention we set out to investigate how B-lymphocytes reorganize their machineries to become cells that are professional secretors. How does the transformation from dormant B-lymphocytes to plasma cells unfold? This question has received little attention. Argon and co-workers followed B cell differentiation by electron microscopy and immunoblotting of some resident ER proteins (Wiest et al., 1990). Two-dimensional electrophoretic analysis of differentiating B cells was performed as early as 1984, but at that time protein identities were not determined (Kettman and Lefkovits, 1984). Recently, Frey et al. made an initial characterization of the proteome of splenocytes, as representatives for B-lymphocytes, but limited their analysis to fully activated cells (Frey et al., 2000).

To follow the differentiation process with time, we chose a dynamic proteomics approach. Using this strategy we could follow overall changes in protein expression during the reorganization of cellular machineries triggered by the activation of a B-lymphocyte. How are Ig secretion and ER development related? At least two possibilities can be envisaged. First, an increase in the synthesis of secretory proteins (Igs) could drive the expansion of the ER via signalling cascades programmed to enhance folding capacity and limit ER-load. These regulatory pathways are referred to as the unfolded protein response (UPR) (Patil and Walter, 2001). Alternatively, the development of an efficient protein factory could precede the actual production and release of antibodies.

We demonstrate that sequential waves of proteins are coordinately expressed during the transformation of B cells into plasma cells. At the first day of activation, mitochondrial and cytosolic chaperones showed highest expression, whereas metabolic enzymes peaked at the third day. ER resident proteins linearly increased during differentiation, accompanied by proteins involved in the redox balance. We witnessed a sharp increase in IgM synthesis only after two days of activation. We conclude that the transformation from dormant B cells to secretory plasma cells is a multistep process. Upon activation, B cells carefully prepare for their role as plasma cells well before IgM secretion starts. First the cell ensures that metabolic capacity and the secretory machinery have expanded enough to accommodate the launch of bulk IgM production.

In identifying proteins that exhibit differences in relative abundance and/or migration one obtains information on the sequence of at least part of the protein. Using this sequence it is possible to screen nucleic acid databases for identical sequences. If it is a known protein the information about the protein can be used to type clusters as mentioned. If the sequence is present in databases but no function has been assigned to the protein (part), the typing of the cluster gives an indication of the role of the protein in the cell. Considering that much of the human genome and the mouse genome has been sequenced it is usually possible to obtain the sequence of the entire coding region of the protein. This coding region can be cloned and the protein produced and purified using methods available to the person skilled in the art. The invention thus further comprises isolating the identified protein and a recombinant protein obtainable by a method of the invention. Since compartmentalization in the cell is an important criterion in the invention, the protein preferably comprises a signal for translocation into a cellular compartment. Preferably, said compartment comprises endoplasmic reticulum with or without the nuclear envelope, the nuclear envelope, the cytosol, the Golgi complex, and/or peroxisomes, however, the nucleus and the mitochondria are other compartments of interest.

The endoplasmic reticulum is a compartment of special interest in the present invention as the proteins in this compartment were shown to be particularly well clustered in time. The method allowed the identification and assignment of function of a number of previously not recognized proteins and proteins with an unknown function. Through the method of the invention it is possible to find proteins that are only conditionally expressed in the collection of cells investigated. Examples of genes identified in the present invention as having an endoplasmic reticulum localization are ERp43, CRELP, CREMP, smERp1, smERp2, smERp3 and Quiescin comprising an amino acid sequence as depicted in figure 1. The invention thus provides an amino acid sequence encoding an ERp43, a CRELP, a CREMP, a smERp1, a smERp2, a smERp3 or a Quiescin protein or functional part, derivative and/or analogue thereof. A functional part, derivative and/or analogue of a said sequence encodes a protein with the same activity or specificity in kind, not necessarily in amount. A derivative of such a protein for instance may be obtained through conservative amino acid substitution. An analogue of such a sequence is a sequence derived from a different species having the same endoplasmic reticulum localization. A preferred example of such an analogue is a human analogue of the proteins having a sequence as depicted in figure 1. The invention further provides a nucleic acid encoding a sequence as depicted in figure 1 or a functional part, derivative and/or analogue thereof. Derivative may comprise nucleic acid modifications for instance such as used to stabilize derived nucleic acid in the blood stream. However, other modifications are also envisioned and within the scope of the invention. Analogues comprise for instance PNA and other variants having the same hybridisation specificity but not necessarily the same hybridisation strength.

The ER is one of the compartments in eukaryotic cells were proteins fold. The proteins that use the ER as a folding compartment are those that reside in the secretory pathway, on the plasma membrane, the nuclear envelope, or proteins that get secreted. To enter the ER, proteins need a specific targeting signal. The targeting signal for the ER, the signal sequence, is a hydrophobic stretch of amino acids at the N-terminus of a protein (Heijne 1986; Zheng and Nicchitta 1999). After translocation of the protein into the ER has begun, this signal sequence is often cleaved off by the membrane bound signal peptidase (Martoglio and Dobberstein 1998).

A feature special for folding in the ER is the possible addition of N-glycans to newly synthesized proteins. These N-glycans are transferred from dolichol phosphate carriers to the folding proteins by oligosaccharyl transferases. Different from other compartments where proteins fold, such as the cytosol or mitochondria, folding in the ER is often accompanied by disulfide bond formation. Disulfide bonds can be important for the structure and function of these proteins (Gething and Sambrook 1992; Raina and Missiakas 1997) or function as checkpoints during the folding process. The oxidizing environment of the ER is reflected by the relatively high concentration of oxidized gluthathione (GSSG). In the cytosol the ratio GSH:GSSG varies between 30 and 100, whereas in the lumen of the ER this ratio is approximately 3 (Hwang et al. 1992).

The ER contains chaperone molecules and folding enzymes. The ER resident proteins may have a C-terminal retention signal that prevents their secretion (Munro and Pelham 1987). The tetrapeptide KDEL (or another tetrapeptide with similar characteristics) binds to a receptor localized in the Golgi apparatus (Scheel and Pelham 1996). When a KDEL containing protein binds to this receptor, the complex travels back to the ER. Here, the KDEL receptor releases its substrate because of the higher pH in the ER. After dissociation, the receptor is transported to the Golgi (Wilson et al. 1993).

The ER resident proteins assist the growing polypeptide chains that enter the ER to reach their mature state (Gething and Sambrook 1992), and function in the quality control of the folding proteins. Only when a protein is folded correctly, it can leave the ER and travel further along the secretory pathway; unfolded or misfolded proteins are retained and eventually degraded (Ellgaard and Helenius 2001).

Chaperones such as BiP and GRP94 bind to newly translated proteins and protect exposed hydrophobic stretches during folding (Flynn et al. 1991; Melnick et al. 1994). Disulfide bonds are introduced and isomerized by oxidoreductases such as PDI and ERp57 (Molinari and Helenius 1999). Other members of the PDI family, such as ERp72 (Mazzarella et al. 1990) and P5 (Kikuchi et al. 2002) seem to have a similar role. These proteins have a common active site, CXXC, within a domain homologous to thioredoxin. At least one of the X amino acids in the motif usually is a basic amino acid. When the active site cysteines of PDI are in the oxidized form (disulfide bonded), this disulfide bond can be transferred to substrate proteins (Lyles and Gilbert 1991). PDI has to be reoxidized to regain oxidase activity. A candidate recharger of PDI is Ero1, since in yeast, it has been shown that this protein is required to keep PDI oxidized and functional (Frand and Kaiser 1999). Whether the human homologue, Ero1-L$\alpha$, has a similar function awaits confirmation (Cabibbo et al. 2000).

Most of the chaperones and folding enzymes have broad substrate specificity. However, some secretory proteins need the assistance of specialized chaperones to fold correctly. Hsp47, for example, is a collagen specific chaperone (Nagata and Hosokawa 1996) that functions early in the secretory pathway (Tasab et al. 2002). Another substrate specific chaperone is Receptor-Associated protein (RAP), which binds preferentially to unfolded endocytic receptors of the LDL receptor gene family (Willnow 1998). However, RAP might also bind to other proteins (Sarti et al. 2000).

Most chaperones and folding enzymes are expressed in all tissues. However, a tissue-specific homologue to PDI has been described. PDIp is a pancreas specific protein with significant sequence similarity to PDI (Desilva et al. 1996), as well as functional similarity (Elliott et al. 1998; Volkmer et al. 1997).

In the present invention a new class of ER resident proteins (ERps) was identified. The class was identified in differentiating B-cells. During B-cell differentiation, the secretory machinery, including the ER, expands. By a dynamic proteomics approach, we observed linear upregulation of the ERps as expected. Unknown proteins that showed similar expression profiles (within the same cluster) during B-cell differentiation are candidate ERps. One of the candidate ERps that we identified in the B-cell screening was an 18.3 kDa protein, further referred to as smERp1 (small ER resident protein 1). The sequence of smERp1 shows hallmarks of ERps. Here we show that smERp1 is indeed an ER resident protein with a novel CXXC motif. The CXXC motif in smERp 1 comprises at least one acidic amino acid. smERp 1 is not alone in this. We have identified at least two other proteins with a similar motif, i.e. smERp2 and smERp3. The different motif is coupled with a different activity or specificity of the class of proteins thus identified. The proteins appear to able to break disulfide bonds in proteins and thereby allow them more time, or a renewed chance to assume a correctly folded state. The invention thus provides a recombinant protein comprising oxidoreductase activity comprising a $CX_1X_2C$ motif, wherein C comprises a cysteine and wherein $X_1$ or $X_2$ comprises an acidic amino acid. The special activity of the smERp proteins can be transferred to other proteins comprising a CXXC motif, by changing at least one of the X amino acids (either X1 or X2) into an acidic amino acid. Preferably, the basic amino acid in the motif is changed into an acidic amino acid. In one embodiment the acidic amino acid is an aspartic acid or a derivative thereof. Since these proteins normally have their activity in the ER it is preferred that they comprise a signal sequence for translocation to the endoplasmic reticulum. smERp1 is an abundant protein in the proteome of activated B cells with an apparent MW of 18 kDa and a pI of 5.4 (Fig 2). smERp1 is absent in non-activated B-lymphocytes, but is upregulated in the course of differentiation (Fig. 3) The signal of this protein was abundant at day 3 and 4 of activation. It accounted for 2% of the total signal in the gel.

The protein spot was excized from gels. After in-gel tryptic digestion, peptides were analysed by MALDI peptide mass mapping and peptide sequences were determined by nanoES tandem MS. The identity of the spot corresponded to a putative ORF in the cDNA with NCBI number AK008016. By comparison of all available ESTs in the NCBI databases, corresponding to AK008016, we determined the consensus sequence of the ORF. The consensus sequence differed from AK008016 at three net. positions: A33C, A92C and G407A, resulting in residue changes L11F, H31P and S136N.

We obtained a cDNA clone from a library. Sequence analysis of this clone confirmed the previous analysis. The ORF contains an ATG codon within a proper initiation context and an in-frame termination codon, predicting a protein of 188 residues. The PSORT algorithm (Heijne, 1986) predicted that the hydrophobic N-terminus of the 188 residue protein could serve as a cleavable ER-targeting signal, with a cleavage site between G21 and D22 (Fig. 4) Evidence for this cleavage came from the sequence of peptide D22-K38, that was apparently generated by signal peptidase and trypsin. Depending on conditions and cell type, signal peptide cleavage may occur a number of residues upstream or downstream. After cleavage of the precursor protein, a MW of 18.3 kDa is predicted for the mature 167 residue protein, corresponding to the apparent MW deduced from gel electrophoresis. A functional part of smERp1 therefore preferably comprises at least part of the mature protein. By peptide sequencing we reached a coverage of 96% for the protein, excized from gel (Fig. 4). The sequence was identical to the consensus sequence, as determined from EST databases.

The protein contained a CXXC motif (Fig 4.), albeit different from the canonical CXXC, found in known ER resident oxidoreductases, such as PDI: CDAC instead of CGHC. No other obvious sequence homology was found with PDI-like proteins. At the C-terminus, the protein contains the tetrapeptide REEL (Fig. 4) shown previously to mediate ER localization (Munro and Pelham, 1987).

In silico BLAST searches of the working draft of the murine genome (Ensembl) revealed that smERp1 is encoded within four exons on the B3 region of chromosome 18. The first exon encodes the leader peptide and the CXXC motif. The last codon encodes the C-terminus of smERp 1 and the 3' UTR, with a potential polyadenylation signal AATAAA at a position ~250 net downstream of the TAG termination codon Fig. 5).

smERp1 has a human homolog: "Similar to RIKEN cDNA 2010001M09 gene" with GenBank accesion number BC021275 (77% identity). The human homologue is thus an analogue of smERp1. At the cDNA level this ORF is identical to proapoptotic caspase adaptor protein (PACAP) with accesion number AF338109 (Bonfoco et al., 2001), except that the PACAP cDNA sequence contained a 14 nct insertion 178 net downstream of the ATG, resulting in a frameshift. The human smERp1 gene could not be traced in the working draft of the human genome as yet. From all available ESTs at the NCBI databases we could deduce a chicken homolog of smERp1 (48% identity) Alignments are shown in fig 6. The only other ESTs showing high homology to smERp1 were from rat and cow, suggesting smERp1 is exclusive for mammals and avians.

No sequence homology with any known family of proteins could be deduced from database searches. A few other proteins of unknown function, however, appeared similar to smERp1. Thus, smERp1 is a member of a larger smERp family of proteins. The smERp1 ORF showed 23% homology with another murine protein, "putative secreted protein ZSIG9", with GenBank accession number AF186115. The alignment is shown in fig. 7. This protein shared relevant characteristics with smERp1, having a predicted cleavable leader peptide, a CGAC potential redox-active motif and a putative ER retention signal HDEL. In analogy to smERp1 we named this protein smERp2 Fig. 8). The human homolog of smERp2 (98% identity) was annotated as "transmembrane protein 4", a putative type II membrane protein, with GenBank accesion number AB015631. smERp2 homologs are also present in Drosphila melanogaster (26%) and Caenorhabditis elegans (35%). Alignment of the smERp2 family is shown in Fig. 9. Yet another putative murine protein with GenBank accession number AK005532, which showed 22% homology with smERp2, we named smERp3 (Fig. 10). Again, smERp3 contained a predicted N-terminal ER targeting signal, a potential redox-active CEVC motif and a putative ER retention signal PDEL at its C-terminus (Fig. 11). The human homolog of smERp3 (90% identity) with GenBank accession number AL035587 was previously annotated as dJ475N16.1 (CTG4A). Other homologs of smERp3 were found in Drosphila melanogaster (35% identity) and in Caenorhabditis elegans (27% identity). Alignments are shown in fig. 12. Also in Arabidopsis thaliana a smERp homolog was found, but sequence identity with the different smERp subfamilies was about equally low (Fig. 13).

Overall similarities among the smERps are evident: They all contain a signal peptide, a potential redox-active CXXC motif, 4 other conserved cysteines and a putative ER retention signal. Three domains with highest homology are indicated (Fig. 14). Initial biochemical characterization shows that smERp1 functions as a reductase (i.e. capable of disrupting disulfide bonds); This could be confirmed for the family of smERps here identified. As for smERp1, the homologues of smERp2 and smERp3 in other species are at least analogues of the identified smERp proteins.

The various homologues give an indication to the person skilled in the art where to introduce changes into the coding region so as not to affect the oxidoreductase activity of the protein and what kind of substitutions, mutations etc. will be tolerated by the protein without affecting the kind of activity or specificity, although the amount of activity might be altered. Proteins resulting from such procedures are also within the scope of the invention adenovirus are at least functional derivatives of smERp12 or 3 as identified in the present invention.

Another protein identified in the present invention comprises ERp43. One spot, showing similar expression kinetics as known ER resident proteins (Fig. 15) had an apparent MW of 43 kDa and pI of 5.2 (Fig. 16). It was excised and analysed by MALDI peptide mass mapping after in-gel tryptic digestion. The identity of this spot corresponded to a murine protein referred to at NCBI as: "Similar to hypothetical protein MGC3178", with GenBank accession number BC016252. The peptide mass mapping covered 62 % of the putative protein sequence. The sequence carried hallmarks of ER resident proteins, being a C-terminal tetrapeptide KDEL that mediates ER residency (Munro and Pelham, 1987), and two CGHC redox active sites, similar as in PDI. However, the predicted MW of 36 kDa did not correspond to the apparent MW of 43 kDa we found and, importantly, an N-terminal signal sequence needed for targeting to the ER was absent (Heijne, 1986). Re-examining the cDNA sequence, containing the ORF encoding "Similar to hypothetical protein MGC3178", we found an alternative ATG codon within a proper initiation context 165 net upstream of the predicted initiator ATG. The resulting ORF did contain a hydrophobic N-terminal signal sequence as predicted by the PSORT algorithm, that would be cleaved between residue Ala34 and Gln35 of the precursor protein. After cleavage, a MW of 43 kDa is predicted, which does correspond to the apparent MW. One peptide had a mass that could only be explained by the presence of the N-terminal extension, giving evidence for its existence. Depending on conditions and cell type, signal peptide cleavage may occur a number of residues upstream or downstream. In addition, a third CGHC redox-active site was present in this N-terminal extension.

To determine whether the extended ORF was the consensus sequence for the protein we found in our gels, we compared all available sequences in the NCBI mouse EST database. This alignment revealed two point mutations or sequence errors in the cDNA of the extended ORF of "Similar to hypothetical protein MGC3178": T555C and A882T, resulting in residue changes L171P and, respectively, Q280L. For both substitutions we found mass spectrometrical evidence, reaching a coverage for the protein without its signal sequence of 78% (Fig. 17). We obtained a cDNA clone from a library. The sequence was identical to the consensus sequence we derived from EST databases. We refer to the extended and corrected ORF of the protein as ERp43.

ERp43 is an ER protein. Both human and murine ESTs primarily originated from secretory tissues such as the liver, the salivary gland, etc. ERp43 homologs are present in Drosophila and man. EST databases reveal that only chordata and arthropods contain ERp43 homologs. The human homolog missed the N-terminus, similarly to murine ERp43. Therefore we reassembled a full length ORF as described above. Also, from ESTs we could reassemble the Xenopus ERp43. Alignments of ERp43 are given in (Fig. 18).

The architecture of ERp43 is remarkably conserved. ERp43 shows high sequence identity within, since it contains three similar domains, homologous to thioredoxin (trx). Computer modelling reveals that all three trx domains show remarkable structural similarities with the a-domain of PDI, including the putative redox-active CGHC motifs (Kemmink J, 1996). We therefore classified ERp43 as a member of the PDI-like family of ER resident oxidoreductases, involved in disulfide bond formation and/or isomerization.

In Silico BLAST searches of the working draft sequence of the murine genome allowed the mapping of ERp43 on chromosome 6. The coding sequence is encoded within ten exons (Fig. 19). Exon 1, 2 and 3 encode the signal sequence and the first trx domain; exon 4, 5, 6 and 7 encode the second trx domain; exon 8, 9 and 10 encode the third trx domain. Exon 10 encodes in addition the 3' UTR including potential polyadenylation sequences. The presence of three ERSEI sequences (ATF6 target (Yoshida H, 2001)) and a UPRE (XBP-1 target (Yoshida H, 2001)) upstream of the initiator ATG, indicate that ERp43 is a UPR target (Fig. 20).

The three trx domains of ERp43 showed high homology with each other. The closest relative of ERp43 in both the murine and human genome appeared ERp57, more specifically the first trx domain showed homology with all three ERp43 trx domains (Fig. 21). From alignments of all PDI a-domain-like trx domains it became evident that the three ERp43 trx domains and the first trx domain of ERp57 as well as the first trx domain of both PDIp and Eug1p from yeast, all shared an additional set of cysteines downstream of the CGHC redox-active site. Interspaced by six residues and in some relatives eight residues (drosophila ERp43 third trx, as well as mpd1 and 2 in yeast), these two cysteines have been conserved in distant relatives within the PDI family from yeast to man. This motif, hereafter referred to as the C(N6)C motif, was lacking from mammalian PDI itself, P5, PDIp and ERp72. Most often in place of cysteines, serines or alanine residues were found in their sequences, nicely illustrating that nature chooses similar substitutions as many investigators do. Interestingly, ERp44, which has a CXXS active site as opposed to the canonical CXXC (Anelli T, 2002) also contained a single cysteine in the other motif: C(N6)A. Schematic representations of a few PDI family members containing the C(N6)C motif or not are given (Fig. 22). Modelling of the cysteines of the C(N6)C motif into the NMR structure of the a-domain of PDI, showed that they are close enough to form a disulfide. This disulfide would close a small loop that is less conserved but it joins sequences that are well conserved amongst the PDI a-domain-like trx folds. The C(N6)C motif is juxtaposed to the redoxactive CGHC module. (Fig. 23). The conservation of the C(N6)C together with its juxtaposition to the redox-active CXXC motif, suggests that also the C(N6)C motif has a role in oxidative folding in eukaryotes. The variations in the C(N6)C motif between the various proteins and the predicted close proximity of the C(N6)C motif to the CXXC motif demonstrates that the C(N6)C motif influences the activity of oxidoreductase having both motifs. The invention thus further provides a protein comprising oxidoreductase activity having a CXXC motif and a C(N6)C motif wherein at least one N amino acid is altered such that the oxidoreductase activity is altered in amount, not necessarily in kind. The C(N6)C motif is preferably present in a region homologous to a thioredoxin sequence or fold. The C(N6)C motif modulates, strengthens, weakens, or changes specificity of the enzymatic activity of the protein it is part of, either by acting together with the $CX_1X_2C$ motif, or alone.

The proteins of the invention may be used to at least facilitate the folding of proteins in a cell and thus aid to the efficiency of the folding machinery in the cell. The proteins may also be used to facilitate the production of a protein of interest in a cell. Many different proteins are currently produced in cells for commercial reasons, such as but not limited to therapeutic purposes. Vaccines are also produced in cells as are proteins for use in or as cosmetics and cleaning materials. The proteins of the invention may favourably be used to increase the proportion of correctly folded produced protein. Where for excretion a certain "correct" folding of the protein of interest is required, such as is the case with for instance excretion of HIV gp120, the total amount of excreted protein of interest can be increased by providing the producer cell with a protein of the invention. Thus the invention further provides a method for producing a protein of interest in a cell comprising ever-expressing a protein of the invention in said cell and producing said protein of interest in said cell. In a preferred embodiment a protein of the invention comprises a protein having oxidoreductase activity comprising a $CX_1X_2C$ motif, wherein C comprises a cysteine and wherein $X_1$ or $X_2$ comprises an acidic amino acid, and/or a C(N6)C motif, wherein N comprises any amino acid. Preferably said oxidoreductase comprises a smERp protein of the invention, preferably smERp1. It is preferred that the protein of interest comprises at least one cysteïne.

The protein of interest may be any type of protein, however, preferably said protein of interest comprises a secreted protein or membrane protein, as these are translated in combination with the ER. Antibodies or functional part, derivative and/or analogue thereof such as fab fragments, single chain antibodies have gained much attention in recent years. There are several reasons for this attention, one reason is that at least some antibodies have proven their therapeutic effect in clinical settings. However, antibodies and their functional part, derivative and/or analogue thereof are produced for a large variety of reasons. Antibodies are often not easily produced by cells. The folding of the protein, particularly in the variable, antigen binding part, is different for each different antibody. This results in a difficult production of at least some antibodies or part, derivative and/or analogue thereof. With a method of the invention it is possible to at least in part improve the production of such difficult antibodies or part, derivative and/or analogue thereof, particularly when the folding of the antibody is difficult or otherwise time consuming. The present invention allows improvement of the amount of correctly folded antibody or functional part, derivative and/or analogue thereof, particularly when the antibody or functional part, derivative and/or analogue thereof is difficult to produce due to folding problems in the ER. A method of the present invention also allows more of the antibody or functional part, derivative and/or analogue thereof to be produced in a particular time frame, particularly when the antibody or functional part, derivative and/or analogue thereof is difficult to produce due to folding problems in the ER. Thus in a preferred embodiment the protein of interest comprises an antibody or a functional part, derivative and/or analogue thereof.

In another embodiment the protein of interest comprises a vaccine protein, such as an envelope or spike protein of a virus.

Many types of cells are used for the production of proteins. The cell of the invention is an ER containing cell, preferably, a mammalian cell. Preferably a cell suitable for large scale production of a protein. A special type of cells that are used for the production of proteins are adenovirus early region 1 containing cells. These cells are amongst others used for the production of adenovirus vectors, adeno-associated virus vectors and chimers between the two. Thus a protein of the invention may also be used for the production of a virus vector. A further characteristic of an adenovirus E1 region containing cell is that these cells are capable of producing a large amount of foreign proteins. Thus preferably, a cell in a method of the invention comprises an adenovirus early region 1. Examples of such cells are 293 cells. The invention thus also provides an isolated or recombinant cell according to the invention comprising an adenovirus early region 1.

Within the scope of the present invention is also an expression vector comprising a gene of interest and a protein of the invention. The invention further provides an isolated or recombinant cell provided with a nucleic acid encoding a protein of the invention or a cell provided with a protein according to the invention. Preferably, a protein of the invention comprises an oxidoreductase protein.

Examples

Example 1

A key question in cell biology is how cells control the equilibrium of the various organelles to maintain their shape. Even more challenging is how they adjust their architecture to comply with new functions. A spectacular example of such a transformation is the differentiation of B-lymphocytes to plasma cells. B-lymphocytes express immunoglobulins (Ig) on their surface as clonal antigen receptors but do not secrete antibodies. These cells have a small cytoplasm, with scarce endoplasmic reticulum (ER) cisternae. Upon binding of specific antigen to the B cell receptor, or by stimulation with mitogens, B cells proliferate and differentiate into plasma cells, each of which secretes thousands of antibodies per second. In IgM secreting plasma cells the components of secreted polymers are the secretory form of μ heavy chains (μs), light (1) and J chains (Reddy and Corley, 1999). This massive Ig production correlates with a highly developed secretory machinery, most being ER (Wiest et al., 1990), like in other professional secretory cells. Many resident ER proteins have been implicated in the folding and assembly of antibody: BiP (Haas and Wabl, 1983) and GRP94 sequentially associate with Ig heavy chains (Melnick et al., 1994), GRP170 was found to bind Ig (Lin et al., 1993), and PDI, ERp72 and many other as yet unidentified microsomal oxidoreductases interact with IgM subunits in a thiol-mediated fashion (Reddy et al., 1996).

We set out to investigate how B- lymphocytes reorganize their machineries to become cells that are professional secretors. How does the transformation from dormant B-lymphocytes to plasma cells unfold? This question has received little attention. Argon and co-workers followed B cell differentiation by electron microscopy and immunoblotting of some resident ER proteins (Wiest et al., 1990). Two-dimensional electrophoretic analysis of differentiating B cells was performed as early as 1984, but at that time protein identities were not determined (Kettman and Lefkovits, 1984). Recently, Frey et al. made an initial characterization of the proteome of splenocytes, as representatives for B-lymphocytes, but limited their analysis to activated cells (Frey et al., 2000).

To follow the differentiation process with time, we chose a dynamic proteomics approach. Using this strategy we could follow overall changes in protein expression during the reorganization of cellular machineries triggered by the activation of a B-lymphocyte. How are Ig secretion and ER development related? At least two possibilities can be envisaged. First, an increase in the synthesis of secretory proteins (Igs) could drive the expansion of the ER via signaling cascades programmed to enhance folding capacity and limit ER-load. These regulatory pathways are referred to as the unfolded protein response (UPR) (Patil and Walter, 2001). Alternatively, the development of an efficient protein factory could precede the actual production and release of antibodies.

Here we report that sequential waves of proteins are coordinately expressed during the transformation of B cells into plasma cells. At the first day of activation, mitochondrial and cytosolic chaperones showed highest expression, whereas metabolic enzymes peaked at the third day. ER resident proteins linearly increased during differentiation, accompanied by proteins involved in the redox balance. We witnessed a sharp increase in IgM synthesis only after two days of activation. We conclude that the transformation from dormant B cells to secretory plasma cells is a multistep process. Upon activation, B cells carefully prepare for their role as plasma cells well before IgM secretion starts. First the cell ensures that metabolic capacity and the secretory machinery have expanded enough to accommodate the launch of bulk IgM production.

Results

**LPS induces Ig secretion and morphological changes in I.29μ+ lymphoma cells**

As a model of B-lymphocyte differentiation we chose the murine B cell lymphoma I.29μ+, which expresses IgM on the surface but does not secrete antibodies. These cells can be induced by lipopolysaccharide (LPS) to secrete IgM (Stavnezer et al., 1985; Alberini et al., 1987). To confirm their validity as a model of plasma cell differentiation, I. 29μ+ cells were treated for 0, 1, 2, 3 or 4 days with LPS. IgM secretion was barely detectable before LPS treatment, but increased for four consecutive days (Figure 24A, lower panel). In addition to secretion, the total amount of intracellular IgM increased upon activation (Figure 24A, upper panel). Ig secretion correlated with morphological changes that characterize plasma cell differentiation: larger cells displaying an intense intracellular staining by anti-IgM antiserum appeared (Figure 24B), becoming more frequent with time. Concomitantly, the average protein content per cell increased (Figure 24C). Flow cytometry confirmed that most cells increased in size, although not uniformly (Figure 24D). Dividing cells became less frequent as differentiation proceeded (Giemsa and Hoechst 33342 staining, not shown), and cell death started in the last two days of induction (data not shown), which could explain why average protein content started to decrease at day 4.

To further characterize LPS induced I.29μ+ differentiation, we followed the expression of CD138 (syndecan-1) (Figure 24E, left panel). While only few unstimulated I.29μ+ cells expressed this marker of mature plasma cells (Chilosi et al., 1999), the vast majority of them became positive within day 3 of LPS stimulation. In contrast, expression of CD45 (B220) did not change significantly, possibly because we activated with mitogen instead of antigen (Figure 24 E, right panel). The complete set of quantifications of flow cytometry and immunofluorescence experiments is provided on a webpage (http://vrc.bijvoet-center.nl/bocl/b_cells). Taken together, these findings confirm that LPS-induced I.29μ+ cells represent an appropriate model to investigate B-plasma cell differentiation in molecular terms (Alberini et al., 1987; Sitia et al., 1985; Stavnezer et al., 1985).

**Drastic changes of the proteome upon B cell differentiation**

To follow changes in protein expression during B cell differentiation, we analyzed post-nuclear supernatants from detergent lysates of the I.29μ+ cells before or after activation. To obtain comparable staining intensities, equal amounts of proteins were separated by two-dimensional (2D) gel electrophoresis and visualized either by silver staining or SYPRO Ruby staining. Spot signals were measured by densitometric scanning or fluorescence imaging, respectively. Matching and quantification of spots were performed with the BioRad PDQuest software package. Overall changes in the proteome during B cell differentiation were evident and reproducible, but the pace of the process varied slightly from one experiment to the other. We therefore used a representative experiment for thorough image analysis (Figure 25).

We compared expression kinetics of the ~400 most abundant spots. Because so many protein spots showed strikingly similar expression kinetics, we clustered 75% of detected protein spots in only five distinct synexpression groups. Corresponding to the day of maximal expression, spots were categorized in clusters I (day 0) through V (day 4) (Figure 26A). All other spots were grouped in cluster O. Silver staining and SYPRO Ruby essentially led to the same clustering, despite the broader linear range of SYPRO Ruby. We selected abundant spots from synexpression clusters. Spots were excized and trypsinized. Mass spectrometry revealed the protein identities of 112 spots up to now, representing ~75 % of the total signal in the gel.

**Expression clusters correlate with biological function**

Next, we categorized identified proteins by biological function: ER resident proteins, cytoskeletal proteins, molecular chaperones, etc. The complete data set of all identified spots is provided on a webpage (http://vrc.bijvoet-center.nl/boc1/b_cells). Most abundant proteins from six functional categories are listed in Table 1. We found that biological function strongly correlated with synexpression clustering. Where Figure 26A shows their expression clustering, Figure 26B shows, for comparison, the same protein spots clustered by biological function.

A few exceptions aside, we found that the cytoskeleton correlated to cluster I, that cytosolic and mitochondrial chaperones correlated to cluster II, that metabolic enzymes on average clustered in IV. Three functional groups, ER resident proteins, IgM subunits and proteins involved in the redox balance, correlated with cluster V. Cluster III contained few proteins, without functional match. Not only did proteins cluster by expression pattern and function, they also clustered by position in the gel, indicating similar mass and pI for proteins of similar function (Figure 26B). Examples of expression kinetics of individual spots are shown in Figure 27A. Apart from the well-established increase of ER proteins and IgM subunits alike (e.g. (Wiest et al., 1990)), we found, in agreement with previous reports, an upregulation of Cathepsin E (Sealy et al., 1996) and a downregulation of MHC class II b subunit (Silacci et al., 1994; Piskurich et al., 2000). This further validates I.29μ+ cells as an excellent model for B cell differentiation.

By monitoring the changes per biological category we could reconstruct the following series of events in the course of B cell differentiation (Figure 27B). First, mitochondrial and cytosolic chaperones peaked at day one. Second, metabolic enzymes peaked at day 3. Third, ER resident proteins gradually increased from day 0 to day 4, accompanied by proteins involved in redox balance. Last, after a lag-time of two days, the IgM subunits l, μ, and J-chain increased. Whereas l and μ were identified in our proteomics screen (Figure 27A), J-chain became detectable only by immunoblotting (Figure 29B). Because data were normalized to total protein amounts per gel, cytoskeletal proteins decreased relative to proteins in other categories (Figure 27, A and B).

**Increase of IgM synthesis follows initial ER expansion**

We found that the increase of IgM subunits follows the initial increase of resident ER proteins. The steady-state levels of intracellular IgM subunits we monitored, however, are the net effect of synthesis, degradation and secretion. We therefore compared synthesis rates of resident ER proteins and IgM subunits by pulse-labeling cells short enough to render secretion and/or degradation irrelevant (Sitia et al., 1987). Cells were activated for 0, 1, 2 or 4 days and were radioactively labeled for 10 min after each activation period. Detergent cell lysates were analysed by 2D gel electrophoresis, normalized for protein content, and signals were detected by phosphor imaging. Signals of proteins and total incorporation of radioactive label were quantified. Labeling abruptly increased the first day, reflecting the overall increase in protein synthesis, only to decrease again at day 4 (Figure 28A). Whereas synthesis of resident ER proteins already started to increase on day 1, as shown in figure 28B for calreticulin, calnexin, and GRP94, synthesis of IgM subunits did not increase until day 2 after activation. We concluded that initial ER expansion indeed preceded bulk IgM biosynthesis and secretion. Continued ER expansion coincided with steeply increased IgM production. Initial ER expansion does not involve a classical XBP-1 mediated UPR

XBP-1 is a prominent UPR signal (Shen et al., 2001; Yoshida et al., 2001; Calfon et al., 2002) but is essential for plasma cell development as well (Reimold et al., 2001). It transduces ER stress to the nucleus as a transactivator of ER resident protein expression (Yoshida et al., 2001). We did not detect XBP-1 on the 2D gels, hence, to examine the involvement of XBP-1 in ER expansion during differentiation, we analyzed XBP-1 expression by immunoblotting nuclear extracts of differentiating cells. For comparison we analyzed total cell extracts of MEF cells treated with tunicamycin, an agent that provokes a strong UPR. In activated I.29μ+ cells (Figure 29A), the 54 kDa form of XBP-1 increased late during differentiation, only reaching high levels after day 2, whereas in tunicamycin treated cells already after 6 hours a prominent XBP-1 band was detected. Interestingly, most XBP-1 was migrating at a position of ~100 kDa at day 4 of differentiation. This band, also induced in tunicamycin treated MEF cells, might represent a heterodimer with c-fos (Ono et al., 1991). The kinetics of accumulation of XBP-1 matched those of all IgM subunits, l and μ (Figures 27 and 28B) as well as J-chain (Figure 29B). We concluded that accumulation of high levels of XBP-1 did not precede the increased load of IgM in the ER, suggesting that while the first stage of ER expansion did not involve a classical UPR, the ER load that starts at day 3 does activate a classical, cargo-induced XBP-1 mediated UPR.

**Discussion**

Following B cell differentiation by a dynamic proteomics approach gave us a broad, unbiased view of the changes that occur. We found that the vast majority of spots varied substantially with time, whereas for instance in the bacterial cell cycle only 15% of spots change (Grunenfelder et al., 2001). Upon analysis of the changes of highly abundant proteins, representing six important cellular machineries, we concluded that the B cell carefully prepares for its secretory role.

**Functionally related clusters of proteins appear in waves.**

Because expression clusters correlated to functional groups, a picture emerged of the series of events that occur in the course of B cell differentiation. Mitochondrial and cytosolic chaperones were upregulated early. Metabolic enzymes peaked later. ER resident folding factors and redox balance proteins were linearly upregulated until the end. In contrast, IgM subunits increased exponentially, after a lag-time of two days. We normalized our analyses based on the amount of protein in the gel, resulting in a relative decrease of cytoskeletal proteins. From the increase in protein content of cells, however, we calculated that cytoskeletal proteins in fact slightly increased per cell. As a consequence, the increases we observed for other clusters are more dramatic than shown (e.g. after 4 days of activation, ER proteins have increased 15-fold compared to cytoskeleton). Per differentiating cell, even this factor of 15-fold is likely an underestimate since at day four not all cells had fully differentiated yet.

Although we found a few exceptions, the correlation between expression timing and protein function was evident. For instance, eleven out of the 12 known ER proteins (ERps) we identified belonged to expression cluster V.

By reversing this argument, can the temporal expression pattern of a protein be exploited to predict its functional role? Indeed, we identified several novel proteins that followed the cluster-function relationship. For example, we found a protein in cluster V, that carried hallmarks of ER resident proteins, being an N-terminal signal sequence needed for targeting to the ER, a C-terminal tetrapeptide RDEL that mediates ER residency, and a putative CRFS redox active site. We named this protein ERp44. Evidence that ERp44 is indeed a bonafide ER folding assistant has been obtained in the meantime (Anelli et al., 2002).

Remarkably, protein categories also clustered in the gel. The tendency of resident ER proteins to focus at acidic pH may be a consequence of their Ca++ binding properties (Glu/Asp stretches) (Lucero et al., 1998). Many cytosolic proteins, whose folding may be facilitated by entering into chaperonin cages, tend to be smaller than 70 kDa. For other clusters the colocalization on gels is less easily understood. Possibly, protein function or the specific milieu within compartments defines what pI and MW range are optimal. This would explain why, despite low sequence identity, the pI of many proteins has been conserved from yeast to mammals (E. van Anken, unpublished observations).

**Getting prepared: providing energy and supplies**

The synthetic requirements of this dramatic cellular expansion likely explain the instantaneous increase of most mitochondrial and cytosolic chaperones, except, not surprisingly, the non stress-inducible HSC70 (Ingolia and Craig, 1982). Their instantaneous upregulation apparently is essential to sustain energy and protein production. As discussed above, the subsequent decrease in this class of proteins is again relative. The cells seem to anticipate their future needs for these proteins, since they are upregulated rapidly to a level that is sufficient throughout the entire differentiation process.

On average, metabolic enzymes peaked at three days after activation. The upregulation of the glycolytic enzymes glyceraldehyde 3-phosphate dehydrogenase and a-enolase likely reflects the increase in energy requirements. Increased levels of ornithine aminotransferase, phosphoglycerate dehydrogenase and phosphoserine aminotransferase may be necessary to facilitate the metabolism of amino acids, serine in particular, to sustain the rise in protein synthesis. The emphasis on serine synthesis could reflect the need of this amino acid for both protein and lipid synthesis; phosphatidylserine may be needed for membrane expansion. The transient upregulation of 6-phosphogluconolactase may reflect membrane expansion and/or a change in redox balance. This enzyme is involved in the pentosephosphate pathway, which generates NADPH, a redox-active molecule a.o. necessary for fatty acid synthesis. The upregulation of delta-levulinic acid dehydratase, involved in heme synthesis, may reflect the growing need of heme for cytochrome synthesis. Total metabolic activity, as monitored by incorporation of radioactive label, rapidly increased twofold the first day of activation only to decrease at day 4. Altogether, these data indicate that the cell first needs energy, as well as protein and membrane synthesis for expansion. Later, metabolic activity can be lower again since now the cell is fully expanded and "only" needs energy for IgM production and maintenance of the new equilibrium.

**Redox balance during differentiation**

As opposed to the cytosol, the ER maintains an oxidative environment (Hwang et al., 1992). Since the ER expands immensely, the cell on average may become more oxidative. Most proteins involved in the redox balance, residing outside of the ER in the cytosol and mitochondria, were linearly upregulated in the same fashion as resident ER proteins. Redox proteins could buffer against oxidative stress leaking out of the ER (Tu and Weissman, 2002). Alternatively, these redox proteins may function to help provide and maintain or influence the oxidative environment in the ER, perhaps to support IgM polymerization. Although in B lymphocytes, secretory μ chains are synthesized and assembled into μ212 'monomers', these do not assemble into polymers (Shachar et al., 1992). Upon B cell activation, IgM monomers start to polymerize via interchain disulfide bonds, concomitant with the beginning of detectable J-chain expression. Expression of J-chains in activated B cells is not required for polymerization per se, but rather ensures that pentamers are formed (Brewer et al., 1994). The sudden change in tendency of IgM monomers to form interchain disulfide-linked polymers may be facilitated by a switch in the redox balance in the ER (Sitia et al., 1990). Redox active proteins could be indirectly involved in either the prevention of polymerization in B cells, or in the stimulation of polymerization in a plasma cell.

**Collective increase of ER proteins**

We witnessed a continuous increase of ER resident proteins throughout the differentiation process, such that at four days after activation a quarter of the total signal in the gel represented ER proteins. The upregulation of resident ER proteins was coordinated such that their relative abundance was almost constant. A notable exception was the relative decrease of calnexin (CNX) within the ER at steady state levels, despite its increased de novo synthesis. Interestingly, this coincided with an increase of the calreticulin (CRT) pool. Perhaps CRT can compensate for the relative decrease of CNX, since CNX and CRT are functionally related proteins (Trombetta and Helenius, 1998). For the remainder, we found no indications that any known ER proteins be recruited specifically during differentiation, although this has been reported for ERp72 and GRP94 (Wiest et al., 1990). Either all identified ER proteins are required for the maturation of IgM, or the ER can only expand en bloc. This would be consistent with the fact that ER proteins share cis-acting elements in their promoter region: ER stress elements (ERSE) (Yoshida et al., 1998; Roy and Lee, 1999; Kokame et al., 2001) or unfolded protein response elements (UPRE) (Wang et al., 2000; Yoshida et al., 2001)

**Does IgM drive ER expansion?**

An important regulatory machinery for ER expansion is represented by the unfolded protein response (UPR). Although the UPR is much more complex in mammals than in yeast, one pathway has been conserved: the Ire1 pathway (reviewed in (Patil and Walter, 2001; Lee, 2001; Ma and Hendershot, 2001). The Ire1 proteins have an unfolded protein sensor domain in the ER lumen. Upon accumulation of unfolded proteins, the effector domain of Ire1 in the cytosol splices the mRNA of the transcription factor Haclp in yeast (Patil and Walter, 2001), or its mammalian homolog XBP-1 (Shen et al., 2001; Yoshida et al., 2001; Calfon et al., 2002). After religation of their transcript, Haclp and XBP-1 are synthesized and enhance expression of resident ER proteins through trans-activation of the ERSEs (for XBP-1) (Yoshida et al., 2001) or UPREs (for both XBP-1 and Haclp) (Patil and Walter, 2001; Yoshida et al., 2001) in their promoter regions. Increased levels of resident ER proteins in turn are supposed to reinforce the folding and assembly capacity of the ER. Indicative of the importance of the UPR in full B cell differentiation is that mice lacking XBP-1 have normal B cells but no Ig secreting plasma cells (Reimold et al., 2001).

We show that during B cell differentiation IgM production follows rather than precedes the initial ER expansion: hence IgM is unlikely to be its driving force. This indicates that the first stage of ER expansion bypasses the classical UPR mechanism, apparently via signals initiated by mitogen or antigen stimulation, such as Blimp-1, a key regulator of B cell differentiation (Shaffer et al., 2002; Turner et al., 1994). The sudden increase in synthesis of IgM subunits after two days and the subsequent accumulation of intracellular IgM subunits, however, does seem to trigger a classical XBP-1 ER load dependent UPR. Only then, after 3 days of activation, XBP-1 reached high levels, as had been reported earlier for primary splenic B cells (Calfon et al., 2002).

Interestingly, mouse embryonic fibroblasts lacking Ire 1 have an intact UPR (Lee et al., 2002). Rather than for the UPR per se, the Ire 1/XBP-1 pathway seems necessary for the development of secretory cells: XBP-1 is crucial for liver development (Reimold et al., 2000) and Ire 1 for gut development in C. elegans (Shen et al., 2001). This suggests that the two-stage launch of ER expansion we observed in B cells may be common to and essential for all cells that differentiate into professional secretors. They first prepare themselves meticulously so as to embark on their new career. Once the first steps are taken, the increased production of cargo may provide a UPR-linked feed-forward circuit that drives further ER expansion (Reimold et al., 2001) and ultimately leads to cell death.

**Experimental Procedures**

**Cell culture and activation of B cells**

We used the I.29μ+ (IgM, 1) lymphoma cell line as model B-lymphocytes (Alberini et al., 1987). Cells were cultured in suspension in RPMI (Life Technologies) supplemented with 10% fetal calf serum LPS free (FCS, Hyclone), glutamax (1 mM), penicillin (100 U/ml), and streptomycin (100 mg/ml), sodium pyruvate (1 mM) and b-mercaptoethanol (50 μM). For differentiation experiments cells were incubated in the presence of 20 μg/ml LPS (Sigma). Cells were harvested before and after 1, 2, 3 or 4 days of differentiation.

**Western blotting**

Cells were washed in phosphate buffered saline (PBS; Life technologies). Fresh culture medium containing 2% FCS was added. Cells were incubated for 5 hours in the presence of LPS before cells were pelleted. Culture media were collected and cells were lysed in MNT (20 mM MES, 100 mM NaCl, 30 mM Tris-HCl pH 7.4), containing 1% Triton X-100 and protease inhibitors (chymostatin, leupeptin, aprotinin, pepstatin A, and PMSF). Cell lysates were centrifuged for 10 min at 17,000 x g to pellet nuclei. Proteins from post-nuclear supernatants from lysates or from culture media were analyzed using 10% sodium dodecyl sulphate polyacrylamide (SDS-PA) gels, and proteins were transferred onto nitrocellulose. The nitrocellulose was saturated with 3% fat free milk (Protifar, Nutricia) for 1 hour, and then incubated with horseradish peroxidase-conjugated anti-IgM or anti-J-chain antibodies for 1 hour. For XBP-1 detection, nuclear pellets from lysates of equal numbers of cells per time point were solubilized in sample buffer (200 mM Tris pH 6.8, 3% SDS, 10% glycerol, 1 mM EDTA and 0.004% bromophenol blue), forced through a needle, sonicated, and heated for 5 min at 95°C. SDS-PAGE and protein transfer onto nitrocellulose was performed as described above. The nitrocellulose was incubated with a polyclonal rabbit anti XBP-1 antibody (Santa Cruz). Antibody binding was detected via ECL (Amersham) and Biomax MR (Kodak) film.

**Immunofluorescence**

Cells were adhered onto poly-L-lysine (Sigma) coated multispot slides for 30 min at 37°C. Cells were fixed in 3% paraformaldehyde for 15 min and permeabilized with 0.1% NP-40 for 10 min. Next, cells were incubated with an FITC-conjugated anti-IgM antibody. IgM expression was monitored by fluorescence microscopy.

**FACS analysis**

R-PE anti-mouse CD138, biotinylated anti-mouse CD45/B220 and FITC-Streptavidin were from BD PharMingen, San Diego USA. At least 5 x 105 I.29μ+ cells activated for various periods of time were washed in PBS and incubated for 30 min with 200 μl of 2.4G2 hybridoma supernatant and 10% rat serum to block Fc receptors (kind gifts of A. Mondino, DiBiT-HSR, Milan). Cells then were stained with relevant antibodies for 30 min at 4°C in the dark. Flow cytometry data were obtained with a FACSCalibur (BD Biosciences) and analyzed using the CellQuest software (BD Biosciences)

**Two-dimensional gel electrophoresis**

Cells were washed twice in ice-cold 0.25% sucrose (w/v), 25 mM HEPES-KOH pH 7.0 and lysed in a small volume of the same solution, containing 1% Triton X-100 and protease inhibitors cocktail. Cell lysates were centrifuged for 10 min at 17,000 x g to pellet nuclei. Protein concentrations of post-nuclear supernatants were determined by DC Protein Assay kit (Biorad). Approximately 200 μg of proteins from post-nuclear lysates were dissolved in reswelling solution (6.0 M urea (Boehringer), 1.9 M thiourea (Sigma), 4% Triton X-100 (Sigma), 15 mM DTT and 0.5 % (v/v) carrier ampholytes pH 3-10 (AP Biotech)). After reswelling of 18-cm Immobiline DryStrips pH 3-10 Non-Linearized (AP Biotech) with our samples, proteins were separated in the first dimension and on subsequently on 10% SDS-PA gels in the second dimension. This technique allows analysis of many proteins but is not exhaustive (Gygi et al., 2000).

**Image analysis and data processing**

Protein spots were visualized by silver staining or by SYPRO Ruby staining (Molecular Probes). Signals from proteins were detected by densitometric scanning of silver stained gels or by fluorescence imaging of SYPRO Ruby stained gels. Spot detection and matching of spots between gels was performed with the PDQuest software package, version 6.0 (Biorad). The 2D gel of the third day of differentiation was the basis for an in silico "reference gel". Normalization between gels was based on total protein amount (i.e. the total signal of valid spots) per gel. Spot intensities were expressed in parts per million (ppm) of total spot intensity per gel. Background was set at 80 ppm and if the intensity of a spot was below 80 ppm at any time point, we corrected this to background, i.e. 80 ppm. If a spot had a lower intensity than 200 ppm (2.5 * background) in every gel, the spot was removed from the analysis set. The remaining 409 spots were clustered according to expression kinetics as follows: if spot intensities differed more than twofold between at least two points, we determined at which time point expression was maximal. To determine whether only a single maximum in expression existed, we set the following criteria: The spot intensity of the time point adjacent to the maximum should not be more than two-fold lower than the spot intensity at one time point further from the maximum etc. If spots had a single maximum according to the above criteria, they were categorized into clusters I, II, III, IV, or V, corresponding to highest expression levels at day 0, 1, 2, 3 or 4. All other spots were grouped in cluster O. Calculations and formulas can be found as supplementary material (http://vrc.bijvoet-center.n/bocl/b_cells).

**Mass spectrometric analysis of protein spots**

Spots were excised and digested with trypsin (Roche Diagnostics, GmbH, sequencing grade) and peptide mixtures from in-gel digests were purified and concentrated. Peptides were subjected to mass spectrometric analysis as described previously (Wilm et al., 1996) with some modifications (Romijn et al, in preparation). Peptide mass fingerprints were acquired on a Voyager DE-STR (Perseptive Biosystems) reflectron time of flight (TOF) mass spectrometer. Peptide sequences were determined by nanoES tandem MS on a quadrupole time of flight mass spectrometer (Q-TOF, Micromass, Manchester, UK) or by MALDI tandem MS on a MALDI quadrupole time of flight mass spectrometer (MALDI-Q-TOF, Micromass, Manchester, UK), or by UltraFlex-TOF/TOF (Bruker Daltonics, Bremen, Germany).

**Protein identification**

Protein identities were revealed either by comparison of peptide mass fingerprints to the theoretical peptide masses of all available proteins in the database, using Profound (Zhang and Chait, 2000), or by comparison of sequence tags from tandem MS analyses to the sequences of proteins or translated ESTs in all public databases, using the peptide sequence tag tool (Mann and Wilm, 1994) and the MS BLAST tool (Shevchenko et al., 2001).

**Metabolic labeling**

Cells were washed once in Hanks' balanced salt solution (Life technologies) and pre-incubated in starvation medium lacking methionine and cysteine for 15 min at 37°C. Cells were pulse labeled for 10 min with 50 μCi of Redivue pro-mix L-[35S] in vitro labeling mix (AP Biotech). Next, cells were detergent lysed and post-nuclear lysates were analyzed on 2D gels as described above. In addition, lysates were analyzed on reducing 1D 10% SDS-PAGE. Gels were dried between Cellophane (Biorad) sheets before exposure of storage phosphor screens (Kodak. Signals of radioactively labeled proteins were detected with the BioRad Personal Molecular Imager and quantified using the Biorad QuantityOne software package. Total incorporation of label was quantified from the 1D.

**Supplementary material**

Our website (http:/vrc.bijvoet-center.nl/bocl/b_cells) displays details on calculations and additional information on expression kinetics of all identified proteins.

References

Alberini, C., Biassoni, R., DeAmbrosis, S., Vismara, D., and Sitia, R. (1987). Differentiation in the murine B cell lymphoma 1.29: individual mu + clones may be induced by lipopolysaccharide to both IgM secretion and isotype switching, Eur J Immunol 17, 555-62.
Anelli, T., Alessio, M., Mezghrani, A., Simmen, T., Talamo, F., Bachi, A., and Sitia, R. (2002). ERp44, a novel endoplasmic reticulum folding assistant of the thioredoxin family, Embo J 21, 835-844.
Brewer, J. W., Randall, T. D., Parkhouse, R. M., and Corley, R. B. (1994). IgM hexamers?, Immunol Today 15, 165-8.
Calfon, M., Zeng, H., Urano, F., Till, J. H., Hubbard, S. R., Harding, H. P., Clark, S. G., and Ron, D. (2002). IRE1 couples endoplasmic reticulum load to secretory capacity by processing the XBP-1 mRNA, Nature 415, 92-6.
Chilosi, M., Adami, F., Lestani, M., Montagna, L., Cimarosto, L., Semenzato, G., Pizzolo, G., and Menestrina, F. (1999). CD138/syndecan-1: a useful immunohistochemical marker of normal and neoplastic plasma cells on routine trephine bone marrow biopsies, Mod Pathol 12, 1101-6.
Fagioli, C., and Sitia, R. (2001). Glycoprotein quality control in the endoplasmic reticulum. Mannose trimming by endoplasmic reticulum mannosidase I times the proteasomal degradation of unassembled immunoglobulin subunits, J Biol Chem 276, 12885-92.
Frey, J. R., Fountoulakis, M., and Lefkovits, I. (2000). Proteome analysis of activated murine B-lymphocytes, Electrophoresis 21, 3730-9.
Grunenfelder, B., Rummel, G., Vohradsky, J., Roder, D., Langen, H., and Jenal, U. (2001). Proteomic analysis of the bacterial cell cycle, Proc Natl Acad Sci U S A 98, 4681-6.
Gygi, S. P., Corthals, G. L., Zhang, Y., Rochon, Y., and Aebersold, R. (2000). Evaluation of two-dimensional gel electrophoresis-based proteome analysis technology, Proc Natl Acad Sci U S A 97, 9390-5.
Haas, I. G., and Wabl, M. (1983). Immunoglobulin heavy chain binding protein, Nature 306, 387-9.
Hwang, C., Sinskey, A. J., and Lodish, H. F. (1992). Oxidized redox state of glutathione in the endoplasmic reticulum, Science 257, 1496-502.
Ingolia, T. D., and Craig, E. A. (1982). Drosophila gene related to the major heat shock-induced gene is transcribed at normal temperatures and not induced by heat shock, Proc Natl Acad Sci U S A 79, 525-9.
Kettman, J., and Lefkovits, I. (1984). Changes in the protein pattern of murine B cells after mitogenic stimulation, Eur J Immunol 14, 778-81.
Kokame, K., Kato, H., and Miyata, T. (2001). Identification of ERSE-II, a new cis-acting element responsible for the ATF6-dependent mammalian unfolded protein response, J Biol Chem 276, 9199-205.
Lee, A. S. (2001). The glucose-regulated proteins: stress induction and clinical applications, Trends Biochem Sci 26, 504-10.
Lee, K., Tirasophon, W., Shen, X., Michalak, M., Prywes, R., Okada, T., Yoshida, H., Mori, K., and Kaufman, R. J. (2002). IRE1-mediated unconventional mRNA splicing and S2P-mediated ATF6 cleavage merge to regulate XBP1 in signaling the unfolded protein response, Genes Dev 16, 452-66.
Lin, H. Y., Masso-Welch, P., Di, Y. P., Cai, J. W., Shen, J. W., and Subjeck, J. R. (1993). The 170-kDa glucose-regulated stress protein is an endoplasmic reticulum protein that binds immunoglobulin, Mol Biol Cell 4, 1109-19.
Lucero, H. A., Lebeche, D., and Kaminer, B. (1998). ERcalcistorin/protein-disulfide isomerase acts as a calcium storage protein in the endoplasmic reticulum of a living cell. Comparison with calreticulin and calsequestrin, J Biol Chem 273, 9857-63.
Ma, Y., and Hendershot, L. M. (2001). The unfolding tale of the unfolded protein response, Cell 107, 827-30.
Mann, M., and Wilm, M. (1994). Error-tolerant identification of peptides in sequence databases by peptide sequence tags, Anal Chem 66, 4390-9.
Melnick, J., Dul, J. L., and Argon, Y. (1994). Sequential interaction of the chaperones BiP and GRP94 with immunoglobulin chains in the endoplasmic reticulum, Nature 370, 373-5.
Ono, S. J., Liou, H. C., Davidon, R., Strominger, J. L., and Glimcher, L. H. (1991). Human X-box-binding protein 1 is required for the transcription of a subset of human class II major histocompatibility genes and forms a heterodimer with c-fos, Proc Natl Acad Sci U S A 88, 4309-12.
Patil, C., and Walter, P. (2001). Intracellular signaling from the endoplasmic reticulum to the nucleus: the unfolded protein response in yeast and mammals, Curr Opin Cell Biol 13, 349-55.
Piskurich, J. F., Lin, K. I., Lin, Y., Wang, Y., Ting, J. P., and Calame, K. (2000). BLIMP-I mediates extinction of major histocompatibility class II transactivator expression in plasma cells, Nat Immunol 1, 526-32.
Reddy, P., Sparvoli, A., Fagioli, C., Fassina, G., and Sitia, R. (1996). Formation of reversible disulfide bonds with the protein matrix of the endoplasmic reticulum correlates with the retention of unassembled Ig light chains, Embo J 15, 2077-85.
Reddy, P. S., and Corley, R. B. (1999). The contribution of ER quality control to the biologic functions of secretory IgM, Immunol Today 20, 582-8.
Reimold, A. M., Etkin, A., Clauss, I., Perkins, A., Friend, D. S., Zhang, J., Horton, H. F., Scott, A., Orkin, S. H., Byrne, M. C., et al. (2000). An essential role in liver development for transcription factor XBP-1, Genes Dev 14, 152-7.
Reimold, A. M., Iwakoshi, N. N., Manis, J., Vallabhajosyula, P., Szomolanyi-Tsuda, E., Gravallese, E. M., Friend, D., Grusby, M. J., Alt, F., and Glimcher, L. H. (2001). Plasma cell differentiation requires the transcription factor XBP-1, Nature 412, 300-7.
Roy, B., and Lee, A. S. (1999). The mammalian endoplasmic reticulum stress response element consists of an evolutionarily conserved tripartite structure and interacts with a novel stress-inducible complex, Nucleic Acids Res 27, 1437-43.
Sealy, L., Mota, F., Rayment, N., Tatnell, P., Kay, J., and Chain, B. (1996). Regulation of cathepsin E expression during human B cell differentiation in vitro, Eur J Immunol 26, 1838-43.
Shachar, I., Amitay, R., Rabinovich, E., Haimovich, J., and Bar-Nun, S. (1992). Polymerization of secretory IgM in B lymphocytes is prevented by a prior targeting to a degradation pathway, J Biol Chem 267, 24241-7.
Shaffer, A. L., Lin, K. I., Kuo, T. C., Yu, X., Hurt, E. M., Rosenwald, A., Giltnane, J. M., Yang, L., Zhao, H., Calame, K., and Staudt, L. M. (2002). Blimp-1 orchestrates plasma cell differentiation by extinguishing the mature B cell gene expression program, Immunity 17, 51-62.
Shen, X., Ellis, R. E., Lee, K., Liu, C. Y., Yang, K., Solomon, A., Yoshida, H., Morimoto, R., Kurnit, D. M., Mori, K., and Kaufman, R. J. (2001). Complementary signaling pathways regulate the unfolded protein response and are required for C. elegans development, Cell 107, 893-903.
Shevchenko, A., Sunyaev, S., Loboda, A., Bork, P., Ens, W., and Standing, K. G. (2001). Charting the proteomes of organisms with unsequenced genomes by MALDI- quadrupole time-of-flight mass spectrometry and BLAST homology searching, Anal Chem 73, 1917-26.
Silacci, P., Mottet, A., Steimle, V., Reith, W., and Mach, B. (1994). Developmental extinction of major histocompatibility complex class II gene expression in plasmocytes is mediated by silencing of the transactivator gene CIITA, J Exp Med 180, 1329-36.
Sitia, R., Neuberger, M., Alberini, C., Bet, P., Fra, A., Valetti, C., Williams, G., and Milstein, C. (1990). Developmental regulation of IgM secretion: the role of the carboxy- terminal cysteine, Cell 60, 781-90.
Sitia, R., Neuberger, M. S., and Milstein, C. (1987). Regulation of membrane IgM expression in secretory B cells: translational and post-translational events, Embo J 6, 3969-77.
Sitia, R., Rubartelli, A., Deambrosis, S., Pozzi, D., and Hammerling, U. (1985). Differentiation in the murine B cell lymphoma 1.29: inductive capacities of lipopolysaccharide and Mycoplasma fermentans products, Eur J Immunol 15, 570-5.
Stavnezer, J., Sirlin, S., and Abbott, J. (1985). Induction of immunoglobulin isotype switching in cultured 1.29 B lymphoma cells. Characterization of the accompanying rearrangements of heavy chain genes, J Exp Med 161, 577-601.
Trombetta, E. S., and Helenius, A. (1998). Lectins as chaperones in glycoprotein folding, Curr Opin Struct Biol 8, 587-92.
Tu, B. P., and Weissman, J. S. (2002). The FAD- and 0(2)-Dependent Reaction Cycle of Erol-Mediated Oxidative Protein Folding in the Endoplasmic Reticulum, Mol Cell 10, 983-94.
Turner, C. A., Jr., Mack, D. H., and Davis, M. M. (1994). Blimp-1, a novel zinc finger-containing protein that can drive the maturation of B lymphocytes into immunoglobulin-secreting cells, Cell 77, 297-306.
Wang, Y., Shen, J., Arenzana, N., Tirasophon, W., Kaufman, R. J., and Prywes, R. (2000). Activation of ATF6 and an ATF6 DNA binding site by the endoplasmic reticulum stress response, J Biol Chem 275, 27013-20.
Wiest, D. L., Burkhardt, J. K., Hester, S., Hortsch, M., Meyer, D. I., and Argon, Y. (1990). Membrane biogenesis during B cell differentiation: most endoplasmic reticulum proteins are expressed coordinately, J Cell Biol 110, 1501-11.
Wilm, M., Shevchenko, A., Houthaeve, T., Breit, S., Schweigerer, L., Fotsis, T., and Mann, M. (1996). Femtomole sequencing of proteins from polyacrylamide gels by nano- electrospray mass spectrometry, Nature 379, 466-9.
Yoshida, H., Haze, K., Yanagi, H., Yura, T., and Mori, K. (1998). Identification of the cis-acting endoplasmic reticulum stress response element responsible for transcriptional induction of mammalian glucose-regulated proteins. Involvement of basic leucine zipper transcription factors, J Biol Chem 273, 33741-9.
Yoshida, H., Matsui, T., Yamamoto, A., Okada, T., and Mori, K. (2001). XBP1 mRNA is induced by ATF6 and spliced by IRE1 in response to ER stress to produce a highly active transcription factor, Cell 107, 881-91.
Zhang, W., and Chait, B. T. (2000). ProFound: an expert system for protein identification using mass spectrometric peptide mapping information, Anal Chem 72, 2482-9.

Example 2

The endoplasmic reticulum (ER) is one of the compartments in eukaryotic cells were proteins fold. The proteins that use the ER as a folding compartment are those that reside in the secretory pathway, on the plasma membrane or proteins that get secreted. To enter the ER, proteins need a specific targeting signal. The targeting signal for the ER, the signal sequence, is a hydrophobic stretch of amino acids at the N-terminus of a protein (Heijne 1986; Zheng and Nicchitta 1999). After translocation of the protein into the ER has begun, this signal sequence is often cleaved off by the membrane bound signal peptidase (Martoglio and Dobberstein 1998).

A feature special for folding in the ER is the addition of N-glycans to newly synthesized proteins. These N-glycans are transferred from dolichol phosphate carriers to the folding proteins by oligosaccharyl transferases. Different from other compartments where proteins fold, such as the cytosol or mitochondria, folding in the ER is accompanied by disulfide bond formation. Disulfide bonds can be important for the structure and function of these proteins (Gething and Sambrook 1992; Raina and Missiakas 1997) or function as checkpoints during the folding process. The oxidizing environment of the ER is reflected by the relatively high concentration of oxidized gluthathione (GSSG). In the cytosol the ratio GSH:GSSG varies between 30 and 100, whereas in the lumen of the ER this ratio is approximately 3 (Hwang et al. 1992).

The ER is packed with chaperone molecules and folding enzymes. The ER resident proteins have a C-terminal retention signal that prevents their secretion (Munro and Pelham 1987). The tetrapeptide KDEL (or another tetrapeptide with similar characteristics) binds to a receptor localized in the Golgi apparatus (Scheel and Pelham 1996). When a KDEL containing protein binds to this receptor, the complex travels to the ER. Here, the KDEL receptor releases its substrate because of the higher pH in the ER. After dissociation, the receptor is transported to the Golgi (Wilson et al. 1993).

The ER resident proteins assist the growing polypeptide chains that enter the ER to reach their mature state (Gething and Sambrook 1992), and function in the quality control of the folding proteins. Only when a protein is folded correctly, it can leave the ER and travel further along the secretory pathway; unfolded or misfolded proteins are retained and eventually degraded (Ellgaard and Helenius 2001).

Chaperones such as BiP and GRP94 bind to newly translated proteins and protect exposed hydrophobic sections during folding (Flynn et al. 1991; Melnick et al. 1994). Disulfide bonds are introduced and isomerized by oxidoreductases such as PDI and ERp57 (Molinari and Helenius 1999). Other members of the PDI family, such as ERp72 (Mazzarella et al. 1990) and P5 (Kikuchi et al. 2002) seem to have a similar role. These proteins have a common active site, CXXC, within a domain homologous to thioredoxin. When the active site cysteïnes of PDI are in the oxidized form (disulfide bonded), this disulfide bond can be transferred to substrate proteins (Lyles and Gilbert 1991). PDI has to be reoxidized to regain oxidase activity. A candidate recharger of PDI is Ero1, since in yeast, it has been shown that this protein is required to keep PDI oxidized and functional (Frand and Kaiser 1999). Whether the human homologue, Ero1-Lα, has a similar function awaits confirmation (Cabibbo et al. 2000).

Most of the chaperones and folding enzymes have broad substrate specificity. However, some secretory proteins need the assistance of specialized chaperones to fold correctly. Hsp47, for example, is a collagen specific chaperone (Nagata and Hosokawa 1996) that functions early in the secretory pathway (Tasab et al. 2002). Another substrate specific chaperone is Receptor-Associated protein (RAP), which binds preferentially to unfolded endocytic receptors of the LDL receptor gene family (Willnow 1998). However, RAP might also bind to other proteins (Sarti et al. 2000).

Most chaperones and folding enzymes are expressed in all tissues. However, a tissue-specific homologue to PDI has been described. PDIp is a pancreas specific protein with significant sequence similarity to PDI (Desilva et al. 1996), as well as functional similarity (Elliott et al. 1998; Volkmer et al. 1997).

In order to identify new ER resident proteins (ERps), differentiating B-cells were used in a biochemical screen (Van Anken et al. submitted). During B-cell differentiation, the secretory machinery, including the ER, expands. By a dynamic proteomics approach, we observed linear upregulation of the ERps as expected. We considered unknown proteins that showed similar expression profiles during B-cell differentiation candidate ERps.

One of the candidate ERps that we identified in the B-cell screening was an 18.3 kDa protein, which we called smERp1 (small ER resident protein 1). The sequence of smERp1 shows hallmarks of ERps. Here we show that smERp1 is indeed an ER resident protein which represents a novel CXXC-motif containing protein family. The effect of smERp1 on the folding of other proteins suggests that smERp1 may function as a reductase.

Results

Identification of smERp1

To identify new ER resident proteins, murine I.29μ+ lymphocytes were activated with LPS for different times. Upon activation, I.29μ+ lymphocytes differentiate into ImmunoglobulinM (IgM) secreting plasma cells with an enormous ER. Proteins from lysates of these differentiating B cells were separated by 2D gel electrophoresis and spot identities were revealed by mass spectrometry. During the metamorphosis of the B-cells, different functional groups of proteins are regulated in characteristic patterns, the ERps were upregulated as expected (Van Anken et al, submitted). Unknown proteins that were similarly upregulated were considered candidate ERps. Among these proteins was an 18.3 kDa protein with a pI of 5.3 (Fig 30a), which we called small ER resident protein 1 (smERp1). smERp1 is undetectable in dormant B-cells, but in the IgM secreting plasma cells, it is one of the most abundant proteins (Fig 30b). Its sequence suggests that smERp1 is an ER resident protein with possible redox activity (Fig 30c). An N-terminal signal sequence is predicted and at the C-terminus of smERp1 the tetrapeptide REEL could serve as a retention signal. The putative active redox-active site of the protein is the CDAC motif.

Evolutionary conservation and genomic organization of smERp1

Database searches allowed us to identify close homologues of smERp in two other vertebrates, Homo sapiens and Gallus gallus (chicken) (Fig 31a and 31b). The Gallus gallus homologue sequence was deduced by assembling a contig of chicken ESTs that were homologous to murine smERp1. More distant homologues of smERp1 were found in several organisms. This group of homologues was called smERp2. BLAST searches with smERp2 allowed us to identify a third group of smERp proteins, which we called smERp3. In Arabidopsis thaliana only a single smERp homologue was found. Homology among the smERp1, smERp2 and smERp3 subfamilies is very low, but they shared features that could be characteristic for their ER residency; they all contained predicted signal sequences and retention signals. All cysteïnes including the CXXC motif were conserved. The smERp proteins show no homology to known proteins, which indicates that the smERp proteins belong to a new protein family, the smERp family. The evolutionary conservation of this family is shown in figure 31b.

Almost all murine and human expressed sequence tags (ESTs) that encoded (at least in part) for smERp1 were derived from B cell enriched sources, such as spleen and bone marrow. This suggests that smERp1 is a B-cell specific protein. ESTs corresponding to smERp2 and smERp3 had less exclusive origins. Still they were predominantly derived from secretory tissue cDNA, as one would expect for candidate ERps.

We could also locate the smERp1 gene in the mouse genome (fig 30d). It is located on chromosome 18, region B3 and consists of four exons and three introns. The CXXC-motif is encoded in the first exon. A putative B lymphocyte-induced maturation protein (Blimp) promotor is found upstream of the ATG start codon. The Blimp-1 protein can induce terminal B-cell differentiation (Turner et al. 1994; Schliephake and Schimpl 1996). The presence of this promotor therefore also suggests that smERp1 is a B-cell specific protein. Downstream of the stopcodon a poly A sequence was present.

smERp1 is a resident ER protein

The presence of a cleavable signal sequence and a REEL potential retention signal suggested that smERp1 resides in the ER. To confirm this HA-tagged smERp1 was expressed in HeLa cells and visualized using immunofluorescence. Its localization was compared to that of endogenous PDI. PDI is present in the ER at high concentrations and was used to serve as an ER-marker. After infection/transfection approximately 20% of the cells were expressing smERp1 at a detectable level. PDI showed a perinuclear and reticular staining pattern, typical for the ER (Fig 32a). The staining pattern of smERp1 resembled the pattern observed for PDI (Fig 32b). Indeed, when the images are merged (Fig 32c), it is apparent that the proteins largely colocalize. This indicates that smERp1 is an ER resident protein, just as PDI.

To confirm the ER residency of smERp1 biochemically, we expressed the protein in a semi-permeable cell (SP) system. As a control, smERp1 was in vitro translated. In contrast to the in vitro system, where only the full length form of the protein was observed (Fig 32d, lane 1), a second form of lower molecular weight can be seen when smERp1 is expressed in SP-cells (Fig 32d, lane2). The largest of these corresponds to the in vitro control, and thus to the full length protein. The smaller form could represent smERp1 that is translocated into the ER where its signal sequence is cleaved off. We determined by a proteinase protection assay whether the ER membrane could protect either form of smERp1. As can be seen in Fig32d, lane 3, only the smallest form was not digested. Upon addition of detergens (Fig32d, lane 4), it was digested, thus it must have been protected by an intact membrane. We concluded that smERp1 is synthesized in a full length form, from which the signal sequence is cleaved off upon translocation into the ER.

smERp1 rapidly folds into a disulfide containing state

To follow oxidative folding, a folding assay was performed using the SP-system (Wilson et al. 1995). smERp1 was translated 30 min in the presence of DTT after which protein synthesis was stopped and the oxidizing environment was restored to enable the protein to fold for various intervals. The oxidative state of smERp1 at the different timepoints was analyzed using non-reducing SDS-PAGE (Fig 33a). Already at the 0 min timepoint, we observed some oxidized protein, despite the DTT. After 2 min all molecules without signal sequence had already folded into a native state (ox1) with a much higher mobility in gel than the reduced protein. This suggests that the disulfide bonds make the protein very compact compared to the reduced state. The reduced form of uncleaved smERp1 disappeared much slower. After 10 min some reduced protein was still detected. A second oxidized form (ox2) appeared after 30 min. This can be either the oxidized form of uncleaved smERp1 or a less compact form of cleaved smERp1.

To determine whether the ox2 band was the oxidized form of uncleaved smERp1 or a less compact form of the cleaved protein, the 60 min sample was analyzed on a two dimensional gel (figure 33b). In the first dimension, the two oxidized forms were separated in a non-reducing gel slice. Next, the proteins were reduced in gel and the gel slice was applied onto the second dimension, in which the proteins were separated by reducing SDS-PAGE. The diagonal (marked by *) represents the position of proteins that at the start of the experiment did not have disulfide bonds. Both forms of smERp1 run above this diagonal. This means that originally, disulfide bonds must have been present. The two forms of the protein have a different molecular weight, the molecular weight of ox1 corresponds to cleaved smERp1, whereas the molecular weight of ox2 corresponds to that of the uncleaved protein. This means that ox2 is the oxidized form of uncleaved smERp1 rather than an alternative less compact oxidized form of the cleaved protein.

To follow the folding of smERp1 in vivo, a pulse chase experiment of HA-tagged smERp1 was performed. During the pulse, DTT was present, to postpone oxidative folding until the chase. In vivo, the folding pattern of smERp1 was the same as in the SP-system. The majority of the proteins folded very fast into a compact disulfide-containing native state, without forming detectable intermediates. The fraction of uncleaved smERp1 was, however, negligible compared to that in the SP-system and no ox2 is formed. After 10 minutes no uncleaved protein can be detected anymore. Thus, signal sequence cleavage is much more effective in vivo than in the SP-system. We concluded that smERp1 gets translocated into the ER, where its signal sequence is instantaneously cleaved off. In the ER, smERp1 rapidly folds into an oxidized state.

smERp1 influences oxidative folding of HA

Many ERps are chaperones or folding factors. Because of this function, they influence the folding kinetics of secretory proteins. To determine whether smERp1 would have such a role, we analyzed its effect on the maturation of model proteins. We first studied the effect of smERp1 on the folding of influenza haemagglutinin (HA) by pulse chase analysis. The folding patterns of HA in the absence and presence of smERp1 were compared to each other.

HA is an 84 kDa protein containing six disulfide bonds and seven N-linked glycans. It forms two domains when properly folded: a globular N-terminal top domain and a stem-like C-terminal domain (Wilson et al. 1981; Wiley and Skehel 1987). During folding of HA, two intermediates can be observed, named IT1 and IT2 (Braakman et al. 1991).

In the absence of smERp1 (Fig 34a), already at the beginning of the chase, IT1, IT2 and native protein were present. In time, the amount of the precusors, IT1 and IT2, decreased and the amount of native protein increased correspondingly. Just below native an additional band was visible which could be due to truncated chains or to mannose trimming of the attached N-glycans. After 30 minutes the native band shifted up in gel. This is also visible in the reducing gel. Probably this shift corresponds to further modifications of the glycans in the Golgi.

In the presence of smERp1 we observed the same intermediates of HA, but folding kinetics differed from those that were observed in the absence of smERp1 (figure 34c). At a chasetime of two minutes IT1 and IT2 are relatively highly populated in the presence of smERp1: about equal amounts were in the intermediate forms as in the native form. Without smERp1 present, the majority of the HA molecules had already reached the native state. Strikingly, the folding intermediates appeared as more discrete bands in the presence of smERp1. Remarkably, also less aggregates of HA were formed in the presence of smERp1. This indicates that the folding of HA is more synchronized when smERp1 is present. We concluded that smERp1 slightly retards folding of HA.

smERp1 has an effect on the folding of HIV envelop protein gp 120

Next, we investigated the effect of smERp1 on the folding of the soluble subunit of HIV envelope protein, gp120 by pulse chase analysis. gp120 contains nine disulfide bonds and 24 N-linked glycans. In contrast to HA, gp 120 folds slowly without forming distinct intermediates. The folding can be followed by the disappearance of the smear of intermediates and the appearance of the native protein that can get secreted. Signal sequence cleavage of gp 120 occurs only after the protein has formed some structural elements, which offers an alternative way to follow gp120 maturation (Land and Braakman 2001). Because properly folded gp 120 gets secreted, it was immunoprecipitated from the culture media collected after 1, 2 and 4 hours of chase as well.

The folding pattern of gp120 in the absence of smERp1 showed the appearance of one discrete folding intermediate (IT1), a smear of less distinct folding intermediates, and a native form (Fig 35a). Furthermore, a substantial amount of gp120 was present as high molecular weight aggregates. These aggregates were disulfide-linked since they disappeared upon reduction. Secreted gp 120 appeared as a smear because of the complex glycosylation that is resistant to EndoH treatment.

In the presence of smERp1 folding kinetics were similar as in the absence of smERp1 as judged by the appearance of intermediates (Fig 35a vs 35c) and the timing of signal sequence cleavage (Fig 35b vs 35d). However, the yield of correctly folded gp 120 increased in the presence of smERp1: more native gp 120 was present intracellularly after 4 hours of chase (Fig 35a vs 35c) and a larger amount was secreted into the medium (Fig 35e). As previously shown for HA, the amount of aggregates is clearly reduced in the presence of smERp1, indicating that the interchain disulfide bonds can be reduced by smERp1. The folding intermediates and the speed with which they disappear were similar to those observed in the absence of smERp1.

We concluded that the presence of smERp1 increases the yield of correctly folded and secreted gp120.

Discussion

The ER is a specialized folding compartment in which proteins that travel through the secretory pathway obtain disulfide bonds. These disulfide bonds are transferred from ER resident oxidoreductases to the substrate proteins. In a B-cell screening we identified a novel ER resident protein which now seems to counteract this flow. This protein, which we called smERp1, appears to act as a reductase.

smERp1 is localized inside the ER

For translocation into the ER, proteins need a C-terminal signal sequence. The protease protection assay showed that the signal sequence of smERp1 is cleaved off inside the ER. In the SP-cells, smERp1 was present both in the full length and the cleaved form. Only the cleaved protein was translocated into the ER; this form was protected from digestion by the protease. The protection was not complete, however, which probably is due to leakiness of the system. Furthermore a faint protease resistant band was present at the position of the full length protein. The localization of this protein fraction is not known. The observation that the signal sequence of both normal and HA-tagged smERp1 is cleaved off confirms mass spectrometry data. When activated B-cells were directly analyzed using MALDI- ToF, peptides originating from two differently cleaved forms of smERp1 were detected. One of these forms showed the predicted signal sequence cleavage site, the other one indicated a cleavage site 3 amino acids further to the N-terminus. This is also observed in human interferon ω1 (Adolf et al. 1990), where the cleavage sites are two amino acids apart.

The presence of a C-terminal REEL retention signal (Haugejorden et al. 1991) suggested that smERp1 is not only synthesized and folded in the ER, but that is in fact an ER resident protein. The ER localization of smERp1 was confirmed using immunofluorescence. In a double labeling experiment, we showed the clear colocalization of smERp1 with PDI, which was used as a marker for the ER.

smERp1 quickly folds into a disulfide containing native state

The folding pattern of smERp1 was analyzed in the SP-system and in vivo, using a pulse chase approach. In both systems, smERp1 folded very fast into a disulfide containing native state. The shift in gel upon oxidation is very large, so the native protein is much more compact than it is in the reduced state. This indicates that a disulfide bond is formed between two distant cysteines, resulting in a large loop in the protein. During the folding of smERp1, we could not detect any folding intermediates. This can either be a reflection of the fast kinetics of smERp1 folding, or further conformational changes are to small to be distinguished from each other after the first big loop has been formed.

The signal sequence cleavage in the SP-system was not very efficient. In vivo, however, hardly any uncleaved smERp1 was detected, so the inefficient cleavage and translocation is probably an artifact of the SP-system. However, the untranslocated fraction of smERp1 could still fold into an oxidized form. This means that the oxidized gluthathione that was added to quench the DTT was sufficient to enable smERp1 to fold outside the ER, without the assistance of the ER folding machinery.

This ease and speed with which smERp1 folds was striking, but not uncommon for ERps. Most ERps contain few cysteïnes and N-glycans. As opposed to secretory proteins, which often need a lot of assistance during folding, ERps fold fast and efficiently to reinforce the folding capacity of the ER.

smERp1 affects disulfide bond formation

The folding pattern of HA was altered by the presence of smERp1. Although the same intermediates were observed in the folding pathway, they disappeared more slowly. Thus, smERp1 retarded the folding kinetics of HA. smERp1 also affected the folding of gp 120. No clear differences in the appearance of folding intermediates were detected, but the presence of smERp1 caused a tremendous increase in the yield of correctly folded and secreted gp120.

In the absence of smERp1 disulfide linked aggregates were formed. This was in contrast to previous studies (Braakman et al. 1991; Land and Braakman 2001). Aggregation might be an artifact caused by the VVT7 heterologous expression system that was used, or, in the case of gp120, by the lack of FCS during the interval between infection and the start of the pulse. Even so, in the presence of smERp1 much less aggregates were detected. Since the total signal did not diminish, we concluded that the decrease of aggregates was due to reduction by smERp1. The proteins that were hereby rescued from the aggregates were enabled to continue the folding route to native protein, which could explain why smERp1 increases the yield.

When comparing the folding patterns of HA and gp120, important differences emerge. In contrast to gp120, the folding pathway of HA is relatively straightforward. Although non-native disulfide bonds are probably formed during folding of HA, this does not cause serious problems in the folding pathway. During the formation of the correct disulfide bonds in gp120 however, probably a lot more shuffling of non-native disulfide bonds is required (Land et al, in preparation). The formation of interchain disulfide bonds also seems to be more severe in the case of gp 120. The different effect of smERp1 on these two proteins can be explained by the difference in their folding patterns. When almost all disulfide bonds that are formed during folding are in the right position, reduction of these bonds will slow down the folding. However, when non-native disulfide bonds are formed, both intra- and intermolecularly, reduction of these bonds will increase the availability of the involved cysteïnes to form other disulfide bonds and hereby increase the overall speed of the folding pathway. These observations indicate that smERp1 functions as an ER resident reductase.

The CXXC motif of smERp1 is uncommon

The putative active site of smERp1 is a CXXC motif, which is typical for ER resident oxidoreductases. The part of known oxidoreductases containing the CXXC motif all show a thioredoxin fold. The three dimensional structure of smERp1 is not known yet, but it would be very interesting to see whether the CXXC motif of smERp1 is located at a similar position as those of known oxidoreductases.

The oxidative state of the two cysteïnes in this motif determines the function of the protein; oxidase, isomerase or reductase. The transfer of a disulfide bond between two proteins happens in two subsequent steps, via the formation of a mixed disulfide bond. The reaction requires a cysteine in which the sulfur atom is present as a reactive thiolate ion, and a disulfide bonded cysteine pair (Grauschopf et al. 1995). The reaction, as depicted in equation 1 goes through a single transition state.

$$R_A\text{-}S^- + R_B\text{-}S\text{-}S\text{-}R_C \rightleftharpoons \left[ \begin{array}{c} \delta^-\ \ \delta^-\ \ \delta^- \\ R_A\text{-}S \cdots \underset{R_B}{S} \cdots S\text{-}RC \end{array} \right] \rightleftharpoons R_A\text{-}S\text{-}S\text{-}R_B + {}^-S\text{-}R_C \qquad \text{(eq. 1)}$$

It has been shown that the central residues in the CXXC motif are critical for the function and activity of the enzyme because these residues are the main responsible for the pKa of the active site cysteïne (Grauschopf et al. 1995; Huber-Wunderlich and Glockshuber 1998). This pKa determines whether the active sites residues are more stable in a disulfide-bonded state or whether they are more stable as a thiolate ion. The CXXC motif of smERp1, CDAC, is uncommon because of the presence of an acidic residue. This acidic residue is conserved among the smERp family, although ER resident oxidoreductases usually have a basic residue in their active site. PDI, ERp57 and ERp72 for example all have CGHC active sites. The presence of a negatively charged central residue probably has an effect on the pKa of the active site cysteines. The negative charge of the aspartic acid could function to destabilize the thiolate ion so that the most stable conformation of the CDAC motif would be the disulfide-bonded, which would provide a driving force for reductase activity. Another possibility is that the aspartic acid would have a role in deprotonating the cysteine. This also would explain the reductase activity of smERp1.

The function of smERp1 in B-cells

EST database searches suggest that smERp1 is a B-cell specific protein. Tissue specificity has been demonstrated before; PDIp is a pancreas specific member of the PDI family, which seems to function similar as PDI (Desilva et al. 1996). Since the experiments on the ER localization and function of smERp1 are not performed in B-cells, care has to be taken to extrapolate the data and conclude about smERp1 in activated B-cells. Still, we can speculate about this.

In dormant B-cells smERp1 is not detectable. But upon activation, smERp1 is highly upregulated and it is one of the most abundant proteins in plasma cells. The appearance of smERp1 coincides with the start of IgM secretion. In dormant B-cells, IgM monomers are assembled but pentamerization does not occur. Instead, monomers are retained in the ER lumen via a mechanism called thiol-retention (Sitia et al. 1990; Isidoro et al. 1996). Proteins in the ER matrix form a mixed disulfide bond with the unpaired cysteïne in the tail of the IgM monomers. In plasma cells however, IgM pentamerizes through forming interchain disulfide bonds between monomers using the tail cysteïnes. Mixed disulfides between ER matrix and IgM monomers are reduced when B-cells switch to secretion. The protein that acts as this reductase is unknown.

It is tempting to speculate that smERp is the protein that enables the IgM pentamerization. It would certainly explain why smERp1 functions as a reductase on our model proteins.

Materials & Methods

2D Electrophoresis and mass spectrometry were performed as described previously (Van Anken et al, submitted)

Plasmid construction

A pBluescriptSK+ construct containing the mouse smERp1 (m smERp1) open reading frame (ORF) behind the T7 promotor was generated using IMAGE clone AA475198 (ResGen) which contained the complete ORF coding for m smERp1 in the pT7T3D-Pac vector. The vector pBluescriptSK+ HIV BanII(-tat) was used as vector source. The HIV insert was removed using a XhoI/NotI digestion and the ORF coding for smERp1 was inserted into this vector fragment, yielding pBluescriptSK+ m smERp1.

A tagged version of in smERp1 was generated by inserting an HA tag (YPYDVPDYA) behind the signal sequence cleavage site. In this HA tag an AatII site was introduced. Two PCR fragments were created using the primer pairs M13F/HAR and M13R/HAF (see below). The template for this reaction was clone AA475198. The obtained PCR products were inserted into the pGEM-T vector using the single A overhang. The ORF coding for the HA-tagged version of smERp1 was cloned into the pBluescriptKS+ HAwt vector in two steps. First the fragment containing the start codon was cloned EagI/SpeI into the pBluescriptKS+ vector. Subsequently the construct was completed by inserting the fragment containing the stop codon using the AatII and SphI sites, yielding pBluescriptKS+ HA m smERp1

Primers used in the cloning of pBluescriptKS+ m smERp1_Hatag

| Primer | Sequence |
|---|---|
| HAR | 5'GGACGTCGTATGGGTACCCGGCGCTCCCCAGGATA3' |
| HAF | 5'CGACGTCCCAGACTACGCAGATAGGGTTTCCCTCTCGG3' |
| M13F | 5'GTAAAACGACGGCCAG3' |
| M13R | 5'CAGGAAACAGCTATGAC3' |

Cells, viruses and transfections

I.29μ+ cells were cultured as described previously (Van Anken et al, submitted). HeLa cells were maintained in minimal essential medium (MEM) supplemented with 10% fetal calf serum (FCS) and non essential amino acids. HT1080 cells were cultured in Dulbecco's modified Eagle's medium supplemented 8% FCS. CHO cells were maintained in MEM alpha medium supplemented with 8% FCS. All media were also supplemented with 100 U/ml penicillin, 100 μg streptomycin/ml and 2mM glutaMAX. The cells were cultured at 37°C in 5% (v/v) CO2.

The recombinant vaccinia virus expressing the T7 polymerase was propagated in HeLa cells (VVT7 to infect HeLa cells and VVT7CP to infect CHO cells). Infections were performed with a multiplicity of infection (m.o.i.) of 4. Thirty minutes post-infection, cells were transiently transfected using Lipofectase (HeLa cells) or Lipofectamine 2000 (CHO cells) according to the instructions supplied by the manufacturer. In total 4 μg of DNA and 10 μl of lipid per dish were used in the transfection. All media, FCS, penicillin, streptomycin, glutaMAX, Lipofectase and Lipofectamine 2000 were obtained from Invitrogen.

Immunofluorescence

HeLa cells were grown on coverslips to 80% confluency, infected with vaccinia virus and transfected with pBluescriptKS+ HA m smERp1 as described above. Four hours after infection, cells were fixed with methanol at-20°C for 10 min, washed with 0.3 % BSA in PBS containing calcium and magnesium ($PBS^{++}$) and blocked with 3% BSA in $PBS^{++}$ for 30 min. Cells were incubated with a mixture of primary antibody at a dilution of 1:50

(α-HA-tag, 12CA5) and 1:750 (α-PDI). After washing with 0.3% BSA in $PBS^{++}$, cells were incubated with a 1:500 dilution of secondary antibody (GAM-Cy2 and GAR-Cy3). The cells were washed and mounted with mowiol. Microscopy was performed using a Leitz Aristoplan microscope (Leica), a 3CCD color vision camera module (Donpisha) controlled with the Sony XC-003 software (Sony)

mRNA transcription

The pT7T3D-Pac m smERp1 construct was linearized using EagI. T7 driven polymerase reactions were performed using the Promega riboprobe system in accordance with the manufacturers instruction, yielding m smERp1 mRNA

Permeabilization of HT1080 cells

HT1080 cells were semi-permeabilized essentially as described (Wilson et al. 1995). Cells were grown to 80% confluency in a 75 ml flask. After trypsinization, cells were semi-permeabilized using digitonin (1 μg/ml) in KHM buffer (110 mM KAc, 2 mM MgAc, 20 mM HEPES pH7.2) at 4°C for 5 min. After washing with HEPES buffer

(90 mM KAc, 50 mM HEPES pH7.2) for 10 min at 4oC, endogenous mRNA was digested using micrococcal nuclease, activated by 1mM CaCl2, at RT for 12 min. 40mM EGTA was used to inactivate the nuclease. Cell remnants were washed and resuspended in KHM buffer.

Translation reaction using semi-permeabilized cells

The cytosol of the semi-permeabilized cells was reconstituted with reticulocyte lysate. An in vitro translation mix (0.02 mM amino acids minus Methionine, 8 mM KCl, 5 mM DTT and 4 µCi Redivue Promix $^{35}$S cell labeling mix (Amersham)) was added to each sample. After addition of in smERp1 mRNA, protein translation was initiated at 30°C. The translation reaction was stopped after 30 minutes by adding 1 mM cycloheximide. The oxidizing environment was restored by adding 15 mM oxidized glutathione to allow oxidative folding. Folding reaction were stopped by adding 20 mM N-ethylmaleimide (NEM) and transferring samples to ice to trap free sulfydryl groups. After washing the samples, they were resuspended in KHM buffer and analyzed by SDS-PAGE.

Proteinase protection assay

2 µg protease K/µl and 10 mM CaCl2 were added to the translation reactions in semi-permeabilised cells. Membranes were solubilized with 1% Triton X-100 or not. After 25 min incubation on ice the protease was stopped by adding 2.5 mM PMSF. Samples were analyzed by SDS-PAGE.

Pulse chase analysis

Pulse chase experiments were essentially performed as described by Braakman et al (1991) or by Land et al (2001) CHO (smERp and HA) or HeLa (gp 120) cells were grown in 6 cm dishes. Five hours after transfection, cells were preincubated in methionine and cysteine free medium for 15 minutes and subsequently pulse-labeled with 50 mCi Redivue Promix $^{35}$S cell labeling mix (Amersham) per dish. For the smERp1 folding assay cells were pulse labeled for 5 min in the presence of 5 mM DTT. For the HA and gp 120 folding assays cells were pulse-labeled for 2 minutes without DTT. Chases were started by adding complete medium containing excess of non-radioactively labeled methionine and cysteïne. After various intervals the chase was stopped by transferring the cells to ice and washing with ice-cold HBSS containing 20 mM NEM to alkylate free sulfydryl groups. Cells were lysed in 0.5% Triton X-100 in MNT buffer (20 mM MES, 100 mM NaCl, 30 mM Tris-HCl pH 7.4) containing 20 mM NEM and protease inhibitors (chymostatin, leupeptin, antipain, pepstatin, PMSF and EDTA). Lysates were either used directly for immunoprecipitation or snap-frozen in liquid nitrogen and stored at -80°C.

Immunoprecipitations

Six µl of the polyclonal anti HA antibody (PINDA) or 8 µl polyclonal gp 120 antibody (antibody 16) was incubated with protein A Sepharose beads (Amersham), and 100 µl of the 12CA5 serum was incubated with a 1:1 mixture of protein A and protein G Sepharose beads (Amersham) for 30 minutes at 4 oC. Cell lysates were centrifuged at 16,000 x g at 4 °C to pellet the nuclei. Three hundred µl (smERp1 and HA samples) or 200 µl (gp 120 samples) of the lysate supernatants were added to the beads and incubated overnight at 4°C. The immunoprecipitates were washed with either a buffer containing 1 mM EDTA, 150 mM NaCl, 50 mM Tris-HCl, pH8 (smERp1 samples) or with a buffer containing 0.1% SDS, 0.05% Triton X-100, 0.3M NaCl, 10 mM Tris-HCl, pH 8.6 (HA and gp 120 samples), resuspended in 20 µl TE ( 10 mM Tris HCl, 1 mM EDTA pH 8.6) and separated on SDS-PAGE. The gp120 samples were treated with EndoH as described (Land and Braakman, 2001) to cleave off the N-glycans.

SDS-PAGE and fluorography

Before loading onto SDS-PA gels, loading buffer was added to the samples to a final concentration of 200 mM Tris-HCl pH6.8, 3% SDS, 10% glycerol, 0.004% Bromophenol blue and 1 mM EDTA. Samples were reduced or not using 50 mM DTT and heated to 95oC for 5 min. After cooling, 100 mM NEM was added to the reduced samples and to the non-reduced smERp1 samples to prevent oxidation in gel. The smERp 1 samples were loaded on 15% SDS-PA gels and the HA and gp 120 samples on 7.5% SDS-PA gels for electrophoresis according to Laemmli (Laemmli 1970). Gels were stained with coomassie, neutralized and impregnated with salicylic acid for fluorography and dried. Kodak Biomax MR film or storage phosphor screens (Kodak) were exposed to the gels. Signals were detected with the Biorad Personal Molecular Imager FX and analysed using the Image Quant software (Amersham).

References

Adolf, GR, I Maurer-Fogy, I Kalsner and K Cantell (1990). "Purification and Characterization of Natural Human Interferon w1." The Journal of Biological Chemistry 265(16): 9290-9295.
Braakman, I, H Hoover-Litty, KR Wagner and A Helenius (1991). "Folding of influenza hemagglutinin in the endoplasmic reticulum." J Cell Biol 114(3): 401-411.

Cabibbo, A, M Pagani, M Fabbri, M Rocchi, MR Farmery, NJ Bulleid and R Sitia (2000). "ERO1-L, a human protein that favors disulfide bond formation in the endoplasmic reticulum." J Biol Chem 275(7): 4827-4833.

Desilva, MG, J Lu, G Donadel, WS Modi, H Xie, AL Notkins, MS Lan, (1996). "Characterization and chromosomal localization of a new protein disulfide isomerase, PDIp, highly expressed in human pancreas." DNA Cell Biol 15(1): 9-16

Ellgaard, L and A Helenius (2001). "ER quality control: towards an understanding at the molecular level." Curr Opin Cell Biol 13(4): 431-437.

Elliott, JG, JD Oliver, J Volkmer, R Zimmermann, S High, (1998). " In vitro characterisation of the interaction between newly synthesised proteins and a pancreatic isoform of protein disulphide isomerase." Eur J Biochem 252 (3): 372-377

Flynn, GC, J Pohl, MT Flocco and JE Rothman (1991). "Peptide-binding specificity of the molecular chaperone BiP." Nature 353(6346): 726-730.

Frand, AR and CA Kaiser (1999). "Ero1p oxidizes protein disulfide isomerase in a pathway for disulfide bond formation in the endoplasmic reticulum." Mol Cell 4(4): 469-477.

Gething, MJ and J Sambrook (1992). "Protein folding in the cell." Nature 355(6355): 33-45.

Grauschopf, U, JR Winther, P Korber, T Zander, P Dallinger and JCA Bardwell (1995). "Why is DsbA Such an Oxidizing Disulfide Catalyst?" Cell 83: 947-955.

Guddat, LW, JC Bardwell, T Zander and JL Martin (1997). "The uncharged surface features surrounding the active site of Escherichia coli DsbA are conserved and are implicated in peptide binding." Protein Sci 6(6): 1148-1156.

Haugejorden, SM, M Srinivasan, M Green, (1991). " Analysis of the retention signals of two resident luminal endoplasmic reticulum proteins by in vitro mutagenesis." J Biol Chem 266(10): 6015-6018.

Heijne, GV (1986). "A new method for predicting signal sequence cleavage sites." Nucleic acids research 14 (11): 4683-4690.

Huber-Wunderlich, M and R Glockshuber (1998). "A single dipeptide sequence modulates the redox properties of a whole enzyme family." Fold Des 3(3): 161-171.

Hwang, C, AJ Sinskey and HF Lodish (1992). "Oxidized redox state of glutathione in the endoplasmic reticulum." Science 257(5076): 1496-1502.

Isidoro, C, C Maggioni, M Demoz, A Pizzagalli, AM Fra and R Sitia (1996). "Exposed Thiols Confer Localization in the Endoplasmic Reticulum by Retention Rather than Retrieval." J. Biol. Chem. 271(42): 26138-26142.

Kikuchi, M, E Doi, I Tsujimoto, T Horibe, Y Tsujimoto, (2002). " Functional analysis of human p5, a protein disulfide isomerase homologue." J Biochem (Tokyo) 132(3): 451-455

Laemmli, UK (1970). "Cleavage of structural proteins during the assembly of the head of bacteriophage T4." Nature 227(259): 680-685.

Land, A and I Braakman, (2001). "Folding of the human immunodeficiency virus type 1 envelope glycoprotein in the endoplasmic reticulum." Biochimie 83(8): 783-790

Lyles, MM and HF Gilbert (1991). "Catalysis of the oxidative folding of ribonuclease A by protein disulfide isomerase: pre-steady-state kinetics and the utilization of the oxidizing equivalents of the isomerase." Biochemistry 30 (3): 619-625.

Martoglio, B and B Dobberstein (1998). "Signal sequences: more than just greasy peptides." Trends Cell Biol 8(10): 410-415.

Mazzarella, RA, M Srinivasan, SM Haugejorden and M Green (1990). "ERp72, an abundant luminal endoplasmic reticulum protein, contains three copies of the active site sequences of protein disulfide isomerase." J Biol Chem 265(2): 1094-1101.

Melnick, J, JL Dul and Y Argon (1994). "Sequential interaction of the chaperones BiP and GRP94 with immunoglobulin chains in the endoplasmic reticulum." Nature 370(6488): 373-375.

Molinari, M and A Helenius (1999). "Glycoproteins form mixed disulphides with oxidoreductases during folding in living cells." Nature 402(6757): 90-93.

Munro, S and HRB Pelham (1987). "A C terminal Signal Prevents Secretion of Luminal ER Proteins." Cell: 899-907.

Nagata, K and N Hosokawa (1996). "Regulation and function of collagen-specific molecular chaperone, HSP47." Cell Struct Funct 21(5): 425-430.

Raina, S and D Missiakas (1997). "Making and breaking disulfide bonds." Annu Rev Microbiol 51: 179-202.

Sarti, M, MG Farquhar and RA Orlando (2000). "The receptor-associated protein (RAP) interacts with several resident proteins of the endoplasmic reticulum including a glycoprotein related to actin." Exp Cell Res 260(2): 199-207.

Scheel, AA and HR Pelham (1996). "Purification and characterization of the human KDEL receptor." Biochemistry 35(31): 10203-10209.

Schliephake, DE and A Schimpl (1996). "Blimp-1 overcomes the block in IgM secretion in lipopolysaccharide/anti- mu F(ab')2-co-stimulated B lymphocytes." Eur J Immunol 26(1): 268-271.
Sitia, R, M Neuberger, C Alberini, P Bet, A Fra, C Valetti, G Williams and C Milstein (1990). "Developmental regulation of IgM secretion: the role of the carboxy- terminal cysteïne." Cell 60(5): 781-790.
Tasab, M, L Jenkinson, NJ Bulleid, (2002). " Sequence-specific Recognition of Collagen Triple Helices by the Collagen-specific Molecular Chaperone HSP47." J Biol Chem 277(38): 35007-35012
Turner, CA, Jr., DH Mack and MM Davis (1994). "Blimp-1, a novel zinc finger-containing protein that can drive the maturation of B lymphocytes into immunoglobulin-secreting cells." Cell 77(2): 297-306.
Van Anken, E, E Romijn, C Maggioni, R Sitia, I Braakman and A Heck (submitted). "sequential waves of functionally related proteins are expressed when B cells prepare for antibody secretion.".
Volkmer, J, S Guth, W Nastainczyk, P Knippel, P Klappa, V Gnau, R Zimmermann, (1997). " Pancreas specific protein disulfide isomerase, PDIp, is in transient contact with secretory proteins during late stages of translocation." FEBS Lett 406(3): 291-295
Wiley, DC and JJ Skehel (1987). "The structure and function of the hemagglutinin membrane glycoprotein of influenza virus." Annu Rev Biochem 56: 365-394.
Willnow, TE (1998). "Receptor-associated protein (RAP): a specialized chaperone for endocytic receptors." Biol Chem 379(8-9): 1025-1031.
Wilson, DW, MJ Lewis and HR Pelham (1993). "pH-dependent binding of KDEL to its receptor in vitro." J Biol Chem 268(10): 7465-7468.
Wilson, IA, JJ Skehel and DC Wiley (1981). "Structure of the haemagglutinin membrane glycoprotein of influenza virus at 3 A resolution." Nature 289(5796): 366-373.
Wilson, R, AJ Allen, J Oliver, JL Brookman, S High and NJ Bulleid (1995). "The translocation, folding, assembly and redox-dependent degradation of secretory and membrane proteins in semi-permeabilized mammalian cells." Biochem J 307(Pt 3): 679-687.
Zheng, T and CV Nicchitta (1999). "Structural Determinants for Signal Sequence Function in the Mammalian Endoplasmic Reticulum." The Journal of Biological Chemistry 274(51): 36623-36630.

Example 3

smERp family

We detected an abundant protein in the proteome of activated B cells with an apparent MW of 18 kDa and a pI of 5.4 (Fig. 2 smERp), that was absent in non-activated B-lymphocytes, but which was dramatically upregulated in the course of differentiation (Fig. 3 smERp). The signal of this protein was so abundant at day 3 and 4 of activation, that it accounted for 2% of the total signal in the gel.

The protein spot was excized from gels. After in-gel tryptic digestion, peptides were analysed by MALDI peptide mass mapping and peptide sequences were determined by nanoES tandem MS. The identity of the spot corresponded to a putative ORF in the cDNA with NCBI number AK008016. By comparison of all available ESTs in the NCBI databases, corresponding to AK008016, we determined the consensus sequence of the ORF. The consensus sequence differed from AK008016 at three net. positions: A33C, A92C and G407A, resulting in residue changes L11F, H31P and S136N.

We obtained a cDNA clone from the I.M.A.G.E. consortium (Research Genetics), containing the AK008016 ORF. Sequence analysis of this clone revealed that it was identical to the consensus ORF, derived from the NCBI murine EST database. The ORF contains an ATG codon within a proper initiation context and an in-frame termination codon, predicting a protein of 188 residues.

The PSORT algorithm (Heijne, 1986) predicted that the hydrophobic N-terminus of the 188 residue protein could serve as a cleavable ER-targeting signal, with a cleavage site between G21 and D22 (Fig. 4 smERp). Evidence for this cleavage came from the sequence of peptide D22-K38, that was apparently generated by signal peptidase and trypsin. Depending on conditions and cell type, signal peptide cleavage may occur a number of residues upstream or downstream. After cleavage of the precursor protein, a MW of 18.3 kDa is predicted for the mature 167 residue protein, corresponding to the apparent MW deduced from gel electrophoresis. By peptide sequencing we reached a coverage of 96% for the protein, excized from gel (Fig. 4 smERp). The sequence was identitical to the consensus sequence, as determined from EST databases.

The protein contained a CXXC motif (Fig. 4 smERp), albeit different from the canonical CXXC, found in most ER resident oxidoreductases, such as PDI: CDAC instead of CGHC. No other obvious sequence homology was found with PDI-like proteins. At the C-terminus, the protein contains the tetrapeptide REEL (Fig. 4 smERp), shown previously to mediate ER localization (Munro and Pelham, 1987). This small protein will be referred to hereafter as small ER resident protein 1 (smERp1).

In silico BLAST searches of the working draft of the murine genome (Ensembl) revealed that smERp1 is encoded within four exons on the B3 region of chromosome 18. The first exon encodes the leader peptide and the CXXC motif. The last codon encodes the C-terminus of smERp 1 and the 3' UTR, with a potential polyadenylation signal AATAAA at a position ~250 net downstream of the TAG termination codon (Fig 5 smERp).

smERp1 has a human homolog: "Similar to RIKEN cDNA

2010001M09 gene" with GenBank accesion number BC021275 (77% identity). At the cDNA level this ORF is identical to proapoptotic caspase adaptor protein (PACAP) with accesion number AF338109 (Bonfoco et al., 2001), except that the PACAP cDNA sequence contained a 14 net insertion 178 net downstream of the ATG, resulting in a frameshift. The human smERp1 gene could not be traced in the working draft of the human genome as yet. From all available ESTs at the NCBI databases we could deduce a chicken homolog of smERp1 (48 % identity) Alignments are shown in Fig 6 smERp. The only other ESTs showing high homology to smERp1 were from rat and cow, suggesting smERp1 is exclusive for mammals and avians.

No sequence homology with any known family of proteins could be deduced from database searches. A few other proteins of unknown function, however, appeared similar to smERp1. Thus, smERp1 is a member of a larger smERp family of proteins.

The smERp1 ORF showed 23 % homology with another murine protein, "putative secreted protein ZSIG9", with GenBank accession number AF186115. The alignment is shown in Fig. 7 smERp. This protein shared relevant characteristics with smERp1, having a predicted cleavable leader peptide, a CGAC potential redox-active motif and a putative ER retention signal HDEL. In analogy to smERp1 we named this protein smERp2 (Fig. 8 smERp). The human homolog of smERp2 (98% identity) was annotated as "transmembrane protein 4", a putative type II membrane protein, with GenBank accesion number AB015631. smERp2 homologs are also present in Drosphila melanogaster (26 %) and Caenorhabditis elegans (35 %). Alignment of the smERp2 family is shown in Fig. 9 smERp.

Yet another putative murine protein with GenBank accession number AK005532, which showed 22 % homology with smERp2, we named smERp3 (Fig. 10 smERp). Again, smERp3 contained a predicted N-terminal ER targeting signal, a potential redox-active CEVC motif and a putative ER retention signal PDEL at its C-terminus (Fig. 11 smERp). The human homolog of smERp3 (90 % identity) with GenBank accession number AL035587 was previously annotated as dJ475N16.1 (CTG4A). Other homologs of smERp3 were found in Drosphila melanogaster (35 % identity) and in Caenorhabditis elegans (27 % identity). Alignments are shown in Fig. 12 smERp. Also in Arabidopsis thaliana a smERp homolog was found, but sequence identity with the different smERp subfamilies was about equally low (Fig. 13 smERp).

Overall similarities among the smERps are evident: They all contain a signal peptide, a potential redox-active CXXC motif, 4 other conserved cysteines and a putative ER retention signal. Three domains with highest homology are indicated (Fig. 14 smERp). Initial biochemical characterization suggests smERp1 can function as a reductase (i. e. capable of disrupting disulfide bonds); see example 2. Sequence similarities could imply that all smERps are reductases.

Bonfoco, E., Li, E., Kolbinger, F., and Cooper, N. R. (2001). Characterization of a novel proapoptotic caspase-2- and caspase-9- binding protein, J Biol Chem 276, 29242-50.

Heijne, G. v. (1986). A new method for predicting signal sequence cleavage sites, Nucl Acids Res 14, 4683, 1986.

Munro, S., and Pelham, H. R. (1987). A C-terminal signal prevents secretion of luminal ER proteins, Cell 48, 899-907.

Example 4

ERp43

One spot, showing similar expression kinetics as known ER resident proteins (Fig 15 ERp43), had an apparent MW of 43 kDa and pI of 5.2 (Fig 16 ERp43). It was excized and analysed by MALDI peptide mass mapping after in-gel tryptic digestion. The identity of this spot corresponded to a murine protein referred to at NCBI as: "Similar to hypothetical protein MGC3178", with GenBank accession number BC016252. The peptide mass mapping covered 62 % of the putative protein sequence. The sequence carried hallmarks of ER resident proteins, being a C-terminal tetrapeptide KDEL that mediates ER residency (Munro and Pelham, 1987), and two CGHC redox active sites, similar as in PDI.

However, the predicted MW of 36 kDa did not correspond to the apparent MW of 43 kDa we found and, more importantly, an N-terminal signal sequence needed for targeting to the ER was absent (Heijne, 1986). Re-examining the cDNA sequence, containing the ORF encoding "Similar to hypothetical protein MGC3178", we found an alternative ATG codon within a proper initiation context 165 nct upstream of the predicted initiator ATG. The resulting ORF did contain a hydrophobic N-terminal signal sequence as predicted by the PSORT algorithm, that would be cleaved between residue Ala34 and Gln35 of the precursor protein. After cleavage, a MW of 43 kDa is predicted, which does correspond to the apparent MW. One peptide had a mass that could only be explained by the presence of the N-terminal extension, giving evidence for its existence. Depending on conditions and cell type, signal peptide cleavage may occur a number of residues upstream or downstream. In addition, a third CGHC redox-active site was present in this N-terminal extension.

To determine whether the extended ORF was the consensus sequence for the protein we found in our gels, we compared all available sequences in the NCBI mouse EST database. This alignment revealed two point mutations or sequence errors in the cDNA of the extended ORF of "Similar to hypothetical protein MGC3178": T555C and A882T, resulting in residue changes L171P and, respectively, Q280L. For both substitutions we found mass spectrometrical evidence, reaching a coverage for the protein without its signal sequence of 78% (Fig 17 ERp43). We resequenced a cDNA clone we obtained from the I.M.A.G.E. consortium (ResearchGenetics). The sequence was identical to the consensus sequence we derived from EST databases. The extended and corrected ORF of the protein will be referred to hereafter as ERp43.

In accordance with its putative role as an ER resident protein, both human and murine ESTs primarily originated from secretory tissues such as the liver, the salivary gland, etc. ERp43 homologs are present in Drosophila and man. EST databases reveal that only chordata and arthropods contain ERp43 homologs. The human homolog missed the N-terminus, similarly to murine ERp43. Therefore we reassembled a full length ORF as described above. Also, from ESTs we could reassemble the Xenopus ERp43. Alignments of ERp43 are given in Fig 18 ERp43.

The architecture of ERp43 is remarkably conserved. ERp43 shows high sequence identity within, since it contains three similar domains, homologous to thioredoxin (trx). Computer modelling reveals that all three trx domains show remarkable structural similarities with the a-domain of PDI, including the putative redox-active CGHC motifs (Kemmink J, 1996). We therefore classified ERp43 as a member of the PDI-like family of ER resident oxidoreductases, probably involved in disulfide bond formation and/or isomerization.

In Silico BLAST searches of the working draft sequence of the murine genome allowed the mapping of ERp43 on chromosome 6. The coding sequence is encoded within ten exons (Fig 19 ERp43). Exon 1 2 and 3 encode the signal sequence and the first trx domain; exon 4,5,6 and 7 encode the second trx domain; exon 8, 9 and 10 encode the third trx domain. Exon 10 encodes in addition the 3' UTR including potential polyadenylation sequences. The presence of three ERSEI sequences (ATF6 target (Yoshida H, 2001)) and a UPRE (XBP-1 target (Yoshida H, 2001)) upstream of the initiator ATG, indicate that ERp43 is a UPR target (Fig 20 ERp43).

The three trx domains of ERp43 showed high homology with each other. The closest relative of ERp43 in both the murine and human genome appeared ERp57, more specifically the first trx domain showed high homology with all three ERp43 trx domains (Fig 21 ERp43). From alignments of all PDI a-domain-like trx domains it became evident that the three ERp43 trx domains and the first trx domain of ERp57 as well as the first trx domain of both Pdip and Eug1p from yeast, all shared an additional set of cysteines downstream of the CGHC redox-active site. Interspaced by six residues and in some relatives eight residues (drosophila ERp43 third trx, as well as mpd1 and 2 in yeast), these two cysteines have been conserved in distant relatives within the PDI family from yeast to man. This motif, hereafter referred to as the CX6C motif, was lacking from mammalian PDI itself, P5, PDIp and ERp72. Most often in place of cysteines, serines or alanine residues were found in their sequences, nicely illustrating that nature chooses similar substitutions as many investigators do. Interestingly, ERp44, which has a CXXS active site as opposed to the canonical CXXC (Anelli T, 2002) also contained a single cysteine in the other motif:: CX6A. Schematic representations of a few PDI family members containing the CX6C motif or not are given (fig 22 ERp43).

Modelling of the cysteines of the CX6C motif into the NMR structure of the a-domain of PDI, showed that they are close enough to form a disulfide. This disulfide would close a small loop that is less conserved but it joins sequences that are well conserved among the PDI a-domain-like trx folds. The CX6C motif is juxtaposed to the redoxactive CGHC module. (Fig 23 ERp43). The conservation of the CX6C together with its juxtaposition to the redox-active CXXC motif, suggests that also the CX6C motif has a role in oxidative folding in eukaryotes. Although omnipresent in the eukaryotic kingdoms of life, the CX6C motif has escaped the attention of researchers so far.

Anelli T, A. M., Mezghrani A, Simmen T, Talamo F, Bachi A, Sitia R (2002). ERp44, a novel endoplasmic reticulum folding assistant of the thioredoxin family., EMBO J 21, 835-44.

Heijne, G. v. (1986). A new method for predicting signal sequence cleavage sites, Nucl Acids Res 14, 4683, 1986.

Kemmink J, D. N., Dijkstra K, Nilges M, Creighton TE (1996). Structure determination of the N-terminal thioredoxin-like domain of protein disulfide isomerase using multidimensional heteronuclear 13C/15N NMR spectroscopy., Biochemistry 35, 7684-91.

Munro, S., and Pelham, H. R. (1987). A C-terminal signal prevents secretion of luminal ER proteins, Cell 48, 899-907.

Yoshida H, M. T., Yamamoto A, Okada T, Mori K (2001). XBP1 mRNA is induced by ATF6 and spliced by IRE1 in response to ER stress to produce a highly active transcription factor., Cell 107, 881-91.

Table 1.

Table 1. Protein identity list.

Proteins are listed per functional category. Cluster numbers are indicated for comparison. SwissProt accession numbers, theoretical and apparent molecular weight and pI are indicated, except for IgM and MHC II subunits (because of idiotypic variation). Proteins that were identified by similarity to homologs in other species are marked (*).

**Brief description of the drawings**

Figure 1. Protein sequences of novel ERps.

Figure 2. TwoD gel

Figure 3. Upregulation of smERp 1 during B cell differentiation.

Figure 4. Murine smERp1

Figure 5. sequence

Figure 6. Alignment smERp1

Figure 7. Blast with smERp1

Figure 8. Murine smERp2

Figure 9.Alignment smERp2

Figure 10. Blast with smERp2

Figure 11. Murine smERp3

Figure 12. Alignment with smERp3

Figure 13. Phylogenetic relationship

Figure 14. Alignment of smERp family

Figure 15. Expression kinetics of ERp43 during B cell differentiation

Figure 16. Two D gel.

Figure 17. ERp43 sequence

Figure 18. Alignment ERp43 from different species

Figure 19. Intron/exon boundaries

Figure 20 Promoter region ERp43

Figure 21. Alignment ERp43 vs PDI family

Figure 22. Various coding regions

Figure 23. Modeling of folding variants.

Figure 24. I.29m+ lymphoma cells as a model for B-plasma cell differentiation.

(A) Induction of IgM secretion. After treatment with LPS for the indicated periods, I.29m+ cells were washed and incubated in fresh medium for five hours to follow IgM secretion. Post-nuclear lysates (upper panel) and harvested culture media (lower panel) were run on reducing 10% SDS-PA gels. Proteins were transferred onto nitrocellulose and decorated with a horse radish peroxidase conjugated anti-IgM antiserum. In the upper panel two bands are visible. Unstimulated I.29μ+ cells synthesize approximately equal amounts of μm and μs (Sitia et al., 1987). The former are transported to the cell surface, whilst the latter are degraded intracellularly. The upper band probably corresponds to mature membrane μ chains. The lower band contains mixtures of immature, EndoH sensitive membrane and secretory μ chains (Fagioli and Sitia, 2001; Sitia et al., 1987).
(B) Appearance of large, IgM producing cells upon LPS stimulation. LPS-stimulated (day 4, bottom two panels) or untreated I.29μ+ cells were grown on coverslips, fixed, permeabilized and stained with FiTC-conjugated anti-IgM antibodies. Bars correspond with 10 μm.
(C) Protein concentration of post nuclear lysates was determined. The average of duplicate measurements of two independent experiments was taken for each day to calculate the -fold change compared to day 0.
(D) Changes in size and shape in differentiating I.29μ+ cells. 5 x 105 cells, treated with LPS for the indicated periods, were analyzed by flow cytometry.
(E) Expression of the plasma membrane marker CD138 by LPS-induced I.29μ+ cells. 5 x 105 cells, treated with LPS for the indicated periods as above, were stained with antibodies specific for CD138 (left panel) or CD45 (right panel) and analyzed by flow cytometry.

Figure 25. Proteome changes during I.29μ+ differentiation.
Proteins present in the post-nuclear supernatants of I.29m+ cells that were activated with LPS for the indicated times were quantitated as described in legend to Figure 24C. Similar aliquots were resolved by 2D electrophoresis.

Figure 26. Protein expression clusters correlate to biological function.

(A) Spots were clustered based on expression profiles. Spots are color-coded per expression cluster. The gel of the third day of activation served as the reference gel.
(B) Protein identities are indicated and proteins are color-coded per functional category. For abbreviations see Table 1. The gel of the third day of activation served as the reference gel.

Figure 27. Changes in expression levels per functional category reveal a series of events during differentiation.

(A) Excerpts from 2D gels of each day from a representative B cell activation experiment are shown for a few proteins per functional group. Proteins of interest are marked (+). Their expression kinetics, determined by densitometry, are depicted in histograms on the right. Spot quantity of the day of maximal expression was set at 100%. If proteins were present in more than one spot, all spots are marked and histograms represent the sum of these spots.
(B) For all identified proteins, represented in Table 1, spot quantities at every day of activation were divided by the spot quantity before activation (day 0), to calculate the -fold change of proteins during the course of differentiation. If a protein was represented by more than one spot, the sum of spot quantities was used. The changes of all proteins listed in Table 1 were averaged per functional category. Day 0 was set at 1 and changes in expression level per functional category were plotted logarithmically, to give a similar deviation from the X-axis for a y-fold increase as for a y-fold decrease.

Figure 28. Expansion of the ER precedes the increase in IgM synthesis.

(A) After exposure to LPS for the indicated times, cells were pulse-labeled with radioactive amino acids for 10 min. Post-nuclear supernatants of cell lysates were resolved by one-dimensional SDS-PAGE and newly synthesized proteins quantified by phosphor imaging. Changes in incorporation per day were calculated as described for Figure 4B.
(B) Aliquots of the same samples were analyzed by 2D electrophoresis. Radioactive signals were detected by phosphor imaging, and identified as in Figures 3 and 4. Excerpts of gels show synthesis levels of three ER proteins, calnexin (CNX), GRP94, and calreticulin (CRT), and two IgM subunits, light chain (1-IgL) and heavy chain (m-IgH), at different time points after activation. Proteins of interest are marked (>). Signals were quantified and depicted in histograms (bottom panels). For each spot, the most intense signal (comparing days) was set at 100%.

Figure 29. ER expansion in differentiating I.29μ+ precedes activation of XBP-1.

(A) Nuclei were isolated from differentiating I.29μ+ cells and resuspended in sample buffer. DNA was destroyed by shearing and sonication. The equivalent of 5 x 105 nuclei was loaded per lane. Lysates of MEF cells treated with or without 10 μg/ml tunicamycin for six hours were also analyzed by SDS-PAGE under reducing conditions. Nitrocellulose blots were decorated with rabbit anti-XBP-1 antibodies followed by horseradish peroxidase conjugated goat anti-rabbit and revealed by ECL.

(B) Post nuclear lysates from differentiating I.29μ+ cells were subjected to SDS-PAGE and transfer to nictrocellulose as in (A), followed by decoration with antibodies against J-chain. Lysates from a murine myeloma (NR[μ1] (Sitia et al., 1987)), were analyzed as positive reference.

Figure 30. The identification of smERp1. Lysates of differentiating B-cells were separated on two dimensional gels. The proteome of B cells that were activated for three days is shown (A). Proteins that were upregulated linearly in time are indicated. Excerpts from gels of different days of activation show the regulational pattern of smERp1, marked with + (B). The sequence of smERp1 (C) shows hallmarks of ER resident proteins such as a predicted signal sequence (underlined), a retention signal (bold) and a possible redox active site (boxed). The mouse smERp1 gene is located on region B3 of chromosome 18. Upstream of the start codon a putative blimp promotor is present. The gene consists of 4 exons. Downstream of the stopcodon a poly adenylation sequence is present.

Figure 31. Evolutionary conservation of smERp1. smERp1 homologues are present in Mus musculus, Homo sapiens and Gallus gallus. An alignment of these proteins is shown in A. More distant homologues of smERp1 were identified in other organisms. The evolutionary relationships between these proteins are depicted in B. Evolutionary distances are represented by the length of the horizontal bars. The smERp homologues can be divided into three subfamilies in animals and a single plant variant of smERp, which together form a new protein family.

Figure 32. smERp1 resides in the ER. Endogenous PDI (A) and HA-tagged smERp1 (B), expressed in HeLa cells, were visualized using Immunofluorescence. The overlay is shown in (C). ER residency of cleaved (Cl) smERp1 was confirmed by a proteinase protection assay (D). smERp1 was translated in vitro (lane 1) or in semi-permeabilized cells (lane 2, 3 and 4). Samples were treated with a protease to digest all proteins that are not protected by an intracellular membrane (lane 3). As a control, membranes were solubilized before digestion (lane 4).

Figure 33. smERp1 folding pattern in the SP-system and in vivo. After 30 minutes of translation in semi-permeabilised cells in the presence of DTT, oxidative folding of smERp1 was followed for the indicated timepoints (A). The origin of the ox1 and ox2 forms observed in the SP-system was determined using a two dimensional gel (C). In the first dimension, smERp1 that was chased for 60 minutes was separated on non-reducing SDS-PAGE. After the proteins were reduced in gel, the gel slice was applied onto the second dimension and separated on reducing SDS-PAGE. The folding pattern of smERp1 was also followed in a pulse chase experiment (D). HA-tagged smERp1 was pulse labeled for 5 minutes in the presence of DTT after which the protein was chased for various intervals. The proteins were separated on 15% gels using reducing (panel B and E) and non-reducing (panel A and D) conditions.

Figure 34. The folding of HA is slowed down by coexpression with smERp1. The folding pattern of HA in the absence (panel A and B) and presence (panel C and D) of smERp1 was investigated using a pulse chase assay. The proteins were chased for 2 minutes and chased for various intervals. Proteins were separated on 7.5% reducing (panel B and D) and non-reducing (panel A and C) SDS-PAGE.

Figure 35. smERp1 increases the efficiency with which gp 120 folds. A pulse chase analysis of gp 120 was performed in the absence (panel A and B) and the presence of smERp1 (panel C and D). Samples were separated by 7.5% SDS-PAGE under nonreducing (panel A and C) or reducing conditions (panel B and D). At later chasetimes gp120 was also immunoprecipitated from the culture media and analyzed by 7.5% SDS-PAGE under reducing conditions.

# Table 1.

| Functional category | Cluster | Protein Identity | | Swiss Prot Accesion No | Mr (kDa) | | pI | |
|---|---|---|---|---|---|---|---|---|
| | | | | | gel | theory | gel | theory |
| Cytoskeleton | I | β-ACT | β-actin | P02570 | 43 | 42 | 5.3 | 5.3 |
| | I | α-TUB | α-2 tubulin | P05213 | 56 | 50 | 5.1 | 5.0 |
| | I | β-TUB | β-5 tubulin | P05218 | 54 | 50 | 4.9 | 4.8 |
| Cytosolic chaperones | I | HSC70 | | P08109 | 70 | 71 | 5.4 | 5.4 |
| | II | HSP90α | | P07901 | 85 | 85 | 5.0 | 5.0 |
| | II | HSP90β | | P11499 | 85 | 83 | 5.0 | 5.0 |
| | II | TCPα | TCP-1-α subunit | P11983 | 60 | 60 | 5.8 | 5.8 |
| | II | TCPβ | TCP-1-β subunit | P80314 | 52 | 57 | 6.2 | 6.0 |
| | II | TCPε | TCP-1-ε subunit | P80316 | 60 | 60 | 5.8 | 5.7 |
| | II | TCPθ | TCP-1-θ subunit | P42932 | 60 | 60 | 5.8 | 5.4 |
| Mitochondrial chaperones | II | GRP75 | | P38647 | 72 | 69 | 5.6 | 5.5 |
| | II | HSP60 | | P19226 | 59 | 58 | 5.5 | 5.4 |
| Metabolism | IV | AR | Aldehyde Reductase | P45376 | 38 | 36 | 6.8 | 6.8 |
| | IV | ALADH | δ-Aminolevulinic acid dehydratase | P10518 | 36 | 36 | 6.3 | 6.3 |
| | IV | ENO | α-Enolase | P17182 | 48 | 47 | 6.4 | 6.4 |
| | V | GAPDH | Glyceraldehyde 3-phosphate DHG | P16858 | 33 | 36 | 8.5 | 8.5 |
| | V | OAT | Ornithine AT | P29758 | 45 | 46 | 6.0 | 5.5 |
| | IV | PGDH | Phosphoglycerate DHG | Q61753 | 55 | 56 | 6.3 | 6.4 |
| | III | 6PGL | 6-Phosphogluconolactonase * | Q9CQ60 | 27 | 27 | 5.4 | 5.6 |
| | IV | PSAT | Phosphoserine AT | Q99K85 | 38 | 41 | 8.1 | 8.2 |
| Redox balance | V | ETF-α | Electron transfer flavoprotein-α * | Q99LC5 | 32 | 33 | 7.2 | 7.7 |
| | V | GST-θ2 | Glutathione-S transferase θ2 | Q61133 | 22 | 28 | 7.3 | 7.2 |
| | V | GST-πB | Glutathione-S transferase πB | P19157 | 21 | 24 | 7.8 | 8.1 |
| | V | NUAM | NADH-ubiquinone oxidoreductase * | Q91VD9 | 78 | 77 | 5.2 | 5.2 |
| | V | PRDX3 | Peroxiredoxin-3 | P20108 | 22 | 22 | 5.7 | 5.9 |
| | V | PRDX4 | Peroxiredoxin-4 | O08807 | 24 | 31 | 6.1 | 6.7 |
| | III | PRDX5 | Peroxiredoxin-5 * | Q91WT2 | 25 | 25 | 5.9 | 7.8 |
| | V | SOD-II | Superoxide dismutase II | P09671 | 20 | 22 | 7.5 | 7.7 |
| ER resident proteins | V | AG-IIβ | Alpha-glucosidase II β subunit | O08795 | 85 | 56 | 4.5 | 4.4 |
| | V | BIP | | P20029 | 74 | 70 | 5.1 | 5.0 |
| | III | CNX | Calnexin | P35564 | 90 | 65 | 4.5 | 4.5 |
| | V | CRT | Calreticulin | P14211 | 58 | 46 | 4.3 | 4.3 |
| | V | CALU | Calumenin | O35887 | 45 | 35 | 4.5 | 4.5 |
| | V | ERp29 | | P57759 | 25 | 26 | 6.2 | 5.7 |
| | V | ERp44 | * | Q9D1Q6 | 44 | 44 | 6.1 | 5.1 |
| | V | ERp57 | | P27773 | 58 | 54 | 5.9 | 5.8 |
| | V | ERp72 | | P08003 | 73 | 70 | 5.2 | 5.0 |
| | V | GRP94 | | P08113 | 93 | 90 | 4.7 | 4.7 |
| | V | GRP170 | | Q9JKR6 | 120 | 107 | 5.2 | 5.1 |
| | V | PDI | | P09103 | 58 | 55 | 4.7 | 4.8 |
| | V | PN | * | Q922R8 | 49 | 46 | 5.1 | 5.0 |
| Immune response | V | CE | Cathepsin E | P70269 | 43 | 43 | 4.1 | 4.5 |
| | V | λ-IgL | λ Ig Light chain | | 23 | | 5.9 | |
| | V | μ-IgH | μ Ig Heavy chain | | 73 | | 6.9 | |
| | I | MHC-IIβ | MHC class II β subunit | | 32 | | 5.9 | |

## Claims

1. A method for identifying a protein from a collection of proteins comprising

- providing at least two protein samples wherein said protein samples are derived from related cells that differ in their activation or differentiation state,
- subjecting each of said samples to a protein separation step,
- selecting a protein that displays an altered relative abundance and/or migration in said separation step,
- and identifying said protein.

**2.** A method according to claim 1, further comprising clustering signals of proteins on the basis of similar differences in the relative abundance and/or migration between said samples.

**3.** A method according to claim 2, further comprising correlating said clusters with an activity of a compartment of a cell.

**4.** A method according to claim 3, wherein said compartment comprises the endoplasmic reticulum.

**5.** A method according to any one of claims 1-4, wherein said related cells are derived from a hemopoietic cell lineage, preferably the B-cell lineage.

**6.** A method according to any one of claims 1-5, further comprising isolating said protein.

**7.** A recombinant protein obtainable by a method according to any one of claims 1-6.

**8.** A recombinant protein according to claim 7, comprising a signal for translocation into a cellular compartment.

**9.** A recombinant protein according to claim 8, wherein said cellular compartment comprises an endoplasmic reticulum, a nuclear envellope, a peroxisome, the cytosol, a mitochondrion or a nucleus.

**10.** A recombinant protein according to claim 9, wherein said protein comprises a sequence as depicted in figure 1, 6, 9, 12, 14, 17, 18, 20, 30 or 31.

**11.** A recombinant nucleic acid encoding a protein according to any one of claims 7-10.

**12.** A recombinant protein comprising oxidoreductase activity comprising a $CX_1X_2C$ motif, wherein C comprises a cysteine and wherein $X_1$ or $X_2$ comprises an acidic amino acid.

**13.** A recombinant protein according to claim 12, further comprising a signal sequence for translocation to the endoplasmic reticulum.

**14.** A recombinant protein comprising oxidoreductase activity comprising a $CX_1X_2C$ motif, wherein either $X_1$ or $X_2$ has been changed into an acidic amino acid.

**15.** A recombinant protein according to any one of claims 12-14, wherein said acidic amino acid comprises an aspartic acid or a derivative thereof.

**16.** A recombinant protein comprising oxidoreductase activity comprising a $CX_1X_2C$ motif, wherein said protein is provided with a $C(N)_6C$ motif, wherein said $X_1$ or $X_2$ comprises an acidic or a basic amino acid residue.

**17.** A recombinant protein according to claim 16, wherein said $X_1$ or $X_2$ comprises a basic amino acid residue

**18.** A recombinant protein according to claim 16 or claim 17, wherein said $CX_1X_2C$ and said $C(N)_6C$ motif are present in a region homologous to a sequence of thioredoxin or a thioredoxin fold.

**19.** A recombinant protein comprising oxidoreductase activity comprising a $CX_1X_2C$ motif and a $C(N)_6C$ motif wherein at least one amino acid in said $C(N)\ _6C$ motif is altered such that oxidoreductase activity of the recombinant protein is altered.

**20.** A method for producing a protein of interest in a cell comprising ever-expressing a protein according to any one of claims 10-19 in said cell and producing said protein of interest in said cell.

**21.** A method according to claim 20, wherein said protein of interest comprises at least one cysteine.

**22.** A method according to claim 20 or claim 21, wherein said protein of interest comprises a secreted protein or membrane protein.

**23.** A method according to any one of claims 20-22, wherein said protein of interest comprises an antibody or a functional part, derivative and/or analogue thereof.

**24.** A method according to claim 23, wherein said protein of interest comprises an envelop protein of a virus.

**25.** A method according to any one of claims 20-24, wherein said cell is a mammalian cell.

**26.** A method according to claim 25, wherein said cell is a cell suitable for large scale production of a protein.

**27.** A method according to claim 26, wherein said cell comprises an adenovirus early region 1.

**28.** An expression vector comprising a gene of interest and a protein according to any of claims 7-10, 12-19.

**29.** An isolated or recombinant cell provided with a nucleic acid according to claim 11 or 12, or a recombinant protein according to any one of claims 7-10, 12-19.

**30.** An isolated or recombinant cell according to claim 29, comprising an adenovirus early region 1.

Figure 1 Sequences of some newly identified proteins

murine CRELP:

MHLLLAAAFGLLLLLPPPGAVASRKPTMCQRCRTLVDKFNQGMANTARKN
FGGGNTAWEEKTLSKYEFSEIRLLEIMEGLCDSSDFECNQLLEQQEEQLE
AWWQTLKKEHPNLFEWFCVHTLKACCLPGTYGPDCQECQGGSERPCSGNG
YCSGDGSRQGDGSCQCHTGYKGPLCIDCTDGFFSLQRNETHSICSACDES
CKTCSGPSNKDCIQCEVGWARVEDACVDVDECAAETSPCSDGQYCENVNG
SYTCEDCDSTCVGCTGKGPANCKECIAGYTKESGQCTDIDECSLEEKACK
RKNENCYNVPGSFVCVCPEGFEETEDACVQTAEGKVTEENPTQPPSREDL

human CRELP:

MRLPRRAALGLLPLLLLLPPAPEAAKKPTPCHRCRGLVDKFNQGMVDTAK
KNFGGGNTAWEEKTLSKYESSEIRLLEILEGLCESSDFECNQMLEAQEEH
LEAWWLQLKSEYPDLFEWFCVKTLKVCCSPGTYGPDCLACQGGSQRPCSG
NGHCSGDGSRQGDGSCRCHMGYQGPLCTDCMDGYFSSLRNETHSICTACD
ESCKTCSGLTNRDCGECEVGWVLDEGACVDVDECAAEPPPCSAAQFCKNA
NGSYTCEDCDSSCVGCTGEGPGNCKECISGYAREHGQCADVDECSLAEKT
CVRKNENCYNTPGSYVCVCPDGFEETEDACVPPAEAEATEGESPTQLPSR
EDL

murine CREMP:

MAPLPPRGLVPSLLWCLSLFLSLPGPVWLQPSPPPHPSPRAEPHPCHTCR
ALVDNFNKGLERTIRDNFGGGNTAWEEEKLSKYKDSETRLVEVLEGVCSR
SDFECHRLLELSEELVENWWFHRQQEAPDLFQWLCSDSLKLCCPSGTFGP
SCLPCPGGTERPCGGYGQCEGEGTRGGSGHCDCQAGYGGEACGQCGLGYF
EAERNSSHLVCSACFGPCARCTGPEESHCLQCKKGWALHHLKCVDIDECG
TEQATCGADQFCVNTEGSYECRDCAKACLGCMGAGPGRCKKCSRGYQQVG
SKCLDVDECETVVCPGENEKCENTEGGYRCVCAEGYRQEDGICVKEQVPE
SAGFFAEMTEDEMVVLQQMFFGVIICALATLAAKGDLVFTAIFIGAVAAM
TGYWLSERSDRVLEGFIKGR

human CREMP:

MAPWPPKGLVPAMLWGLSLFLNLPGPIWLQPSPPPQSSPPPQPHPCHTCR

GLVDSFNKGLERTIRDNFGGGNTAWEEENLSKYKDSETRLVEVLEGVCSK
SDFECHRLLELSEELVESWWFHKQQEAPDLFQWLCSDSLKLCCPAGTFGP
SCLPCPGGTERPCGGYGQCEGEGTRGGSGHCDCQAGYGGEACGQCGLGYF
EAERNASHLVCSACFGPCARCSGPEESNCLQCKKGWALHHLKCVDIDECG
TEGANCGADQFCVNTEGSYECRDCAKACLGCMGAGPGRCKKCSPGYQQVG
SKCLDVDECETEVCPGENKQCENTEGGYRCICAEGYKQMEGICVKEQIPE
SAGFFSEMTEDELVVLQQMFFGIIICALATLAAKGDLVFTAIFIGAVAAM
TGYWLSERSDRVLEGFI

human Quiescin-1:

MRRCNSGSGPPPSLLLLLLWLLAVPGANAAPRSALYSPSDPLTLLQADTV
RGAVLGSRSAWAVEFFASWCGHCIAFAPTWKALAEDVKAWRPALYLAALD
CAEETNSAVCRDFNIPGFPTVRFFKAFTKNGSGAVFPVAGADVQTLRERL
IDALESHHDTWPPACPPLEPAKLEEIDGFFARNNEEYLALIFEKGGSYLG
REVALDLSQHKGVAVRRVLNTEANVVRKFGVTDFPSCYLLFRNGSVSRVP
VLMESRSFYTAYLQRLSGLTREAAQTTVAPTTANKIAPTVWKLADRSKIY
MADLESALHYILRIEVGRFPVLEGQRLVALKKFVAVLAKYFPGRPLVQNF
LHSVNEWLKRQKRNKIPYSFFKTALDDRKEGAVLAKKVNWIGCQGSEPHF
RGFPCSLWVLFHFLTVQAARQNVDHSQEAAKAKEVLPAIRGYVHYFFGCR
DCASHFEQMAAASMHRVGSPNAAVLWLWSSHNRVNARLAGAPSEDPQFPK
VQWPPRELCSACHNERLDVPVWDVEATLNFLKAHFSPSNIILDFPAAGSA
ARRDVQNVAAAPELAMGALELESRNSTLDPGKPEMMKSPTNTTPHVPAEG
PEASRPPKLHPGLRAAPGQEPPEHMAELQRNEQEQPLGQWHLSKRDTGAA
LLAESRAEKNRLWGPLEVRRVGRSSKQLVDIPEGQLEARAGRGRGQWLQV
LGGGFSYLDISLCVGLYSLSFMGLLAMYTYFQAKIRALKGHAGHPAA

murine Quiescin-2:

MAAARAVARDPGAYARQPPSLRAARLPRLLFLLAVVAAVGPREGGGARLY
REGSDAVWLLDSGSVRSATGNSSAAWLVQFHSSWCGHCIGYAPTWRALAA
DVRDWAAAIRVAALDCAEEKNQDVCRTYDIHFYPTFRYFKAFTKEFTTGE
NFKGPDRELRTVRQTMIDFLQNHTEGTWPPACPPLDPIQSSDILSFMDSH
SGQYHAIVFESNGSYVGREVILDLIPYENIMVSRALDTDKAFLGTLGITS
VPSCYLIYPNGSHGLVNVAKPLRSFFSSHLKSLPDVRKKSLFLPEKSNKE
EKSEVVVWKEFDRAKLYTADLESGLHYLLRVELAAHRSLAGAQLKTFRDF
VTVVAKLFPGRPAVKKLLETLQEWLANLPLDKIPYNAILDLVNNKMQISG
IFLTSHVKWVGCQGSRLELRGYPCSLWKLFHTLTVQASTHPEALAGTGFE

GHPQAVLQAIRRYIRTFFGCKECGEHFEEMAKESMDSVKTPDQAVLWLWR
KHNMVNSRLAGHLSEDPKFPKVPWPTPDLCPACHEEIKGLDSWNEGQVLL
FLKQHYSRDNLVDAYSVDQGSPGSVLRARPWLGQMARLSHVNLLPHFPVE
EVSSLKPGVLCLKTNKPRSSLGVSKDEHSWSVSLRIGPI

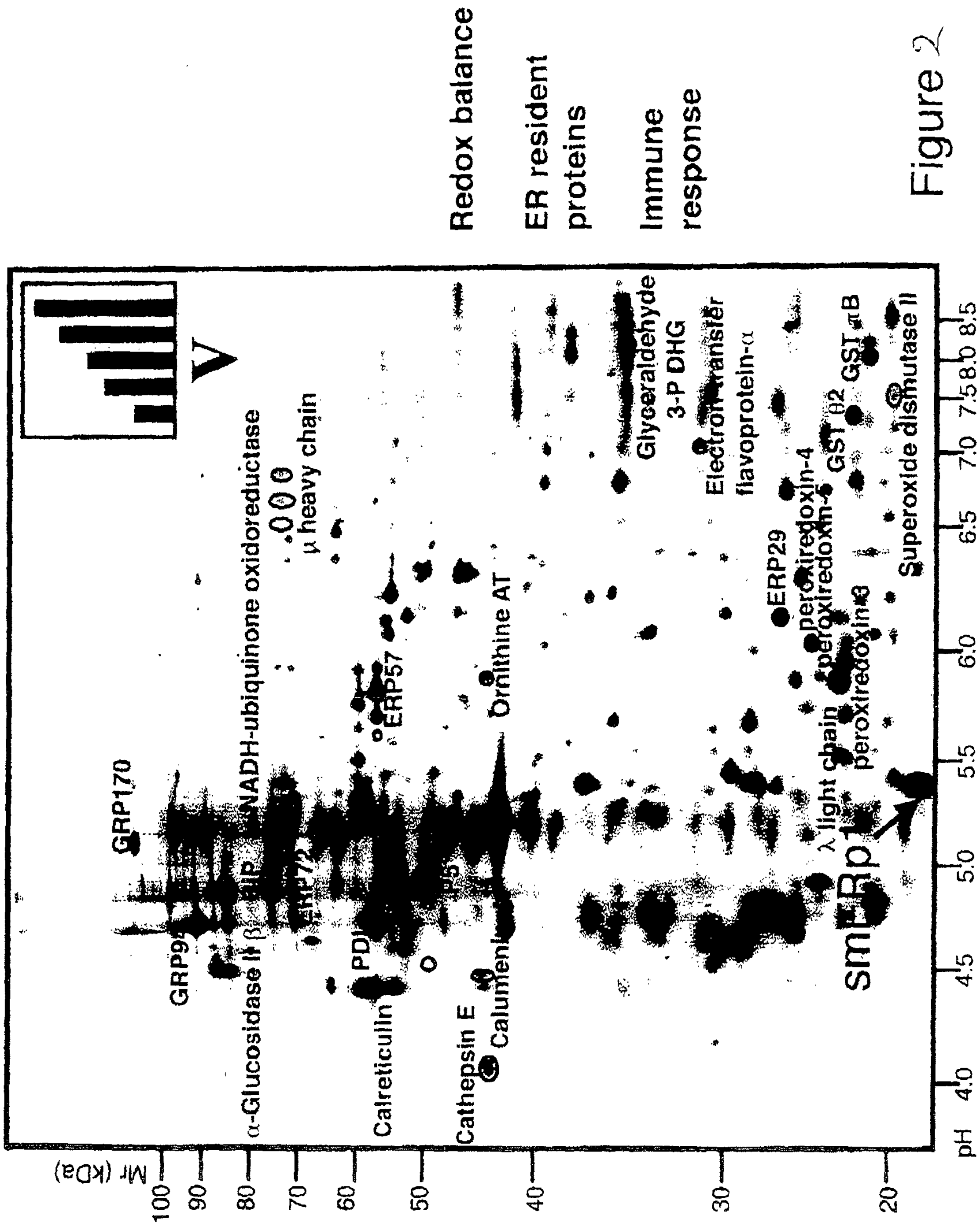
Redox balance
ER resident proteins
Immune response
Mr (kDa)
100
90
80
70
60
50
40
30
20
pH
4.0
4.5
5.0
5.5
6.0
6.5
7.0
7.5
8.0
8.5
GRP170
GRP94
α-Glucosidase II β
NADH-ubiquinone oxidoreductase
μ heavy chain
ERP72
PDI
Calreticulin
ERP57
Cathepsin E
Calumenin
Ornithine AT
Glyceraldehyde 3-P DHG
Electron transfer flavoprotein-α
ERP29
peroxiredoxin-4
peroxiredoxin-3
λ light chain
smERp1
GST θ2
GST πB
Superoxide dismutase II

Figure 2

Upregulation of smERp1 during B cell differentiation

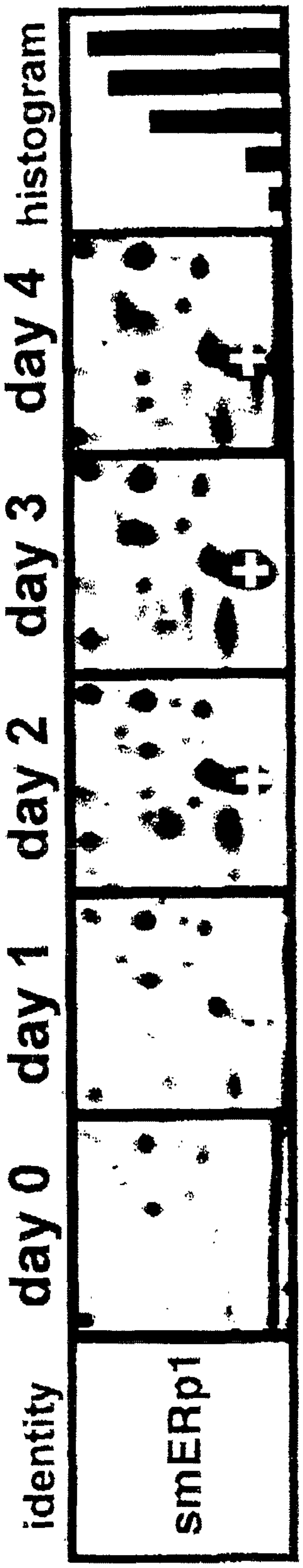


Figure 3

## Murine smERp1:

*MRLPLPLLLLFGCRAILGSAG***DRVSLSASAPTL**

**DDEEKYSAHMPAHLRCDACRAVAFQMGQRLAK***A*

*EAKSH***TPDASGLQELSESTYTDVLDQTCSQNWQ**

**SYGVHEVNQMKRLTGPGLSKGPEPRISVMISGG**

**PWPNRLSKTCFHYLGEFGEDQIYEAYRQGQANL**

**EALLCGGTHGPCSQEILAQREEL**

☐ probable signal sequence
☐ probable redox-active site
☐ probable ER retention signal
Covered by MS in **BOLD**
Not covered by MS in *Italic*

Figure 4

```
gacagcctgtgctccaagaagtaagttcagaggcc

ATGAGACTGCCTCTGCCACTGTTGCTACTGTTCGGGTGCAGGGCTATCCTGGGGAGCGCCGGGGATAGGGTTTCCCTCTCGGCTTCGGCTCCCACACTGGATGATGAAGAGAAGTACTCG
 M  R  L  P  L  P  L  L  L  L  F  G  C  R  A  I  L  G  S  A  G  D  R  V  S  L  S  A  S  A  P  T  L  D  D  E  E  K  Y  S

GCTCATATGCCGGCTCACCTGCGCTGCGATGCCTGCCGGGCTGTGGCCTTCCAG*ATGGGGCAACGTCTGGCGAAAGCAGAGGCTAAATCTCACACTCCAGACGCCAGTGGATTGCAGGAG
 A  H  M  P  A  H  L  R  C  D  A  C  R  A  V  A  F  Q   M  G  Q  R  L  A  K  A  E  A  K  S  H  T  P  D  A  S  G  L  Q  E

CTGAGTGAATCCACGTACACAGATGTCCTGGACCAGACCTGCTCTCAGAACTGGCAGTC*CTATGGAGTTCATGAAGTGAACCAGATGAAGCGTCTCACGGGCCCAGGACTTAGCAAGGGG
 L  S  E  S  T  Y  T  D  V  L  D  Q  T  C  S  Q  N  W  Q  S   Y  G  V  H  E  V  N  Q  M  K  R  L  T  G  P  G  L  S  K  G

CCAGAGCCAAGAATCAGCGTGATGATTTCTGGGGGTCCCTGGCCCAATAG*GCTCTCCAAGACGTGTTTCCACTACCTGGGTGAGTTTGGAGAGGACCAGATCTATGAAGCCTACCGCCAA
 P  E  P  R  I  S  V  M  I  S  G  G  P  W  P  N  R   L  S  K  T  C  F  H  Y  L  G  E  F  G  H  D  Q  I  Y  E  A  Y  R  Q

GGCCAAGCGAATCTGGAGGCGCTGCTCTGTGGGGGCACCCATGGGCCCTGCTCACAGGAGATCCTGGCCCAGAGAGAAGAGCTTTAGtccaacctgctgcacttctggatcttctctaat
 G  Q  A  N  L  E  A  L  L  C  G  G  T  H  G  P  C  S  Q  E  I  L  A  Q  R  E  E  L

tttattattattaatggctgattagaggcaggctctcatcatgtaggccaggctggccttaaacttgtcatcctgctcagcctcgaaagtgctgcatttaagtcctgagccttttgtgc

ttgaccatcctatataatttttcaactgtggtggtggggaggggacagggaagcctgactctagctgtcaatcttctccctccacctctcgatggggtactgggactgaggctgccttt

ctactttcaAATAAAgctttgaaag
```

polyadenylation signal indicated in BLUE
intron position indicated by asterisks

```
Intron/exon bounderies:
1/3883              1734057                                    4032174
ATG - EXON 1 - GGCCTTCCAGGTAAGCTCTG - INTRON 1 - CTCATTCCAGATGGGGCAAC - EXON 2

                    2084958                                    5281209
      EXON 2 - ACTGGCAGTCGTGAGTTGTT - INTRON 2 - TTACCATCAGCTATGGAGTT - EXON 3

                    4095392                                    5489410                602   850
      EXON 3 - GGCCCAATAGGTAATAGCAT - INTRON 3 - GCTCCCCTAGGCTCTCCAAG - EXON 4 - TAG - polyA
```

Figure 5

## Alignment smERp1

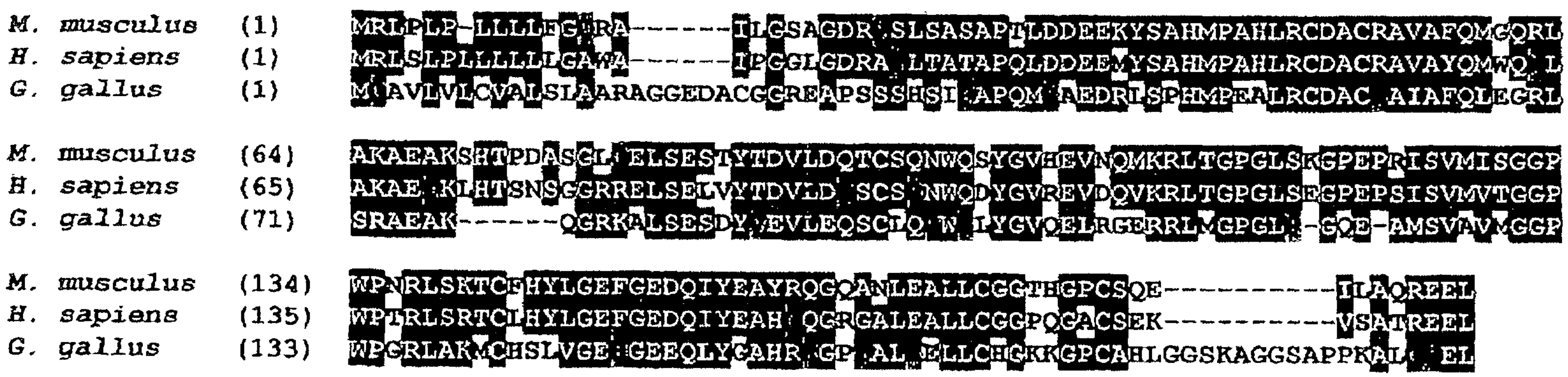

Figure 6

# BLASTP with smERP1, identifying smERp2

```
Score = 33.1 bits (74), Expect = 2.5
Identities = 39/164 (23%)
Positives = 69/164 (41%)
Gaps = 29/164 (17%)

LRCDACRAVAFQMGQRLAKAEAK------SHTPDASGLQEL-------SESTYTDVLDQT
L C ACRA+   ++   +A+ + K      S   +  G Q +       SE+  T++L++
LHCGACRALVDELEWEIARVDPKKTIQMGSFRINPDGSQSVVEVPYARSEAHLTELLEEV

CSQNWQSYG------VHEVNQMKRLTGPGLSKGPE---PRISVMISGGPWPSRLSKTCFH
C +  + YG      H  N ++ ++  G S   +    RI   ISG      L   C
CDR-MKEYGEQIDPSTHRKNYVRVVSRNGESSELDLQGIRIDSDISG-----TLKFACES

YLGEFGEDQIYEAYRQGQANLEALLCGGTHGPCSQEILAQREEL
 + E+ ED++ E + +   N++  LC       C   +    +EL
IVEEY-EDELIEFFSREADNVKDKLCSKRTDLCDHALHRSHDEL
```

Figure 7

**Murine smERp2:**

```
MKGWGWLALLLGVLLGTAWARRSQDLHCGACRA
LVDELEWEIARVDPKKTIQMGSFRINPDGSQSV
VEVPYARSEAHLTELLEEVCDRMKEYGEQIDPS
THRKNYVRVVSRNGESSELDLQGIRIDSDISGT
LKFACESIVEEYEDELIEFFSREADNVKDKLCS
KRTDLCDHALHRSHDEL
```

□ probable signal sequence
□ probable redox-active site
□ probable ER retention signal
C conserved cysteine

Figure 8

## Alignment smERp2

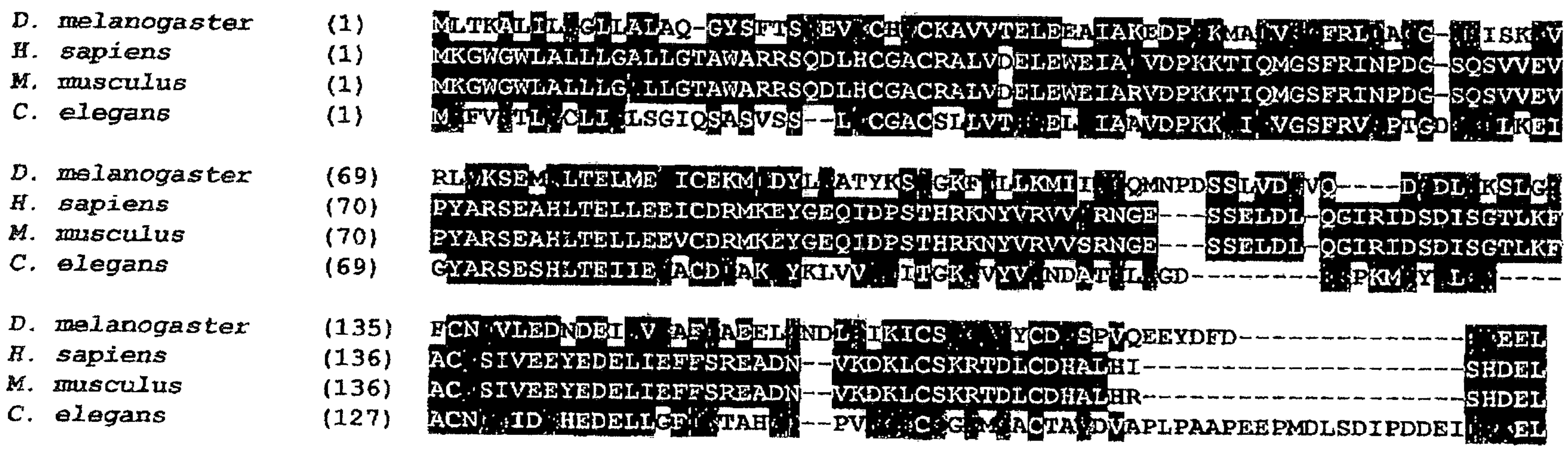


Figure 9

# BLASTP smERp2, identifying smERp3

```
Score = 37.4 bits (85), Expect = 0.12
Identities = 34/151 (22%)
Positives = 70/151 (45%)
Gaps = 17/151 (11%)

CGACRALVDELEWEIARVDPKKTIQMGSFRINPDGSQSVVEVPYARSEAHLTELLEEVCD
C  C+ +  EL+          K +     + I  DG  S V+  Y +S+  L E+ E +C
CEVCKYVAVELKSAFEETGKTKEVIDTGYGIL-DGKGSGVK--YTKSDLRLIEVTETICK

RMKEYGEQIDPSTHRKNYVRVVSRNGES-SELDLQGIRIDSDIS-----------GTLKF
R+ +Y    +  T     + + +S   E+   L  +G+++  DI               LK
RLLDYSLHKE-RTGSNRFAKGMSETFETLHNLVHKGVKVVMDIPYELWNETSAEVADLKK

ACESIVEEYEDELIEFF-SREADNVKDKLCS
 C+ +VEE+E+ + +++ + + +++ + LC+
QCDVLVEEFEEVIEDWYRNHQEEDLTEFLCA
```

Figure 10

**Murine smERp3:**

```
MESMSELAPRCLLFPLLLLLPLLLLPAPKLGPSPAGAEETDWVR
LPSKCEVCKYVAVELKSAFEETGKTKEVIDTGYGILDGKGSGVK
YTKSDLRLIEVTETICKRLLDYSLHKERTGSNRFAKGMSETFET
LHNLVHKGVKVVMDIPYELWNETSAEVADLKKQCDVLVEEFEEV
IEDWYRNHQEEDLTEFLCANHVLKGKDTSCLAERWSGKKGDIAS
LGGKKSKKKRSGVKGSSSGSSKQRKELGGLGEDANAEEEEGVQK
ASPLPHSPPDEL
```

□ probable signal sequence
□ probable redox-active site
□ probable ER retention signal
C conserved cysteine

Figure 11

## Alignment smERp3

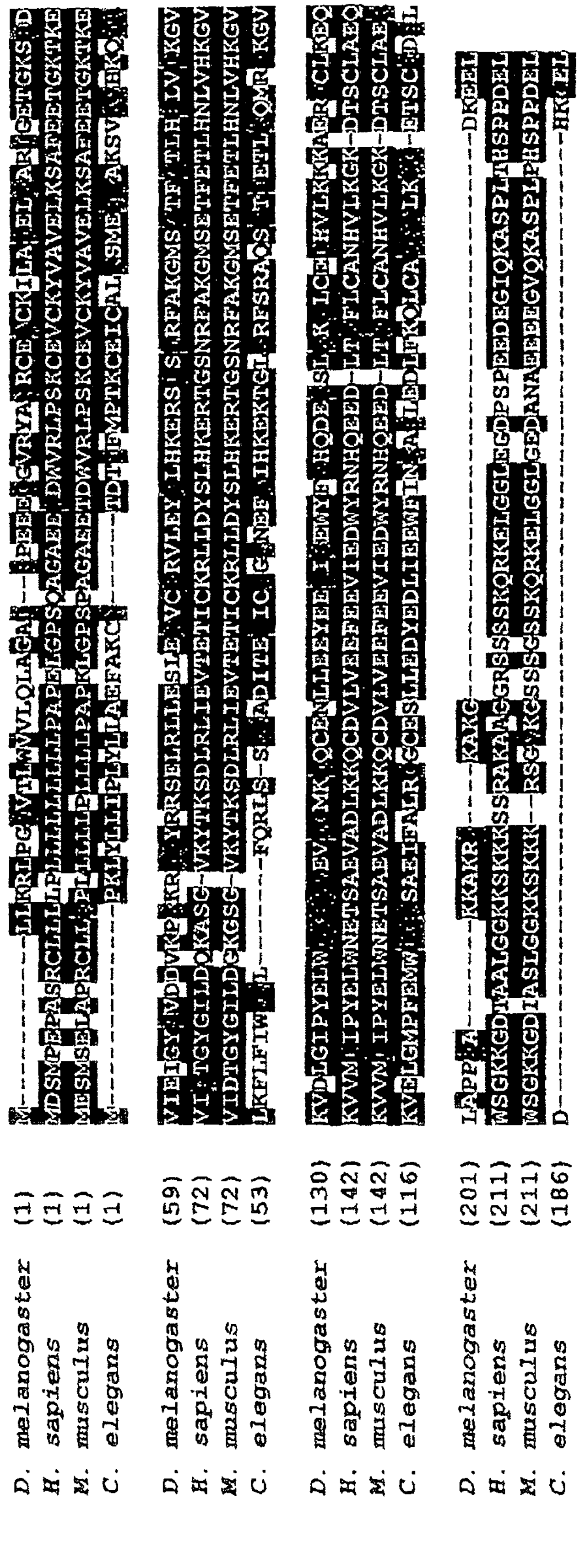

Figure 12

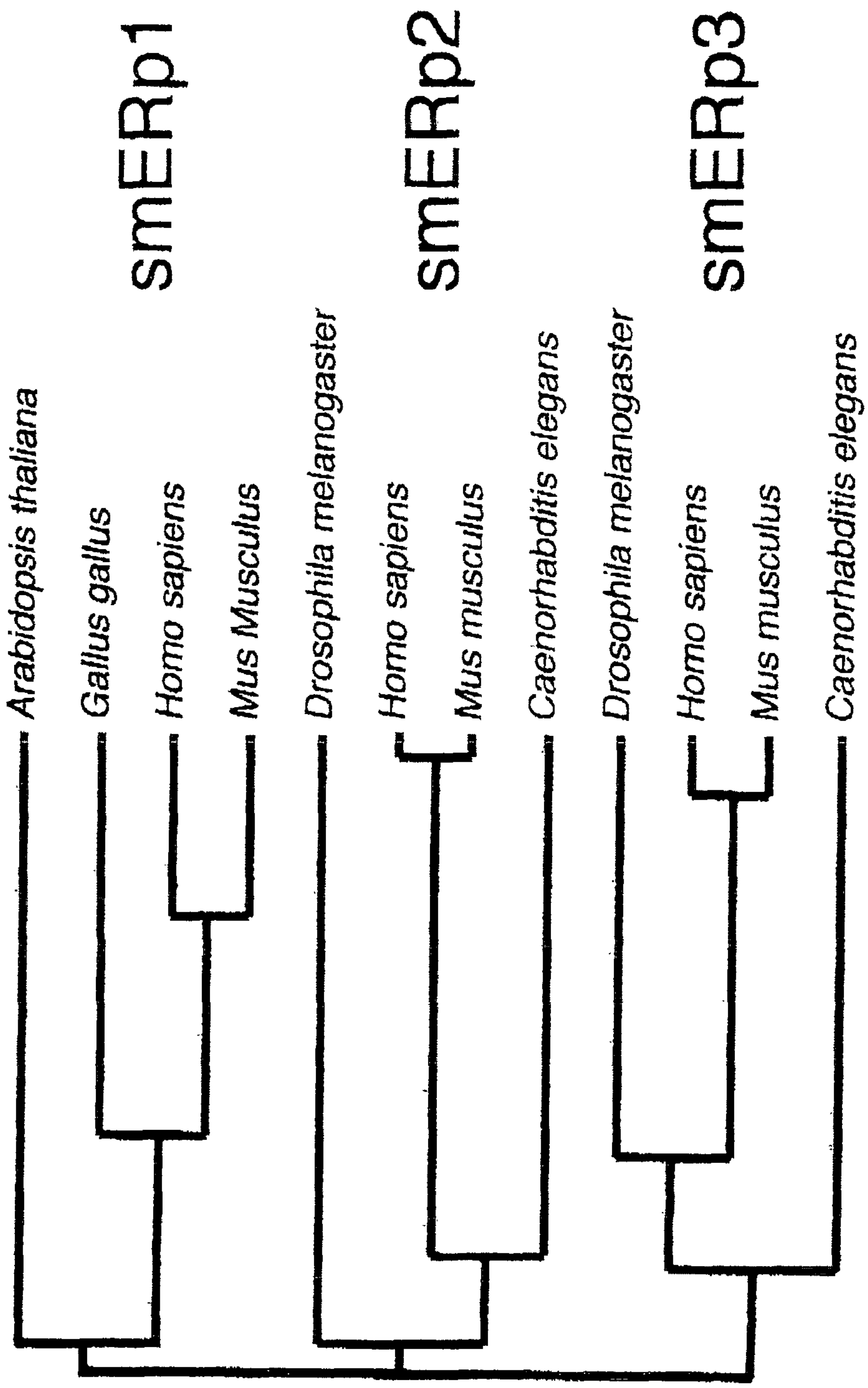

Arabidopsis thaliana
Gallus gallus
Homo sapiens
Mus Musculus
smERp1
Drosophila melanogaster
Homo sapiens
Mus musculus
Caenorhabditis elegans
smERp2
Drosophila melanogaster
Homo sapiens
Mus musculus
Caenorhabditis elegans
smERp3


Figure 13

Alignment of smERp family

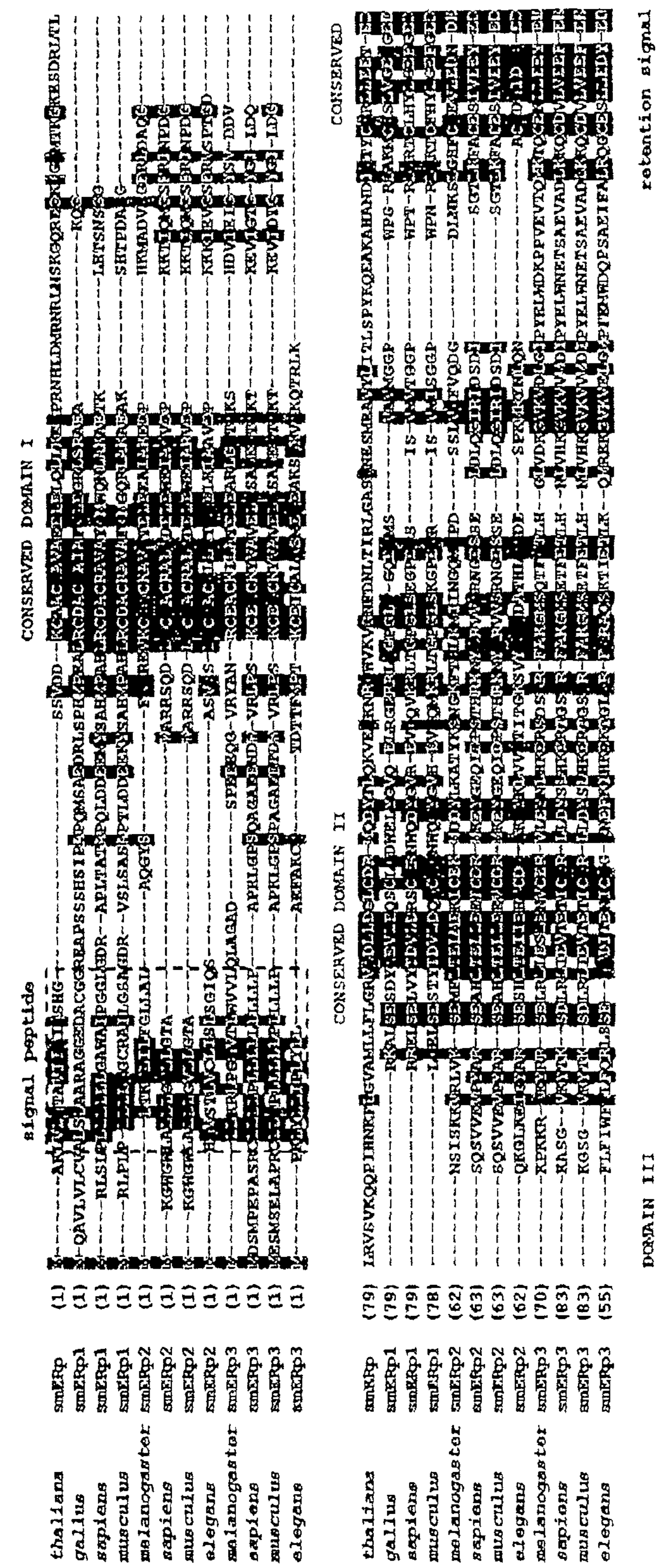


figure 4

**Expression kinetics of ERp43 during B cell differentiation**

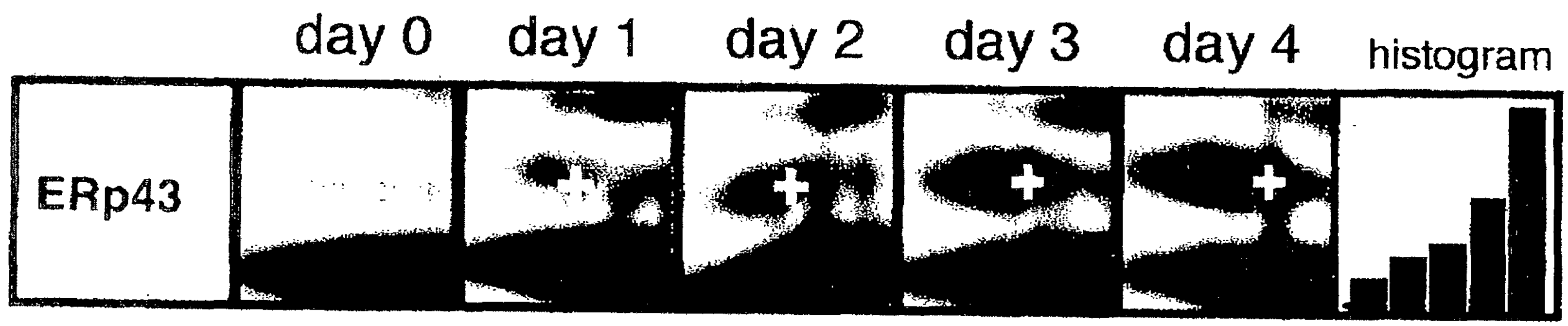


Figure 15

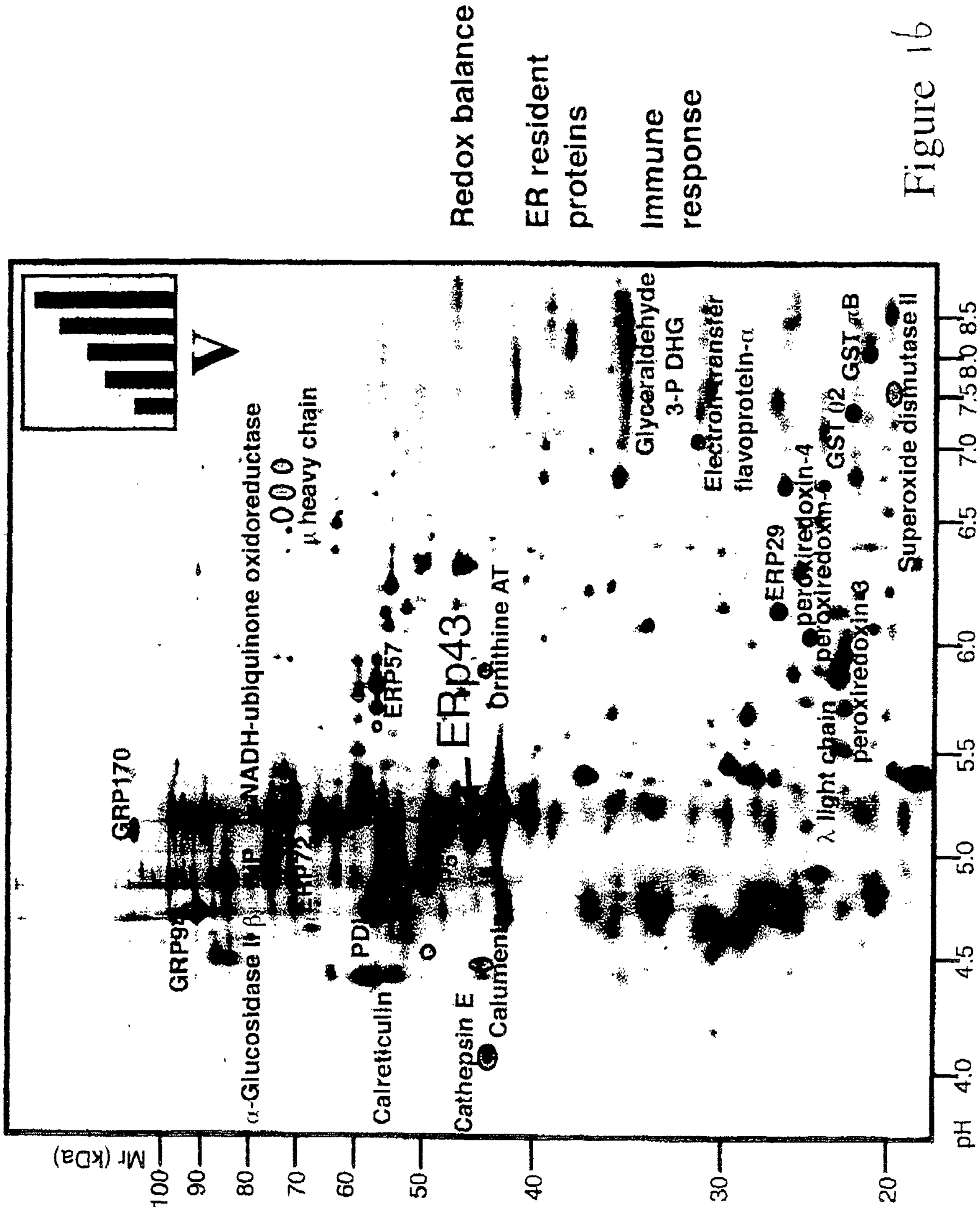
Redox balance
ER resident proteins
Immune response
V
GRP170
GRP94
α-Glucosidase II
NADH-ubiquinone oxidoreductase
μ heavy chain
ERP72
PDI
Calreticulin
ERP57
ERp43
Ornithine AT
Cathepsin E
Calumenin
Glyceraldehyde 3-P DHG
Electron transfer flavoprotein-α
ERP29
peroxiredoxin-4
peroxiredoxin-6
peroxiredoxin-3
λ light chain
GST θ2
GST πB
Superoxide dismutase II
Mr (kDa)
100
90
80
70
60
50
40
30
20
pH
4.0
4.5
5.0
5.5
6.0
6.5
7.0
7.5
8.0
8.5

Figure 16

## ERp43 sequence

```
MPPRPGRLLQPLAGLPALATLLLLLGARKGARAQEVEADSGVEQ
DPHAKHLYTADMFTHGIQSAAHFVMFFAPWCGHCQRLQPTWNDL
GDKYNSMEDAKVYVAKVDCTADSDVCSAQGVRGYPTLKFFKPGQ
EAVKYQGPRDFETLENWMLQTLNEEPATPEPEAEPPRAPELKQG
LYELSANNFELHVSQGNHFIKFFAPWCGHCKALAPTWEQLALGL
EHSETVKIGKVDCTQHYAVCSEHQVRGYPTLLWFRDGKKVDQYK
GKRDLESLRDYVQSQLQGSEAAPETVEPSEAPVMAAEPTGDKGT
VLALTEKSFEDTIAQGITFVKFYAPWCGHCKNLAPTWEELSKKE
FPGLSDVTIAEVDCTAERNVCSKYSVRGYPTLLLFRGGEKVGEH
NGGRDLDSLHSFVLRQAKDEL
```

☐ probable redox-active site
☐ probable ER retention signal
☐ probable signal sequence

Coverage = 78%

Figure 17

# Alignment ERp43 from different species

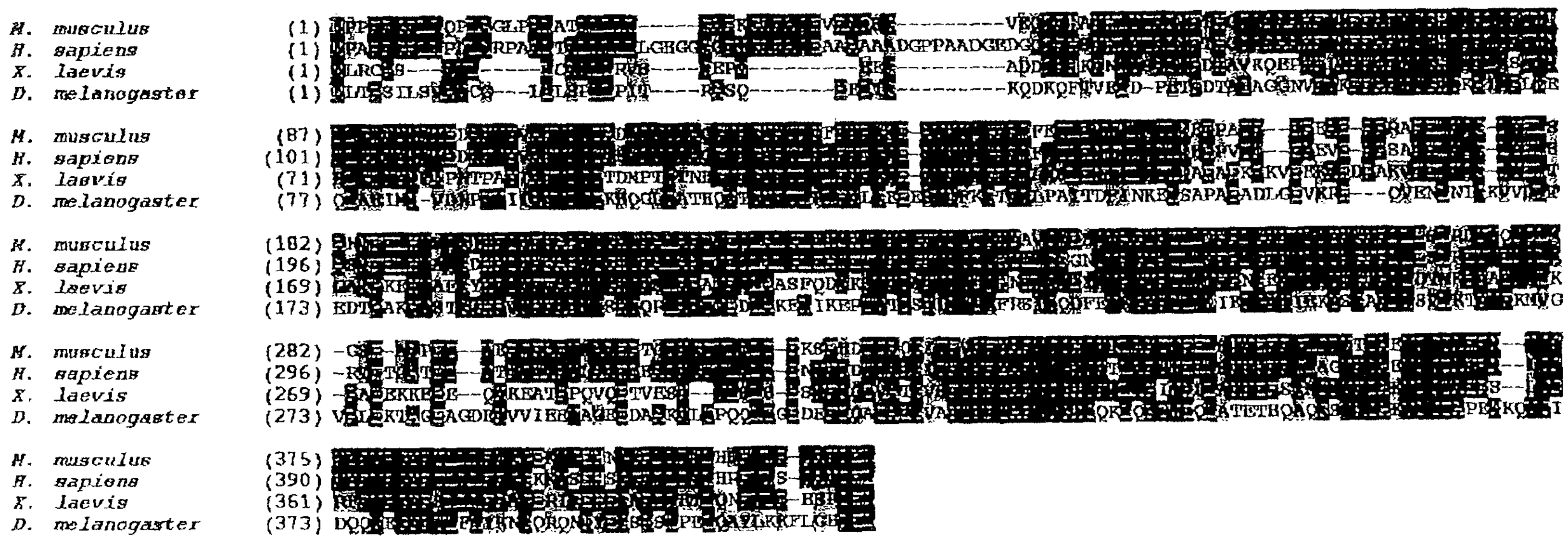


Figure 18

# Intron/Exon boundaries

```
START
1/67905              22267681                          62973223
ATG - EXON 1 - TCGCGCCCTGGTAACTGCAG - INTRON 1 - TTCCCTGTAGGTGTGGACAC - EXON 2

                     37162808                          58583372
      EXON 2 - GATACCCCACGTGAGTAAGG - INTRON 2 - CTTCTTTCAGCCTGAAGTTT - EXON 3

                     47758476                          52657478
      EXON 3 - GGAGCCAGCCGTGAGTGTCC - INTRON 3 - CTTCCATTAGACACCGGAGC - EXON 4

                     57452560                          49735575
      EXON 4 - GTTTCTCAAGGTAAGGATGG - INTRON 4 - TTCCCCGCAGGCAACCACTT - EXON 5

                     69049620                          47475691
      EXON 5 - GATTGGCAAGGTAAGTCCAA - INTRON 5 - CTGTTTTCAGGTTGACTGCA - EXON 6

                     77747387                          46586778
      EXON 6 - TGGCAAGAAGGTATGTCTTT - INTRON 6 - TCTTGGACAGGTGGATCAGT - EXON 7

                     91846441                          44303919
      EXON 7 - GGGTGACAAGGTAGGTGTTT - INTRON 7 - TCCCCTGAAGGGCACTGTGC - EXON 8

                    100144216                         430561002
      EXON 8 - ATGCTCCGTGGTAAGTTGCC - INTRON 8 - TCTCTTCTAGGTGTGGCCAC - EXON 9

                    113142924                         412041132               1254
      EXON 9 - CAAGTACTCGGTGGGTACCT - INTRON 9 - TCCTTTAAAGGTACGAGGTT - EXON 10 - TAG
                                                                              STOP
```

Figure 19

**PROMOTER REGION ERP43**

```
TAAACATTTACAGGTGGACGAACCTAACATGAGAATAGTTACAGATGATG
TGAGTTTCCCTGCCCCTCCCTTTACCACCACCCCCAACTGTTGCTAACCC ERSE-I i
AGGCTTTTGGTTTAGGGAGGCTGAGGCTAATAAAGATCAGGTGATTTTCA ERSE-I ii
GAAGTGTTGCTCAGAAGTCCAATACAAGATGGCCATGTACTCTTCTTACT ERSE-I iii
ATGCCCGTTGCTTTTTCCCAATAAAAGATCACCATATATATGTCTTATGA
TGCCAGCTTCCTTTTCATCGGTACAGAGCATAGCTCTAGGACCTCTCTCA
AGTGTCTAGTGGTTGTTTCGTTCTCCCTAACTTAGCTCCAGGTTTTCAAG
GCAGGGCAGGGAGCTTAGATTGCCACCCGACTTAAAGTTCAAGAGTTGGC
GTGGGTCCATGCGCCCCAGCCCAGGCGTTAGCTCTCACCCAGAACAGCTG
TTGCCATTCATTTTCCTATCCAGGGTGAGTTAGGGCACACAGGGCACTAG
CCAGGGCCTGAAGGAAGGGCCACTTTGACTGCCTTTCCTTTTCAAAGTGA
AGGCAGGGCAGGATAACAGAGGAGGTGCTCTTGGGAAGTGGTTTCATCAC
ACCCCCTGAGGCCAGGCCCTCAAATCACAGTTGAGCTTTGGCTAAGAATC
TGACTTATGCAAGAGCAAACCCCACACACGTTTGCCTGATTTCCTCCTTT
CTGCTGACAGCAGCTCAGCTTGGTCCTAAACTCTCGGGTCTCTTGGTGTC
TGATTAGTTATCAACAGAAGCTGCCACTGTTCCATAGCTTGGCAGCTTGG
CCTCTTCGTCCCTAGCAGGGAAAGGCTGATGAGGAGCTTGGGAAGGAGAA
TGAGCTCAGCGGGATGGAAGGGAACCGGACTGAGATAGCGCTGGAGGTGC
TGCACAAGGACCCCAGGACCTAAGCGACCTCTTCCTGGTGGCTTTCAGCA
GAAGACAGCAAGTTGCTAGGTGAGCCGGCGGGACAGGGGGCGTGGCTCTG
CGAGCGGGGGCGTGATTGAGGCGTGGCTCTGCGGTCGTGGGAGGAGCCGG
GGCGGGAGCGGCGCCGCGGAGGGGACCCGCGGCACGAGCGAGAGCTCGCC
AGCCCCGCCACGATGCCCCCGCGCCCAGGACGCCTCCTCCAGCCGCTGGC E
CGGGCTGCCGGCCCTGGCCACGCTCCTGCTGCTGCTCGGGGCGCGCAAAG X
GCGCCCGGGCCCAGGAGGTGGAAGCGGACAGCGGGGTCGAGCAGGACCCG O
CACGCCAAGCACCTGTATACGGCCGACATGTTCACGCACGGGATCCAGAG N
CGCCGCGCACTTCGTCATGTTCTTCGCGCCCTGGTAACTGCAGCTCCAGC I
AGGCCGGCGGAGCCCGATCTGGGACCGGGGACCAGGGACCTGGAGCCGGG
GACCGGGGAGAGGGCGCGAGGCCGCGCAGGCTGCGCCGGGGGCGTGGGCC
GCGCTCGGGCCTCGCCGCTGACGTGGCGCTAGCCGCCGGCCTGCGAGCTA ATF6/XBP-1
GGAAGGGCGGGCCCCGAGGGGAGACCCGGAAAAGCCTCTCCACCCCGCCA
CACTGCTGGAGTCCAGGCATCCCCCGCTTCTGTCCCCTGCCCAGCTCCCC
AAACTTCCTGTGCTGGCCGGACGCGGCGGGAGCCCGGCGTCCACGTGCCC
TATCCTGAAGGGTTCTTCCTGCTGGCCCATCAATATTGGTTGGATTGGA ERSE-I iv*
AATCTAAGGCCCCCTCAAGATGTGATTGCCCACTTTGTGTGTCGAGGGTA
GGAAAAGATAGCCTAGGTCGGGAACTGAGACTTCCCCGGCCCCTGGAGTC
```

Figure 20

# Alignment ERp43 vs PDI family

```
Mm PDI a       :  APWCGHCKAL----RLAKVDATEESDLAQQY
Mm PDI a'      :  APWCGHCKQL----IIAKMDSTANEVEAVKV
Mm ERP57 a     :  APWCGHCKRL----PLAKVDCTANTNTCNKY
Mm ERP57 a'    :  APWCGHCKNL----VIAKMDATANDVPSPYE
Mm ERP43 a     :  APWCGHCQRL----YVAKVDCTADSDVCSAQ
Mm ERP43 a'    :  APWCGHCKAL----KIGKVDCTQHYAVCSEH
Mm ERP43 a''   :  APWCGHCKNL----TIAEVDCTAERNVCSKY
Mm ERP44 a     :  ADWCRFSQML----VFARVDCDQHSDIAQRY
Sc Pdi1 a      :  APWCGHCKNM----TLAQIDCTENQDLCMEH
Sc Pdi1 a'     :  APWCGHCKRL----LIAKLDHTENDVRGVVI
Sc Eug1 a      :  APWCLHSQIL----PVVQIDCEANSMVCLQQ
Sc Eug1 a'     :  ATWCIHSKRF----LIAEVDSGANDILSFPV
```

Figure 21

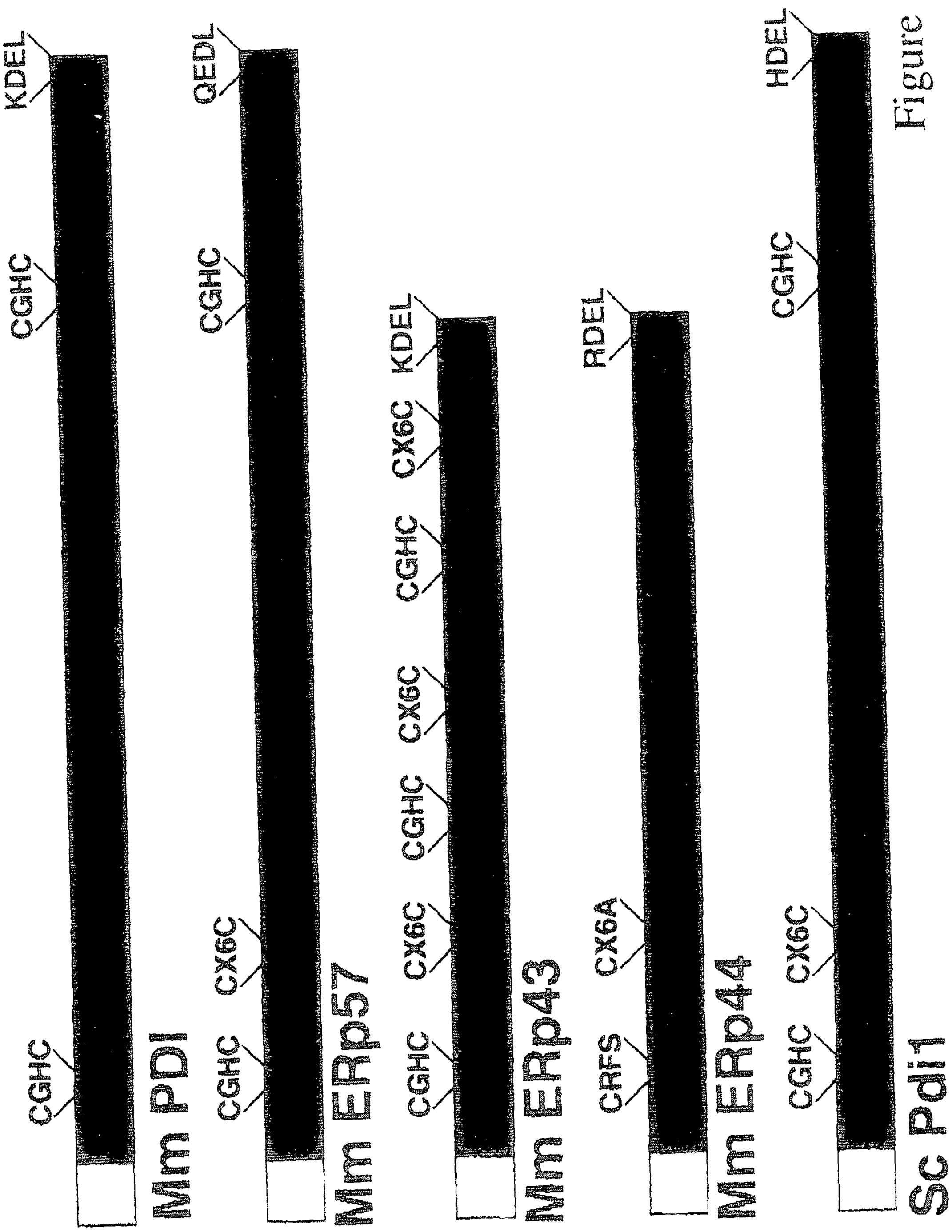
Mm PDI
CGHC
CGHC
KDEL
Mm ERp57
CGHC
CX6C
CGHC
QEDL
Mm ERp43
CGHC
CX6C
CGHC
CX6C
CGHC
CX6C
KDEL
Mm ERp44
CRFS
CX6A
RDEL
Sc Pdi1
CGHC
CX6C
CGHC
HDEL

Figure 22

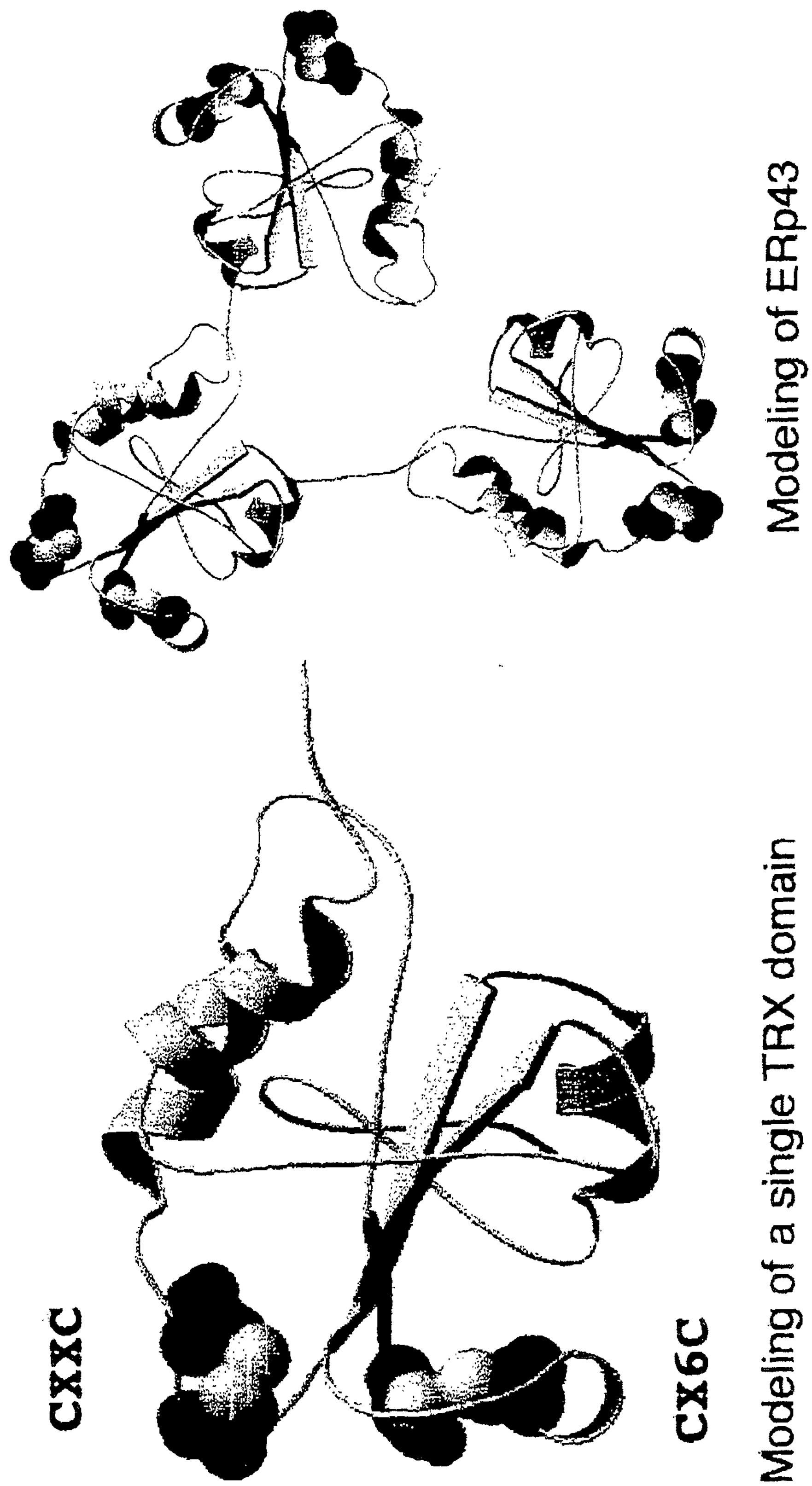


Figure 23

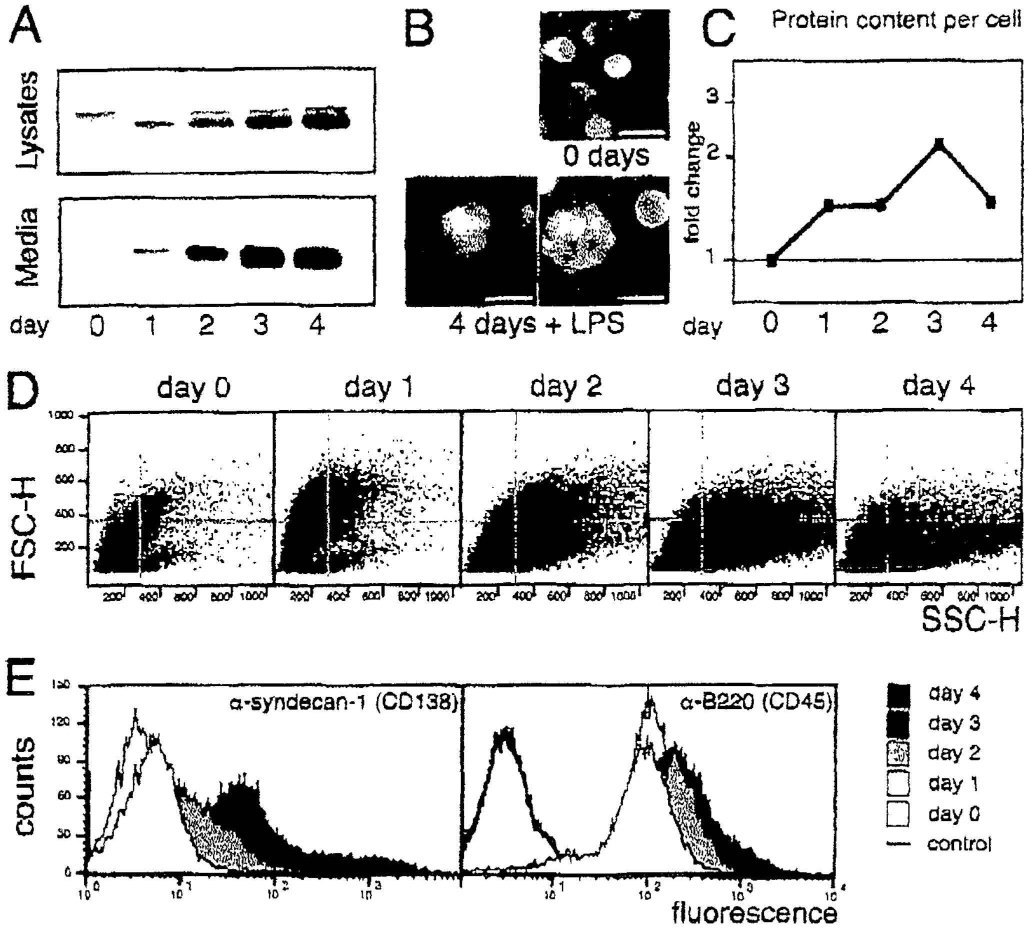
A
Lysates
Media
day 0 1 2 3 4
B
0 days
4 days + LPS
C
Protein content per cell
fold change
day 0 1 2 3 4
D
day 0
day 1
day 2
day 3
day 4
FSC-H
SSC-H
E
α-syndecan-1 (CD138)
α-B220 (CD45)
counts
fluorescence
day 4
day 3
day 2
day 1
day 0
control

Figure 24

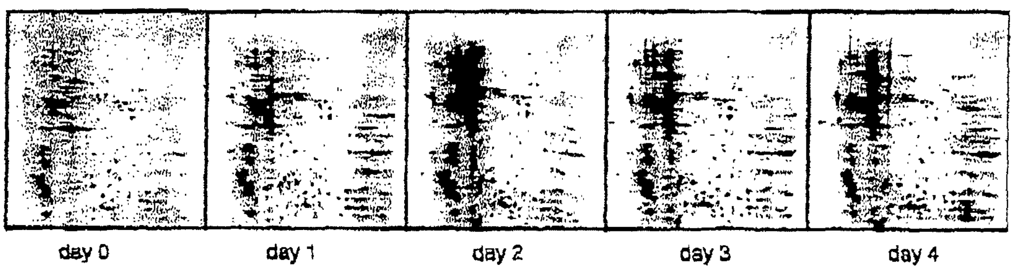


Figure 25

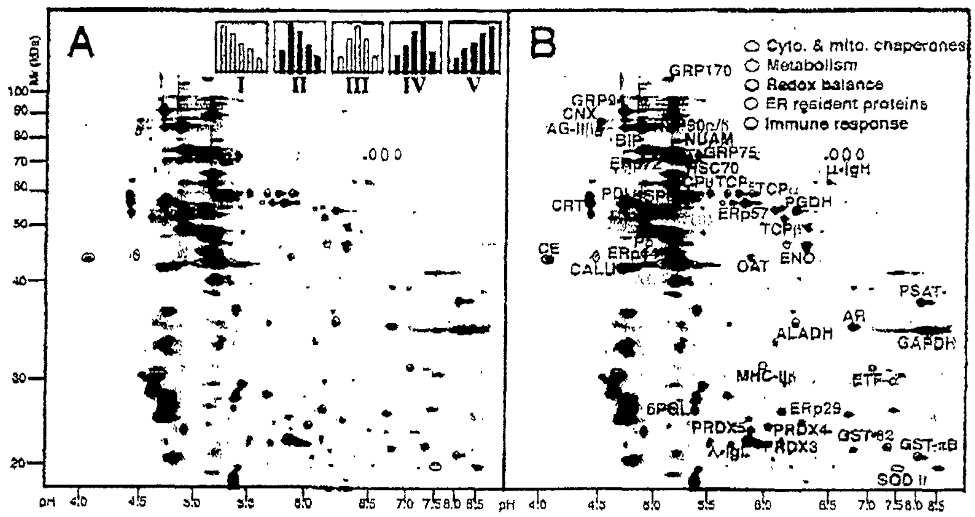
A
B
I
II
III
IV
V
Mr (kDa)
pH
GRP170
Cyto. & mito. chaperones
Metabolism
Redox balance
ER resident proteins
Immune response
CRT
CALU
OAT
ENO
PSAT
AR
ALADH
GAPDH
ERp29
PRDX4
GST-πB

Figure 26

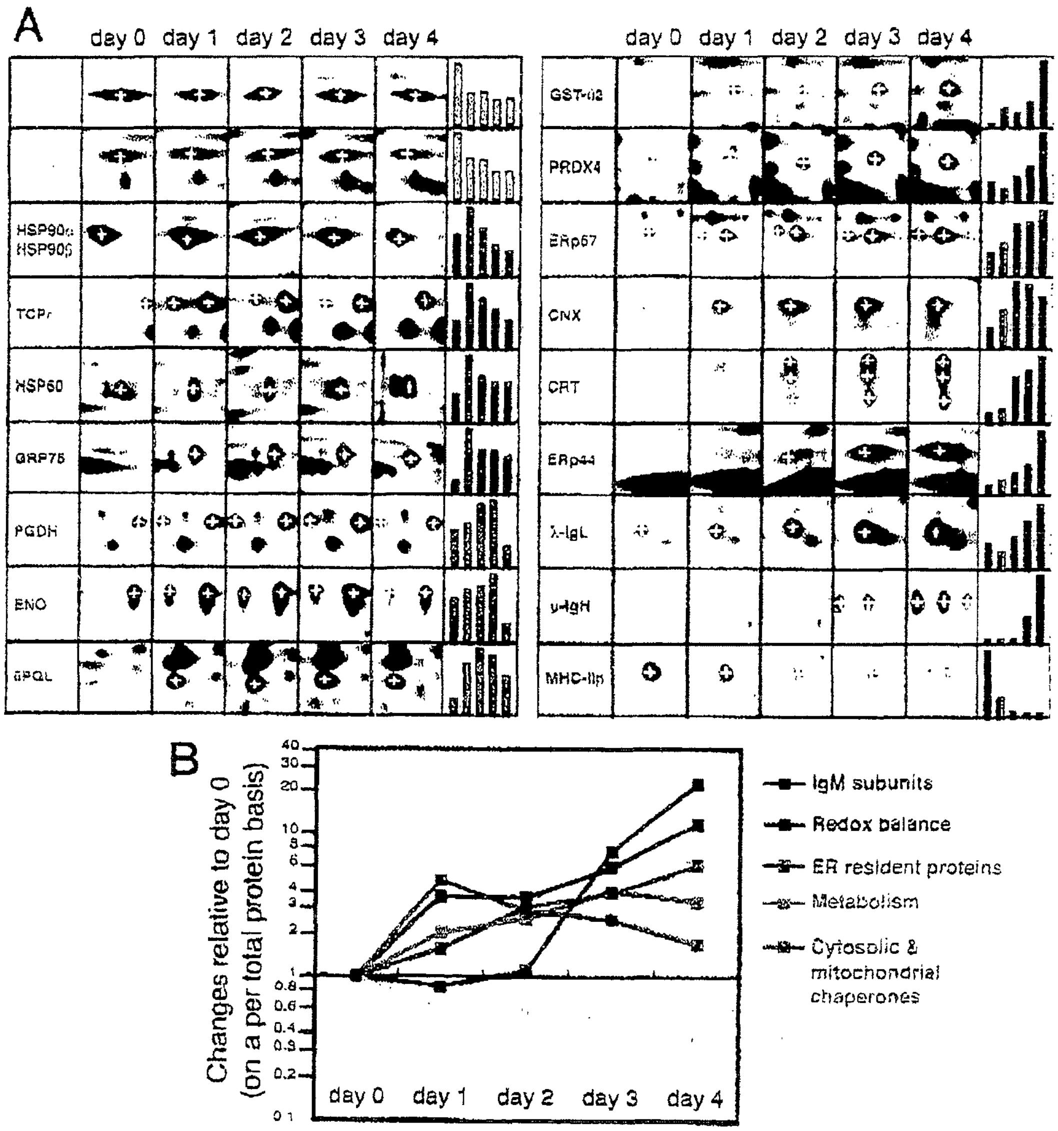
A
day 0 day 1 day 2 day 3 day 4
HSP90α HSP90β
TCPγ
HSP60
GRP78
PGDH
ENO
GPQL
GST-ω2
PRDX4
ERp57
CNX
CRT
ERp44
λ-IgL
μ-IgH
MHC-IIβ
B
Changes relative to day 0 (on a per total protein basis)
40 30 20 10 8 6 4 3 2 1 0.8 0.6 0.4 0.3 0.2 0.1
day 0 day 1 day 2 day 3 day 4
IgM subunits
Redox balance
ER resident proteins
Metabolism
Cytosolic & mitochondrial chaperones

Figure 27

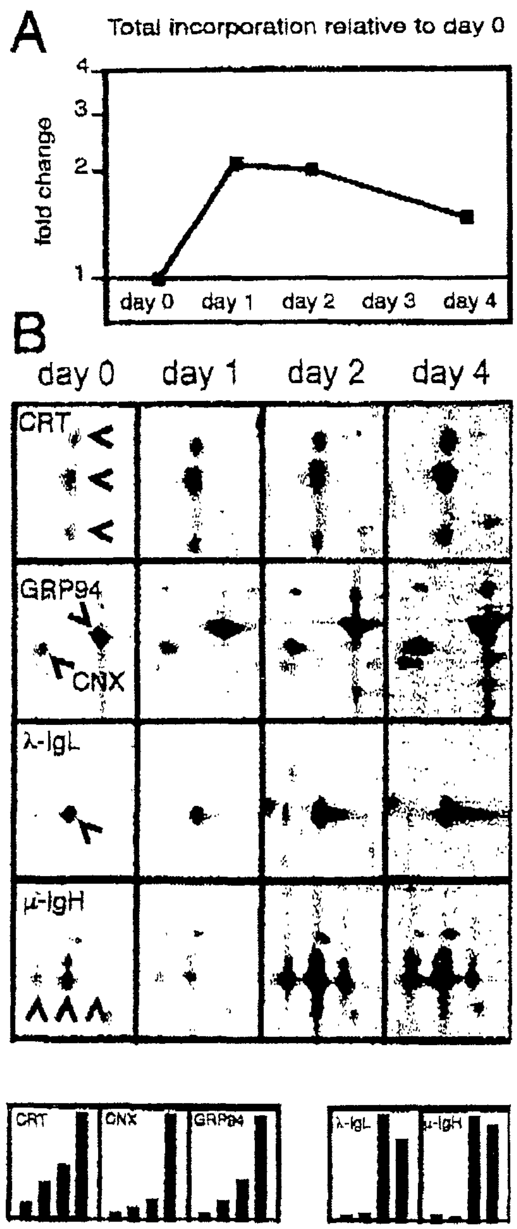
A
Total incorporation relative to day 0
fold change
4
3
2
1
day 0
day 1
day 2
day 3
day 4
B
day 0
day 1
day 2
day 4
CRT
GRP94
CNX
λ-IgL
μ-IgH
CRT
CNX
GRP94
λ-IgL
μ-IgH

Figure 28

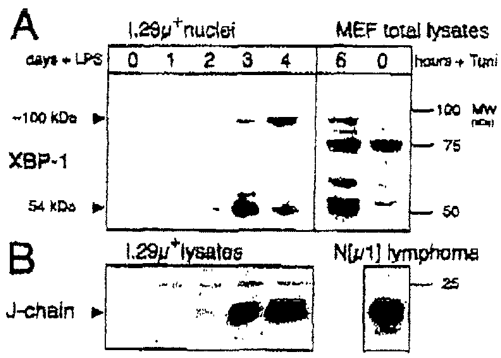
A
I.29μ⁺nuclei
MEF total lysates
days + LPS
0 1 2 3 4
6 0
hours + Tuni
~100 KDa
XBP-1
54 KDa
100 MW
75
50
B
I.29μ⁺lysates
N[μ1] lymphoma
J-chain
25

Figure 29

Figure 30

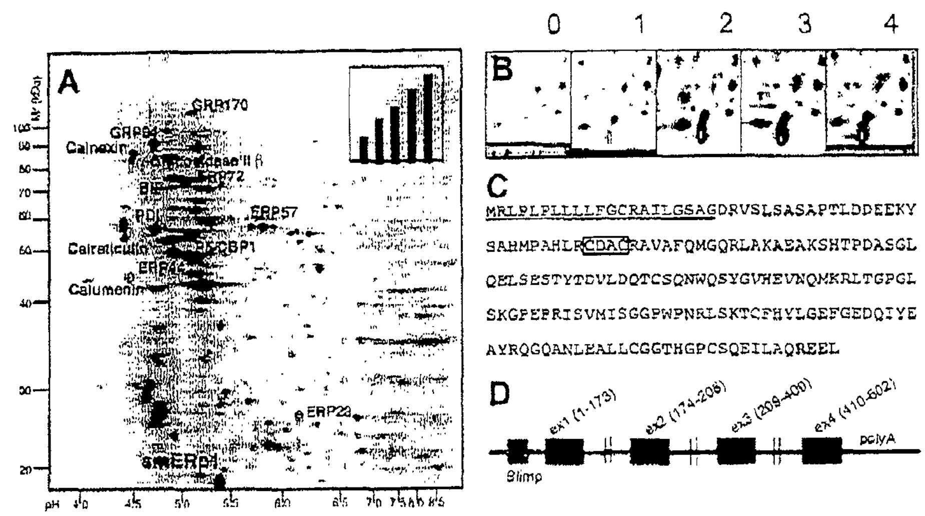
A
GRP170
Calnexin
ERP72
ERP57
PDI
Calreticulin
ERP44
Calumenin
ERP28
40
30
20
pH
B
0
1
2
3
4
C
MRLPLPLLLLFGCRAILGSAGDRVSLSASAPTLDDEEKY
SAHMPAHLRCDACRAVAFQMGQRLAKAEAKSHTPDASGL
QELSESTYTDVLDQTCSQNWQSYGVHEVNQMKRLTGPGL
SKGPEPRISVMISGGPWPNRLSKTCFHVLGEFGEDQIYE
AYRQGQANLEALLCGGTHGPCSQEILAQREEL
D
ex1 (1-173)
ex2 (174-208)
ex3 (209-400)
ex4 (410-602)
polyA
Slimp

Figure 31

A

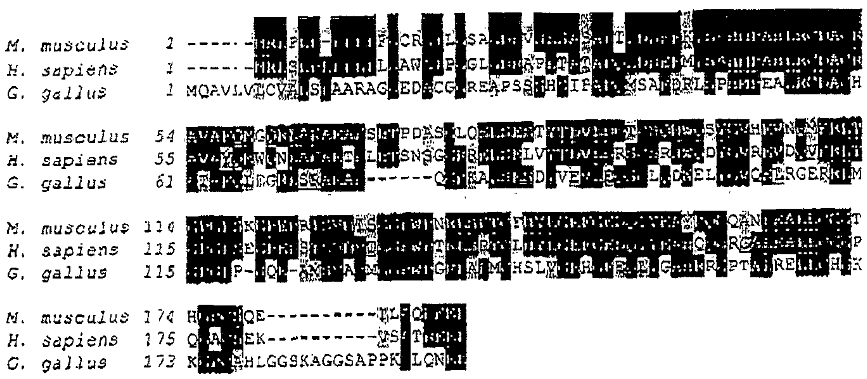


B

smERp1 subfamily

smERp2 subfamily

smERp3 subfamily

Figure 32

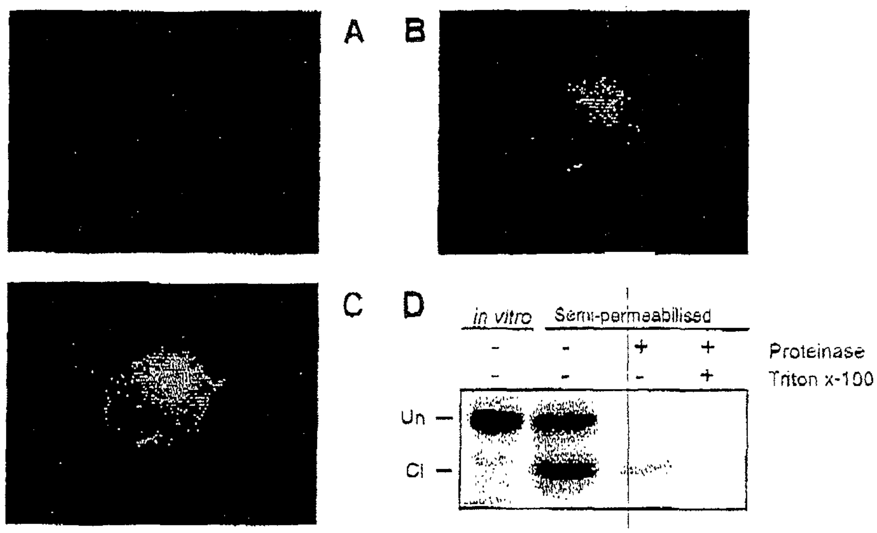
A
B
C
D
in vitro
Semi-permeabilised
Proteinase
Triton x-100
Un
Cl

Figure 33

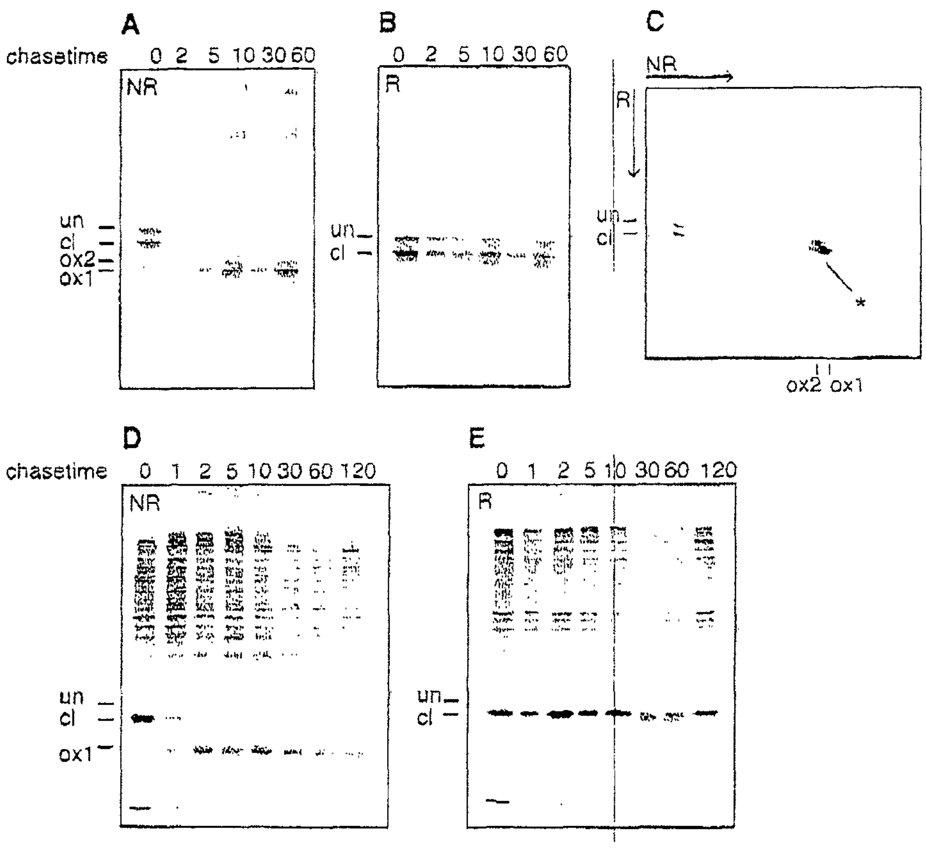
A
chasetime 0 2 5 10 30 60
NR
un
cl
ox2
ox1
B
0 2 5 10 30 60
R
un
cl
C
NR
R
un
cl
*
ox2 ox1
D
chasetime 0 1 2 5 10 30 60 120
NR
un
cl
ox1
E
0 1 2 5 10 30 60 120
R
un
cl

Figure 34

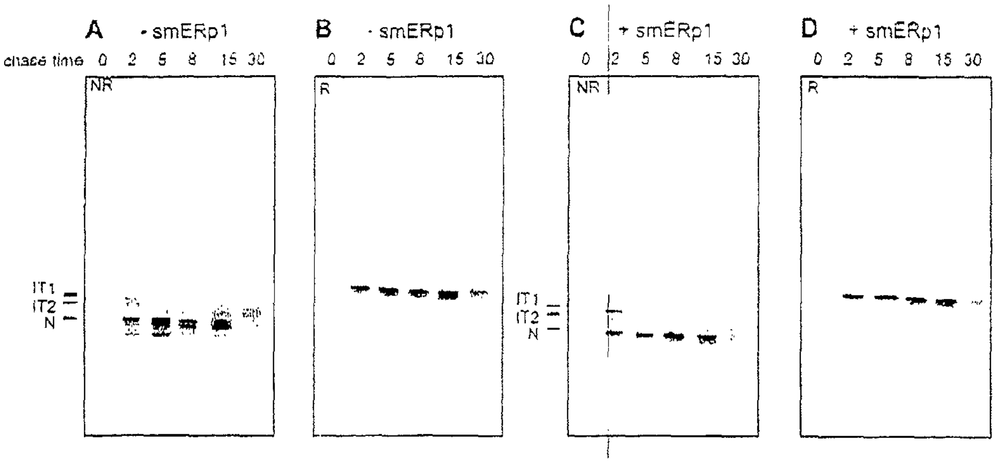
A - smERp1
B - smERp1
C + smERp1
D + smERp1
chase time 0 2 5 8 15 30
0 2 5 8 15 30
0 2 5 8 15 30
0 2 5 8 15 30
NR
R
NR
R
IT1
IT2
N
IT1
IT2
N

Figure 35

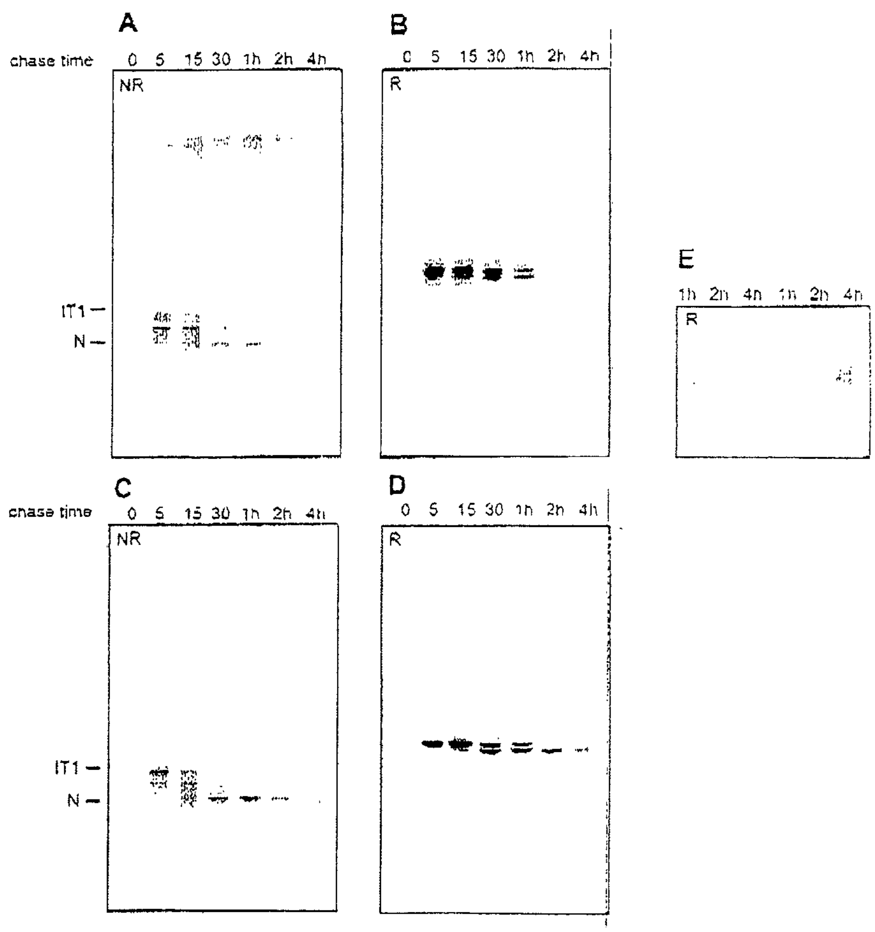
A
chase time
0 5 15 30 1h 2h 4h
NR
IT1
N
B
0 5 15 30 1h 2h 4h
R
E
1h 2h 4h 1h 2h 4h
R
C
chase time
0 5 15 30 1h 2h 4h
NR
IT1
N
D
0 5 15 30 1h 2h 4h
R

European Patent Office

# EUROPEAN SEARCH REPORT

Application Number
EP 03 07 5460

| Category | Citation of document with indication, where appropriate, of relevant passages | Relevant to claim | CLASSIFICATION OF THE APPLICATION (Int.Cl.7) |
|---|---|---|---|
| X | LINDQUIST JONATHAN A ET AL: "ER-60, a chaperone with thiol-dependent reductase activity involved in MHC class I assembly." EMBO (EUROPEAN MOLECULAR BIOLOGY ORGANIZATION) JOURNAL, vol. 17, no. 8, 15 April 1998 (1998-04-15), pages 2186-2195, XP002246985 ISSN: 0261-4189 * the whole document * --- | 1-6 | C07K14/47 G01N27/447 |
| X | WO 98 23950 A (BRUCE JAMES ALEXANDER ;OXFORD GLYCOSCIENCES UK LTD (GB); PLATT ALB) 4 June 1998 (1998-06-04) * the whole document * ----- | 1,2,6 | |
| | | | TECHNICAL FIELDS SEARCHED (Int.Cl.7) |
| | | | C07K G01N |

~~The present search report has been drawn up for all claims~~

| Place of search | Date of completion of the search | Examiner |
|---|---|---|
| MUNICH | 9 July 2003 | Schwachtgen, J-L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number
EP 03 07 5460

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-7

European Patent Office

**LACK OF UNITY OF INVENTION SHEET B**

Application Number
EP 03 07 5460

The Search Division considers that the present European patent application does not comply with the requirements of unity of invention and relates to several inventions or groups of inventions, namely:

1. Claims: 1-7

   a method for identifying a protein from a collection of poteins.

2. Claims: 7-30 (partially)

   a recombinant protein comprising a sequence as depicted in figure 1. Subject-matter related thereto.

3. Claims: 7-30 (partially)

   a recombinant protein comprising a sequence as depicted in figure 6. Subject-matter related thereto.

4. Claims: 7-30 (partially)

   a recombinant protein comprising a sequence as depicted in figure 9. Subject-matter related thereto.

5. Claims: 7-30 (partially)

   a recombinant protein comprising a sequence as depicted in figure 12. Subject-matter related thereto.

6. Claims: 7-30 (partially)

   a recombinant protein comprising a sequence as depicted in figure 14. Subject-matter related thereto.

7. Claims: 7-30 (partially)

   a recombinant protein comprising a sequence as depicted in figure 17. Subject-matter related thereto.

8. Claims: 7-30 (partially)

   a recombinant protein comprising a sequence as depicted in figure 18. Subject-matter related thereto.

9. Claims: 7-30 (partially)

   a recombinant protein comprising a sequence as depicted in

European Patent Office

## LACK OF UNITY OF INVENTION SHEET B

Application Number

EP 03 07 5460

The Search Division considers that the present European patent application does not comply with the requirements of unity of invention and relates to several inventions or groups of inventions, namely:

figure 20. Subject-matter related thereto.

10. Claims: 7-30 (partially)

a recombinant protein comprising a sequence as depicted in figure 30. Subject-matter related thereto.

11. Claims: 7-30 (partially)

a recombinant protein comprising a sequence as depicted in figure 31. Subject-matter related thereto.

## ANNEX TO THE EUROPEAN SEARCH REPORT ON EUROPEAN PATENT APPLICATION NO. EP 03 07 5460

This annex lists the patent family membersrelating to the patent documents cited in the above-mentioned European search report.
The members are as contained in the European Patent Office EDP file on
The European Patent Office is in no way liable for these particulars which are merely given for the purpose of information.

09-07-2003

| Patent document cited in search report | Publication date | Patent family member(s) | Publication date |
|---|---|---|---|
| WO 9823950 A | 04-06-1998 | AT 236392 T | 15-04-2003 |
| | | AU 713259 B2 | 25-11-1999 |
| | | AU 5230098 A | 22-06-1998 |
| | | CN 1242076 A ,B | 19-01-2000 |
| | | DE 69720505 D1 | 08-05-2003 |
| | | DK 941477 T3 | 28-07-2003 |
| | | EP 1302768 A2 | 16-04-2003 |
| | | EP 0941477 A1 | 15-09-1999 |
| | | WO 9823950 A1 | 04-06-1998 |
| | | JP 2001504938 T | 10-04-2001 |
| | | NO 992583 A | 28-07-1999 |
| | | US 2002191825 A1 | 19-12-2002 |
| | | US 6278794 B1 | 21-08-2001 |
| | | US 6480618 B1 | 12-11-2002 |
| | | US 6064754 A | 16-05-2000 |
| | | ZA 9710792 A | 01-12-1998 |

EPO FORM P0459

For more details about this annex : see Official Journal of the European Patent Office, No. 12/82

SEQUENCE LISTING

<110> Universiteit Utrecht Holding B.V.

<120> Method for the characterization of proteins, isolated proteins and uses therefore

<130> P62915EP00

<140> EP 03075460.0
<141> 2003-02-17

<160> 75

<170> PatentIn version 3.1

<210> 1
<211> 4
<212> PRT
<213> Homo sapiens

<220>
<221> SITE
<222> (1)..(4)
<223> retention signal of ER resident proteins

<400> 1

Lys Asp Glu Leu
1

<210> 2
<211> 4
<212> PRT
<213> Homo sapiens

<220>
<221> SITE
<222> (1)..(4)
<223> active site of ER chaperone molecule

<220>
<221> MISC_FEATURE
<222> (2)..(3)
<223> "X" stands for any amino acid

<400> 2

Cys Xaa Xaa Cys
1

<210> 3
<211> 4
<212> PRT
<213> Mus musculus

<220>
<221> SITE
<222> (1)..(4)
<223> active site of smERp1

<400> 3

Cys Asp Ala Cys
1

<210> 4
<211> 4
<212> PRT
<213> Mus musculus

<220>
<221> SITE
<222> (1)..(4)
<223> active site of PDI

<400> 4

Cys Gly His Cys
1

<210> 5
<211> 6
<212> DNA
<213> Mus musculus

<220>
<221> misc_feature
<222> (1)..(6)
<223> potential polyadenylation signal

<400> 5
aataaa 6

<210> 6
<211> 35
<212> DNA
<213> Artificial Sequence

<220>
<223> primer HAR

<400> 6
ggacgtcgta tgggtacccg gcgctcccca ggata 35

<210> 7
<211> 38
<212> DNA
<213> Artificial Sequence

<220>
<223> primer HAF

<400> 7
cgacgtccca gactacgcag atagggtttc cctctcgg 38

<210> 8
<211> 16
<212> DNA
<213> Artificial Sequence

<220>
<223> primer M13F

<400> 8
gtaaaacgac ggccag 16

<210> 9
<211> 17
<212> DNA
<213> Artificial Sequence

<220>
<223> primer M13R

<400> 9
caggaaacag ctatgac 17

<210> 10
<211> 4
<212> PRT
<213> Mus musculus

<220>
<221> SITE
<222> (1)..(4)
<223> CXXS active site

<220>
<221> MISC_FEATURE
<222> (2)..(3)
<223> "X" stands for any amino acid

<400> 10

Cys Xaa Xaa Ser
1

<210> 11
<211> 350
<212> PRT
<213> Mus musculus

<220>
<221> SITE
<222> (1)..(350)
<223> CRELP

<400> 11

Met His Leu Leu Leu Ala Ala Ala Phe Gly Leu Leu Leu Leu Leu Pro
1 5 10 15

Pro Pro Gly Ala Val Ala Ser Arg Lys Pro Thr Met Cys Gln Arg Cys
20 25 30

Arg Thr Leu Val Asp Lys Phe Asn Gln Gly Met Ala Asn Thr Ala Arg
35 40 45

Lys Asn Phe Gly Gly Gly Asn Thr Ala Trp Glu Glu Lys Thr Leu Ser
50 55 60

Lys Tyr Glu Phe Ser Glu Ile Arg Leu Leu Glu Ile Met Glu Gly Leu
65 70 75 80

Cys Asp Ser Ser Asp Phe Glu Cys Asn Gln Leu Leu Glu Gln Gln Glu
85 90 95

Glu Gln Leu Glu Ala Trp Trp Gln Thr Leu Lys Lys Glu His Pro Asn
100 105 110

Leu Phe Glu Trp Phe Cys Val His Thr Leu Lys Ala Cys Cys Leu Pro
115 120 125

Gly Thr Tyr Gly Pro Asp Cys Gln Glu Cys Gln Gly Gly Ser Glu Arg
130 135 140

Pro Cys Ser Gly Asn Gly Tyr Cys Ser Gly Asp Gly Ser Arg Gln Gly
145 150 155 160

Asp Gly Ser Cys Gln Cys His Thr Gly Tyr Lys Gly Pro Leu Cys Ile
165 170 175

Asp Cys Thr Asp Gly Phe Phe Ser Leu Gln Arg Asn Glu Thr His Ser
180 185 190

Ile Cys Ser Ala Cys Asp Glu Ser Cys Lys Thr Cys Ser Gly Pro Ser
195 200 205

Asn Lys Asp Cys Ile Gln Cys Glu Val Gly Trp Ala Arg Val Glu Asp
210 215 220

Ala Cys Val Asp Val Asp Glu Cys Ala Ala Glu Thr Ser Pro Cys Ser
225 230 235 240

Asp Gly Gln Tyr Cys Glu Asn Val Asn Gly Ser Tyr Thr Cys Glu Asp
245 250 255

Cys Asp Ser Thr Cys Val Gly Cys Thr Gly Lys Gly Pro Ala Asn Cys
260 265 270

Lys Glu Cys Ile Ala Gly Tyr Thr Lys Glu Ser Gly Gln Cys Thr Asp
275 280 285

Ile Asp Glu Cys Ser Leu Glu Glu Lys Ala Cys Lys Arg Lys Asn Glu
290 295 300

Asn Cys Tyr Asn Val Pro Gly Ser Phe Val Cys Val Cys Pro Glu Gly
305 310 315 320

Phe Glu Glu Thr Glu Asp Ala Cys Val Gln Thr Ala Glu Gly Lys Val
325 330 335

Thr Glu Glu Asn Pro Thr Gln Pro Pro Ser Arg Glu Asp Leu
340 345 350

<210> 12
<211> 353
<212> PRT
<213> Homo sapiens

<220>
<221> SITE
<222> (1)..(353)
<223> CRELP

<400> 12

Met Arg Leu Pro Arg Arg Ala Ala Leu Gly Leu Leu Pro Leu Leu Leu
1 5 10 15

Leu Leu Pro Pro Ala Pro Glu Ala Ala Lys Lys Pro Thr Pro Cys His
20 25 30

Arg Cys Arg Gly Leu Val Asp Lys Phe Asn Gln Gly Met Val Asp Thr
35 40 45

Ala Lys Lys Asn Phe Gly Gly Gly Asn Thr Ala Trp Glu Glu Lys Thr
50 55 60

Leu Ser Lys Tyr Glu Ser Ser Glu Ile Arg Leu Leu Glu Ile Leu Glu
65 70 75 80

Gly Leu Cys Glu Ser Ser Asp Phe Glu Cys Asn Gln Met Leu Glu Ala
85 90 95

Gln Glu Glu His Leu Glu Ala Trp Trp Leu Gln Leu Lys Ser Glu Tyr
100 105 110

Pro Asp Leu Phe Glu Trp Phe Cys Val Lys Thr Leu Lys Val Cys Cys
115 120 125

Ser Pro Gly Thr Tyr Gly Pro Asp Cys Leu Ala Cys Gln Gly Gly Ser
130 135 140

Gln Arg Pro Cys Ser Gly Asn Gly His Cys Ser Gly Asp Gly Ser Arg
145 150 155 160

Gln Gly Asp Gly Ser Cys Arg Cys His Met Gly Tyr Gln Gly Pro Leu
165 170 175

Cys Thr Asp Cys Met Asp Gly Tyr Phe Ser Ser Leu Arg Asn Glu Thr
180 185 190

His Ser Ile Cys Thr Ala Cys Asp Glu Ser Cys Lys Thr Cys Ser Gly
195 200 205

Leu Thr Asn Arg Asp Cys Gly Glu Cys Glu Val Gly Trp Val Leu Asp
    210                 215                 220

Glu Gly Ala Cys Val Asp Val Asp Glu Cys Ala Ala Glu Pro Pro Pro
225                 230                 235                 240

Cys Ser Ala Ala Gln Phe Cys Lys Asn Ala Asn Gly Ser Tyr Thr Cys
                245                 250                 255

Glu Asp Cys Asp Ser Ser Cys Val Gly Cys Thr Gly Glu Gly Pro Gly
            260                 265                 270

Asn Cys Lys Glu Cys Ile Ser Gly Tyr Ala Arg Glu His Gly Gln Cys
        275                 280                 285

Ala Asp Val Asp Glu Cys Ser Leu Ala Glu Lys Thr Cys Val Arg Lys
    290                 295                 300

Asn Glu Asn Cys Tyr Asn Thr Pro Gly Ser Tyr Val Cys Val Cys Pro
305                 310                 315                 320

Asp Gly Phe Glu Glu Thr Glu Asp Ala Cys Val Pro Pro Ala Glu Ala
                325                 330                 335

Glu Ala Thr Glu Gly Glu Ser Pro Thr Gln Leu Pro Ser Arg Glu Asp
            340                 345                 350

Leu

<210> 13
<211> 420
<212> PRT
<213> Mus musculus

<220>
<221> SITE
<222> (1)..(420)
<223> CREMP

<400> 13

Met Ala Pro Leu Pro Pro Arg Gly Leu Val Pro Ser Leu Leu Trp Cys
1               5                   10                  15

Leu Ser Leu Phe Leu Ser Leu Pro Gly Pro Val Trp Leu Gln Pro Ser
            20                  25                  30

Pro Pro Pro His Pro Ser Pro Arg Ala Glu Pro His Pro Cys His Thr
        35                  40                  45

Cys Arg Ala Leu Val Asp Asn Phe Asn Lys Gly Leu Glu Arg Thr Ile

50 55 60

Arg Asp Asn Phe Gly Gly Gly Asn Thr Ala Trp Glu Glu Glu Lys Leu
65 70 75 80

Ser Lys Tyr Lys Asp Ser Glu Thr Arg Leu Val Glu Val Leu Glu Gly
85 90 95

Val Cys Ser Arg Ser Asp Phe Glu Cys His Arg Leu Leu Glu Leu Ser
100 105 110

Glu Glu Leu Val Glu Asn Trp Trp Phe His Arg Gln Gln Glu Ala Pro
115 120 125

Asp Leu Phe Gln Trp Leu Cys Ser Asp Ser Leu Lys Leu Cys Cys Pro
130 135 140

Ser Gly Thr Phe Gly Pro Ser Cys Leu Pro Cys Pro Gly Gly Thr Glu
145 150 155 160

Arg Pro Cys Gly Gly Tyr Gly Gln Cys Glu Gly Glu Gly Thr Arg Gly
165 170 175

Gly Ser Gly His Cys Asp Cys Gln Ala Gly Tyr Gly Gly Glu Ala Cys
180 185 190

Gly Gln Cys Gly Leu Gly Tyr Phe Glu Ala Glu Arg Asn Ser Ser His
195 200 205

Leu Val Cys Ser Ala Cys Phe Gly Pro Cys Ala Arg Cys Thr Gly Pro
210 215 220

Glu Glu Ser His Cys Leu Gln Cys Lys Lys Gly Trp Ala Leu His His
225 230 235 240

Leu Lys Cys Val Asp Ile Asp Glu Cys Gly Thr Glu Gln Ala Thr Cys
245 250 255

Gly Ala Asp Gln Phe Cys Val Asn Thr Glu Gly Ser Tyr Glu Cys Arg
260 265 270

Asp Cys Ala Lys Ala Cys Leu Gly Cys Met Gly Ala Gly Pro Gly Arg
275 280 285

Cys Lys Lys Cys Ser Arg Gly Tyr Gln Gln Val Gly Ser Lys Cys Leu
290 295 300

Asp Val Asp Glu Cys Glu Thr Val Val Cys Pro Gly Glu Asn Glu Lys
305 310 315 320

Cys Glu Asn Thr Glu Gly Gly Tyr Arg Cys Val Cys Ala Glu Gly Tyr

325 330 335

Arg Gln Glu Asp Gly Ile Cys Val Lys Glu Gln Val Pro Glu Ser Ala
340 345 350

Gly Phe Phe Ala Glu Met Thr Glu Asp Glu Met Val Val Leu Gln Gln
355 360 365

Met Phe Phe Gly Val Ile Ile Cys Ala Leu Ala Thr Leu Ala Ala Lys
370 375 380

Gly Asp Leu Val Phe Thr Ala Ile Phe Ile Gly Ala Val Ala Ala Met
385 390 395 400

Thr Gly Tyr Trp Leu Ser Glu Arg Ser Asp Arg Val Leu Glu Gly Phe
405 410 415

Ile Lys Gly Arg
420

<210> 14
<211> 417
<212> PRT
<213> Homo sapiens

<220>
<221> SITE
<222> (1)..(417)
<223> CREMP

<400> 14

Met Ala Pro Trp Pro Pro Lys Gly Leu Val Pro Ala Met Leu Trp Gly
1 5 10 15

Leu Ser Leu Phe Leu Asn Leu Pro Gly Pro Ile Trp Leu Gln Pro Ser
20 25 30

Pro Pro Pro Gln Ser Ser Pro Pro Pro Gln Pro His Pro Cys His Thr
35 40 45

Cys Arg Gly Leu Val Asp Ser Phe Asn Lys Gly Leu Glu Arg Thr Ile
50 55 60

Arg Asp Asn Phe Gly Gly Gly Asn Thr Ala Trp Glu Glu Glu Asn Leu
65 70 75 80

Ser Lys Tyr Lys Asp Ser Glu Thr Arg Leu Val Glu Val Leu Glu Gly
85 90 95

Val Cys Ser Lys Ser Asp Phe Glu Cys His Arg Leu Leu Glu Leu Ser
100 105 110

```
Glu Glu Leu Val Glu Ser Trp Trp Phe His Lys Gln Gln Glu Ala Pro
        115                 120                 125

Asp Leu Phe Gln Trp Leu Cys Ser Asp Ser Leu Lys Leu Cys Cys Pro
    130                 135                 140

Ala Gly Thr Phe Gly Pro Ser Cys Leu Pro Cys Pro Gly Gly Thr Glu
145                 150                 155                 160

Arg Pro Cys Gly Gly Tyr Gly Gln Cys Glu Gly Glu Gly Thr Arg Gly
                165                 170                 175

Gly Ser Gly His Cys Asp Cys Gln Ala Gly Tyr Gly Gly Glu Ala Cys
            180                 185                 190

Gly Gln Cys Gly Leu Gly Tyr Phe Glu Ala Glu Arg Asn Ala Ser His
        195                 200                 205

Leu Val Cys Ser Ala Cys Phe Gly Pro Cys Ala Arg Cys Ser Gly Pro
    210                 215                 220

Glu Glu Ser Asn Cys Leu Gln Cys Lys Lys Gly Trp Ala Leu His His
225                 230                 235                 240

Leu Lys Cys Val Asp Ile Asp Glu Cys Gly Thr Glu Gly Ala Asn Cys
                245                 250                 255

Gly Ala Asp Gln Phe Cys Val Asn Thr Glu Gly Ser Tyr Glu Cys Arg
            260                 265                 270

Asp Cys Ala Lys Ala Cys Leu Gly Cys Met Gly Ala Gly Pro Gly Arg
        275                 280                 285

Cys Lys Lys Cys Ser Pro Gly Tyr Gln Gln Val Gly Ser Lys Cys Leu
    290                 295                 300

Asp Val Asp Glu Cys Glu Thr Glu Val Cys Pro Gly Glu Asn Lys Gln
305                 310                 315                 320

Cys Glu Asn Thr Glu Gly Gly Tyr Arg Cys Ile Cys Ala Glu Gly Tyr
                325                 330                 335

Lys Gln Met Glu Gly Ile Cys Val Lys Glu Gln Ile Pro Glu Ser Ala
            340                 345                 350

Gly Phe Phe Ser Glu Met Thr Glu Asp Glu Leu Val Val Leu Gln Gln
        355                 360                 365

Met Phe Phe Gly Ile Ile Ile Cys Ala Leu Ala Thr Leu Ala Ala Lys
    370                 375                 380
```

Gly Asp Leu Val Phe Thr Ala Ile Phe Ile Gly Ala Val Ala Ala Met
385 390 395 400

Thr Gly Tyr Trp Leu Ser Glu Arg Ser Asp Arg Val Leu Glu Gly Phe
405 410 415

Ile

<210> 15
<211> 747
<212> PRT
<213> Homo sapiens

<220>
<221> SITE
<222> (1)..(747)
<223> Quiescin-1

<400> 15

Met Arg Arg Cys Asn Ser Gly Ser Gly Pro Pro Pro Ser Leu Leu Leu
1 5 10 15

Leu Leu Leu Trp Leu Leu Ala Val Pro Gly Ala Asn Ala Ala Pro Arg
20 25 30

Ser Ala Leu Tyr Ser Pro Ser Asp Pro Leu Thr Leu Leu Gln Ala Asp
35 40 45

Thr Val Arg Gly Ala Val Leu Gly Ser Arg Ser Ala Trp Ala Val Glu
50 55 60

Phe Phe Ala Ser Trp Cys Gly His Cys Ile Ala Phe Ala Pro Thr Trp
65 70 75 80

Lys Ala Leu Ala Glu Asp Val Lys Ala Trp Arg Pro Ala Leu Tyr Leu
85 90 95

Ala Ala Leu Asp Cys Ala Glu Glu Thr Asn Ser Ala Val Cys Arg Asp
100 105 110

Phe Asn Ile Pro Gly Phe Pro Thr Val Arg Phe Phe Lys Ala Phe Thr
115 120 125

Lys Asn Gly Ser Gly Ala Val Phe Pro Val Ala Gly Ala Asp Val Gln
130 135 140

Thr Leu Arg Glu Arg Leu Ile Asp Ala Leu Glu Ser His His Asp Thr
145 150 155 160

Trp Pro Pro Ala Cys Pro Pro Leu Glu Pro Ala Lys Leu Glu Glu Ile
165 170 175

Asp Gly Phe Phe Ala Arg Asn Asn Glu Glu Tyr Leu Ala Leu Ile Phe
180 185 190

Glu Lys Gly Gly Ser Tyr Leu Gly Arg Glu Val Ala Leu Asp Leu Ser
195 200 205

Gln His Lys Gly Val Ala Val Arg Arg Val Leu Asn Thr Glu Ala Asn
210 215 220

Val Val Arg Lys Phe Gly Val Thr Asp Phe Pro Ser Cys Tyr Leu Leu
225 230 235 240

Phe Arg Asn Gly Ser Val Ser Arg Val Pro Val Leu Met Glu Ser Arg
245 250 255

Ser Phe Tyr Thr Ala Tyr Leu Gln Arg Leu Ser Gly Leu Thr Arg Glu
260 265 270

Ala Ala Gln Thr Thr Val Ala Pro Thr Thr Ala Asn Lys Ile Ala Pro
275 280 285

Thr Val Trp Lys Leu Ala Asp Arg Ser Lys Ile Tyr Met Ala Asp Leu
290 295 300

Glu Ser Ala Leu His Tyr Ile Leu Arg Ile Glu Val Gly Arg Phe Pro
305 310 315 320

Val Leu Glu Gly Gln Arg Leu Val Ala Leu Lys Lys Phe Val Ala Val
325 330 335

Leu Ala Lys Tyr Phe Pro Gly Arg Pro Leu Val Gln Asn Phe Leu His
340 345 350

Ser Val Asn Glu Trp Leu Lys Arg Gln Lys Arg Asn Lys Ile Pro Tyr
355 360 365

Ser Phe Phe Lys Thr Ala Leu Asp Asp Arg Lys Glu Gly Ala Val Leu
370 375 380

Ala Lys Lys Val Asn Trp Ile Gly Cys Gln Gly Ser Glu Pro His Phe
385 390 395 400

Arg Gly Phe Pro Cys Ser Leu Trp Val Leu Phe His Phe Leu Thr Val
405 410 415

Gln Ala Ala Arg Gln Asn Val Asp His Ser Gln Glu Ala Ala Lys Ala
420 425 430

Lys Glu Val Leu Pro Ala Ile Arg Gly Tyr Val His Tyr Phe Phe Gly
435 440 445

Cys Arg Asp Cys Ala Ser His Phe Glu Gln Met Ala Ala Ala Ser Met
450 455 460

His Arg Val Gly Ser Pro Asn Ala Ala Val Leu Trp Leu Trp Ser Ser
465 470 475 480

His Asn Arg Val Asn Ala Arg Leu Ala Gly Ala Pro Ser Glu Asp Pro
485 490 495

Gln Phe Pro Lys Val Gln Trp Pro Pro Arg Glu Leu Cys Ser Ala Cys
500 505 510

His Asn Glu Arg Leu Asp Val Pro Val Trp Asp Val Glu Ala Thr Leu
515 520 525

Asn Phe Leu Lys Ala His Phe Ser Pro Ser Asn Ile Ile Leu Asp Phe
530 535 540

Pro Ala Ala Gly Ser Ala Ala Arg Arg Asp Val Gln Asn Val Ala Ala
545 550 555 560

Ala Pro Glu Leu Ala Met Gly Ala Leu Glu Leu Glu Ser Arg Asn Ser
565 570 575

Thr Leu Asp Pro Gly Lys Pro Glu Met Met Lys Ser Pro Thr Asn Thr
580 585 590

Thr Pro His Val Pro Ala Glu Gly Pro Glu Ala Ser Arg Pro Pro Lys
595 600 605

Leu His Pro Gly Leu Arg Ala Ala Pro Gly Gln Glu Pro Pro Glu His
610 615 620

Met Ala Glu Leu Gln Arg Asn Glu Gln Glu Gln Pro Leu Gly Gln Trp
625 630 635 640

His Leu Ser Lys Arg Asp Thr Gly Ala Ala Leu Leu Ala Glu Ser Arg
645 650 655

Ala Glu Lys Asn Arg Leu Trp Gly Pro Leu Glu Val Arg Arg Val Gly
660 665 670

Arg Ser Ser Lys Gln Leu Val Asp Ile Pro Glu Gly Gln Leu Glu Ala
675 680 685

Arg Ala Gly Arg Gly Arg Gly Gln Trp Leu Gln Val Leu Gly Gly Gly
690 695 700

Phe Ser Tyr Leu Asp Ile Ser Leu Cys Val Gly Leu Tyr Ser Leu Ser
705 710 715 720

Phe Met Gly Leu Leu Ala Met Tyr Thr Tyr Phe Gln Ala Lys Ile Arg
725 730 735

Ala Leu Lys Gly His Ala Gly His Pro Ala Ala
740 745

<210> 16
<211> 639
<212> PRT
<213> Mus musculus

<220>
<221> SITE
<222> (1)..(639)
<223> Quiescin-2

<400> 16

Met Ala Ala Ala Arg Ala Val Ala Arg Asp Pro Gly Ala Tyr Ala Arg
1 5 10 15

Gln Pro Pro Ser Leu Arg Ala Ala Arg Leu Pro Arg Leu Leu Phe Leu
20 25 30

Leu Ala Val Val Ala Ala Val Gly Pro Arg Glu Gly Gly Gly Ala Arg
35 40 45

Leu Tyr Arg Glu Gly Ser Asp Ala Val Trp Leu Leu Asp Ser Gly Ser
50 55 60

Val Arg Ser Ala Thr Gly Asn Ser Ser Ala Ala Trp Leu Val Gln Phe
65 70 75 80

His Ser Ser Trp Cys Gly His Cys Ile Gly Tyr Ala Pro Thr Trp Arg
85 90 95

Ala Leu Ala Ala Asp Val Arg Asp Trp Ala Ala Ala Ile Arg Val Ala
100 105 110

Ala Leu Asp Cys Ala Glu Glu Lys Asn Gln Asp Val Cys Arg Thr Tyr
115 120 125

Asp Ile His Phe Tyr Pro Thr Phe Arg Tyr Phe Lys Ala Phe Thr Lys
130 135 140

Glu Phe Thr Thr Gly Glu Asn Phe Lys Gly Pro Asp Arg Glu Leu Arg
145 150 155 160

Thr Val Arg Gln Thr Met Ile Asp Phe Leu Gln Asn His Thr Glu Gly
165 170 175

Thr Trp Pro Pro Ala Cys Pro Pro Leu Asp Pro Ile Gln Ser Ser Asp
180 185 190

Ile Leu Ser Phe Met Asp Ser His Ser Gly Gln Tyr His Ala Ile Val
195 200 205

Phe Glu Ser Asn Gly Ser Tyr Val Gly Arg Glu Val Ile Leu Asp Leu
210 215 220

Ile Pro Tyr Glu Asn Ile Met Val Ser Arg Ala Leu Asp Thr Asp Lys
225 230 235 240

Ala Phe Leu Gly Thr Leu Gly Ile Thr Ser Val Pro Ser Cys Tyr Leu
245 250 255

Ile Tyr Pro Asn Gly Ser His Gly Leu Val Asn Val Ala Lys Pro Leu
260 265 270

Arg Ser Phe Phe Ser Ser His Leu Lys Ser Leu Pro Asp Val Arg Lys
275 280 285

Lys Ser Leu Phe Leu Pro Glu Lys Ser Asn Lys Glu Glu Lys Ser Glu
290 295 300

Val Val Val Trp Lys Glu Phe Asp Arg Ala Lys Leu Tyr Thr Ala Asp
305 310 315 320

Leu Glu Ser Gly Leu His Tyr Leu Leu Arg Val Glu Leu Ala Ala His
325 330 335

Arg Ser Leu Ala Gly Ala Gln Leu Lys Thr Phe Arg Asp Phe Val Thr
340 345 350

Val Val Ala Lys Leu Phe Pro Gly Arg Pro Ala Val Lys Lys Leu Leu
355 360 365

Glu Thr Leu Gln Glu Trp Leu Ala Asn Leu Pro Leu Asp Lys Ile Pro
370 375 380

Tyr Asn Ala Ile Leu Asp Leu Val Asn Asn Lys Met Gln Ile Ser Gly
385 390 395 400

Ile Phe Leu Thr Ser His Val Lys Trp Val Gly Cys Gln Gly Ser Arg
405 410 415

Leu Glu Leu Arg Gly Tyr Pro Cys Ser Leu Trp Lys Leu Phe His Thr
420 425 430

Leu Thr Val Gln Ala Ser Thr His Pro Glu Ala Leu Ala Gly Thr Gly
435 440 445

Phe Glu Gly His Pro Gln Ala Val Leu Gln Ala Ile Arg Arg Tyr Ile
450 455 460

Arg Thr Phe Phe Gly Cys Lys Glu Cys Gly Glu His Phe Glu Glu Met
465 470 475 480

Ala Lys Glu Ser Met Asp Ser Val Lys Thr Pro Asp Gln Ala Val Leu
485 490 495

Trp Leu Trp Arg Lys His Asn Met Val Asn Ser Arg Leu Ala Gly His
500 505 510

Leu Ser Glu Asp Pro Lys Phe Pro Lys Val Pro Trp Pro Thr Pro Asp
515 520 525

Leu Cys Pro Ala Cys His Glu Glu Ile Lys Gly Leu Asp Ser Trp Asn
530 535 540

Glu Gly Gln Val Leu Leu Phe Leu Lys Gln His Tyr Ser Arg Asp Asn
545 550 555 560

Leu Val Asp Ala Tyr Ser Val Asp Gln Gly Ser Pro Gly Ser Val Leu
565 570 575

Arg Ala Arg Pro Trp Leu Gly Gln Met Ala Arg Leu Ser His Val Asn
580 585 590

Leu Leu Pro His Phe Pro Val Glu Glu Val Ser Ser Leu Lys Pro Gly
595 600 605

Val Leu Cys Leu Lys Thr Asn Lys Pro Arg Ser Ser Leu Gly Val Ser
610 615 620

Lys Asp Glu His Ser Trp Ser Val Ser Leu Arg Ile Gly Pro Ile
625 630 635

<210> 17
<211> 188
<212> PRT
<213> Mus musculus

<220>
<221> SITE
<222> (1)..(188)
<223> smERp1

<400> 17

Met Arg Leu Pro Leu Pro Leu Leu Leu Leu Phe Gly Cys Arg Ala Ile
1 5 10 15

Leu Gly Ser Ala Gly Asp Arg Val Ser Leu Ser Ala Ser Ala Pro Thr
20 25 30

Leu Asp Asp Glu Glu Lys Tyr Ser Ala His Met Pro Ala His Leu Arg

```
        35                  40                  45

Cys Asp Ala Cys Arg Ala Val Ala Phe Gln Met Gly Gln Arg Leu Ala
    50                  55                  60

Lys Ala Glu Ala Lys Ser His Thr Pro Asp Ala Ser Gly Leu Gln Glu
65                  70                  75                  80

Leu Ser Glu Ser Thr Tyr Thr Asp Val Leu Asp Gln Thr Cys Ser Gln
                85                  90                  95

Asn Trp Gln Ser Tyr Gly Val His Glu Val Asn Gln Met Lys Arg Leu
            100                 105                 110

Thr Gly Pro Gly Leu Ser Lys Gly Pro Glu Pro Arg Ile Ser Val Met
        115                 120                 125

Ile Ser Gly Gly Pro Trp Pro Asn Arg Leu Ser Lys Thr Cys Phe His
    130                 135                 140

Tyr Leu Gly Glu Phe Gly Glu Asp Gln Ile Tyr Glu Ala Tyr Arg Gln
145                 150                 155                 160

Gly Gln Ala Asn Leu Glu Ala Leu Leu Cys Gly Gly Thr His Gly Pro
                165                 170                 175

Cys Ser Gln Glu Ile Leu Ala Gln Arg Glu Glu Leu
            180                 185

<210>  18
<211>  900
<212>  DNA
<213>  Mus musculus

<220>
<221>  misc_feature
<222>  (1)..(900)
<223>  smERp

<220>
<221>  CDS
<222>  (36)..(599)
<223>

<400>  18
gacagcctgt gctccaagaa gtaagttcag aggcc atg aga ctg cct ctg cca       53
                                       Met Arg Leu Pro Leu Pro
                                       1               5

ctg ttg cta ctg ttc ggg tgc agg gct atc ctg ggg agc gcc ggg gat     101
Leu Leu Leu Leu Phe Gly Cys Arg Ala Ile Leu Gly Ser Ala Gly Asp
            10                  15                  20

agg gtt tcc ctc tcg gct tcg gct ccc aca ctg gat gat gaa gag aag     149
Arg Val Ser Leu Ser Ala Ser Ala Pro Thr Leu Asp Asp Glu Glu Lys
        25                  30                  35
```

```
tac tcg gct cat atg ccg gct cac ctg cgc tgc gat gcc tgc cgg gct      197
Tyr Ser Ala His Met Pro Ala His Leu Arg Cys Asp Ala Cys Arg Ala
    40                  45                  50

gtg gcc ttc cag atg ggg caa cgt ctg gcg aaa gca gag gct aaa tct      245
Val Ala Phe Gln Met Gly Gln Arg Leu Ala Lys Ala Glu Ala Lys Ser
55                  60                  65                  70

cac act cca gac gcc agt gga ttg cag gag ctg agt gaa tcc acg tac      293
His Thr Pro Asp Ala Ser Gly Leu Gln Glu Leu Ser Glu Ser Thr Tyr
                75                  80                  85

aca gat gtc ctg gac cag acc tgc tct cag aac tgg cag tcc tat gga      341
Thr Asp Val Leu Asp Gln Thr Cys Ser Gln Asn Trp Gln Ser Tyr Gly
            90                  95                  100

gtt cat gaa gtg aac cag atg aag cgt ctc acg ggc cca gga ctt agc      389
Val His Glu Val Asn Gln Met Lys Arg Leu Thr Gly Pro Gly Leu Ser
        105                 110                 115

aag ggg cca gag cca aga atc agc gtg atg att tct ggg ggt ccc tgg      437
Lys Gly Pro Glu Pro Arg Ile Ser Val Met Ile Ser Gly Gly Pro Trp
    120                 125                 130

ccc aat agg ctc tcc aag acg tgt ttc cac tac ctg ggt gag ttt gga      485
Pro Asn Arg Leu Ser Lys Thr Cys Phe His Tyr Leu Gly Glu Phe Gly
135                 140                 145                 150

gag gac cag atc tat gaa gcc tac cgc caa ggc caa gcg aat ctg gag      533
Glu Asp Gln Ile Tyr Glu Ala Tyr Arg Gln Gly Gln Ala Asn Leu Glu
                155                 160                 165

gcg ctg ctc tgt ggg ggc acc cat ggg ccc tgc tca cag gag atc ctg      581
Ala Leu Leu Cys Gly Gly Thr His Gly Pro Cys Ser Gln Glu Ile Leu
            170                 175                 180

gcc cag aga gaa gag ctt tagtccaacc tgctgcactt ctggatcttc            629
Ala Gln Arg Glu Glu Leu
        185

tctaatttta ttattattaa tggctgatta gaggcaggct ctcatcatgt aggccaggct    689

ggccttaaac ttgtcatcct gctcagcctc gaaagtgctg catttaagtc ctgagccttt    749

ttgtgcttga ccatcctata taattttttc aactgtggtg gtggggaggg gacagggaag    809

cctgactcta gctgtcaatc ttctccctcc acctctcgat ggggtactgg gactgaggct    869

gcctttctac tttcaaataa agctttgaaa g                                   900
```

<210> 19
<211> 188
<212> PRT
<213> Mus musculus

<220>
<221> misc_feature
<222> (1)..(900)
<223> smERp

<400> 19

```
Met Arg Leu Pro Leu Pro Leu Leu Leu Leu Phe Gly Cys Arg Ala Ile
1               5                   10                  15
```

Leu Gly Ser Ala Gly Asp Arg Val Ser Leu Ser Ala Ser Ala Pro Thr
20 25 30

Leu Asp Asp Glu Glu Lys Tyr Ser Ala His Met Pro Ala His Leu Arg
35 40 45

Cys Asp Ala Cys Arg Ala Val Ala Phe Gln Met Gly Gln Arg Leu Ala
50 55 60

Lys Ala Glu Ala Lys Ser His Thr Pro Asp Ala Ser Gly Leu Gln Glu
65 70 75 80

Leu Ser Glu Ser Thr Tyr Thr Asp Val Leu Asp Gln Thr Cys Ser Gln
85 90 95

Asn Trp Gln Ser Tyr Gly Val His Glu Val Asn Gln Met Lys Arg Leu
100 105 110

Thr Gly Pro Gly Leu Ser Lys Gly Pro Glu Pro Arg Ile Ser Val Met
115 120 125

Ile Ser Gly Gly Pro Trp Pro Asn Arg Leu Ser Lys Thr Cys Phe His
130 135 140

Tyr Leu Gly Glu Phe Gly Glu Asp Gln Ile Tyr Glu Ala Tyr Arg Gln
145 150 155 160

Gly Gln Ala Asn Leu Glu Ala Leu Leu Cys Gly Gly Thr His Gly Pro
165 170 175

Cys Ser Gln Glu Ile Leu Ala Gln Arg Glu Glu Leu
180 185

<210> 20
<211> 20
<212> DNA
<213> Mus musculus

<220>
<221> misc_feature
<222> (1)..(20)
<223> intron/exon boundary

<400> 20
ggccttccag gtaagctctg 20

<210> 21
<211> 20
<212> DNA
<213> Mus musculus

<220>
<221> misc_feature
<222> (1)..(20)
<223> intron/exon boundary

<400> 21
ctcattccag atggggcaac 20

<210> 22
<211> 20
<212> DNA
<213> Mus musculus

<220>
<221> misc_feature
<222> (1)..(20)
<223> intron/exon boundary

<400> 22
actggcagtc gtgagttgtt 20

<210> 23
<211> 20
<212> DNA
<213> Mus musculus

<220>
<221> misc_feature
<222> (1)..(20)
<223> intron/exon boundary

<400> 23
ttaccatcag ctatggagtt 20

<210> 24
<211> 20
<212> DNA
<213> Mus musculus

<220>
<221> misc_feature
<222> (1)..(20)
<223> intron/exon boundary

<400> 24
ggcccaatag gtaatagcat 20

<210> 25
<211> 20
<212> DNA
<213> Mus musculus

<220>
<221> misc_feature
<222> (1)..(20)
<223> intron/exon boundary

<400> 25
gctcccctag gctctccaag 20

<210> 26
<211> 189

<212> PRT
<213> Homo sapiens

<220>
<221> SITE
<222> (1)..(189)
<223> smERp1

<400> 26

```
Met Arg Leu Ser Leu Pro Leu Leu Leu Leu Leu Leu Gly Ala Trp Ala
1               5                   10                  15

Ile Pro Gly Gly Leu Gly Asp Arg Ala Pro Leu Thr Ala Thr Ala Pro
            20                  25                  30

Gln Leu Asp Asp Glu Glu Met Tyr Ser Ala His Met Pro Ala His Leu
        35                  40                  45

Arg Cys Asp Ala Cys Arg Ala Val Ala Tyr Gln Met Trp Gln Asn Leu
    50                  55                  60

Ala Lys Ala Glu Thr Lys Leu His Thr Ser Asn Ser Gly Gly Arg Arg
65                  70                  75                  80

Glu Leu Ser Glu Leu Val Tyr Thr Asp Val Leu Asp Arg Ser Cys Ser
                85                  90                  95

Arg Asn Trp Gln Asp Tyr Gly Val Arg Glu Val Asp Gln Val Lys Arg
            100                 105                 110

Leu Thr Gly Pro Gly Leu Ser Glu Gly Pro Glu Pro Ser Ile Ser Val
        115                 120                 125

Met Val Thr Gly Gly Pro Trp Pro Thr Arg Leu Ser Arg Thr Cys Leu
    130                 135                 140

His Tyr Leu Gly Glu Phe Gly Glu Asp Gln Ile Tyr Glu Ala His Gln
145                 150                 155                 160

Gln Gly Arg Gly Ala Leu Glu Ala Leu Leu Cys Gly Gly Pro Gln Gly
                165                 170                 175

Ala Cys Ser Glu Lys Val Ser Ala Thr Arg Glu Glu Leu
            180                 185
```

<210> 27
<211> 197
<212> PRT
<213> Gallus gallus

<220>
<221> SITE
<222> (1)..(197)
<223> smERp1

<400> 27

Met Gln Ala Val Leu Val Leu Cys Val Ala Leu Ser Leu Ala Ala Arg
1 5 10 15

Ala Gly Gly Glu Asp Ala Cys Gly Gly Arg Glu Ala Pro Ser Ser Ser
20 25 30

His Ser Ile Pro Ala Pro Gln Met Ser Ala Glu Asp Arg Leu Ser Pro
35 40 45

His Met Pro Glu Ala Leu Arg Cys Asp Ala Cys His Ala Ile Ala Phe
50 55 60

Gln Leu Glu Gly Arg Leu Ser Arg Ala Glu Ala Lys Gln Gly Arg Lys
65 70 75 80

Ala Leu Ser Glu Ser Asp Tyr Val Glu Val Leu Glu Gln Ser Cys Leu
85 90 95

Gln Asp Trp Glu Leu Tyr Gly Val Gln Glu Leu Arg Gly Glu Arg Arg
100 105 110

Leu Met Gly Pro Gly Leu Pro Gly Gln Glu Ala Met Ser Val Ala Val
115 120 125

Met Gly Gly Pro Trp Pro Gly Arg Leu Ala Lys Met Cys His Ser Leu
130 135 140

Val Gly Glu His Gly Glu Glu Gln Leu Tyr Gly Ala His Arg Arg Gly
145 150 155 160

Pro Thr Ala Leu Arg Glu Leu Leu Cys His Gly Lys Lys Gly Pro Cys
165 170 175

Ala His Leu Gly Gly Ser Lys Ala Gly Gly Ser Ala Pro Pro Lys Ala
180 185 190

Leu Gln Asn Glu Leu
195

<210> 28
<211> 142
<212> PRT
<213> Mus musculus

<220>
<221> SITE
<222> (1)..(142)
<223> part of smERp1

<400> 28

```
Leu Arg Cys Asp Ala Cys Arg Ala Val Ala Phe Gln Met Gly Gln Arg
1               5                   10                  15

Leu Ala Lys Ala Glu Ala Lys Ser His Thr Pro Asp Ala Ser Gly Leu
            20                  25                  30

Gln Glu Leu Ser Glu Ser Thr Tyr Thr Asp Val Leu Asp Gln Thr Cys
        35                  40                  45

Ser Gln Asn Trp Gln Ser Tyr Gly Val His Glu Val Asn Gln Met Lys
    50                  55                  60

Arg Leu Thr Gly Pro Gly Leu Ser Lys Gly Pro Glu Pro Arg Ile Ser
65                  70                  75                  80

Val Met Ile Ser Gly Gly Pro Trp Pro Asn Arg Leu Ser Lys Thr Cys
                85                  90                  95

Phe His Tyr Leu Gly Glu Phe Gly Glu Asp Gln Ile Tyr Glu Ala Tyr
            100                 105                 110

Arg Gln Gly Gln Ala Asn Leu Glu Ala Leu Leu Cys Gly Gly Thr His
        115                 120                 125

Gly Pro Cys Ser Gln Glu Ile Leu Ala Gln Arg Glu Glu Leu
    130                 135                 140
```

```
<210>  29
<211>  157
<212>  PRT
<213>  Mus musculus

<220>
<221>  SITE
<222>  (1)..(157)
<223>  part of smERp2

<400>  29
```

```
Leu His Cys Gly Ala Cys Arg Ala Leu Val Asp Glu Leu Glu Trp Glu
1               5                   10                  15

Ile Ala Arg Val Asp Pro Lys Lys Thr Ile Gln Met Gly Ser Phe Arg
            20                  25                  30

Ile Asn Pro Asp Gly Ser Gln Ser Val Val Glu Val Pro Tyr Ala Arg
        35                  40                  45

Ser Glu Ala His Leu Thr Glu Leu Leu Glu Glu Val Cys Asp Arg Met
    50                  55                  60

Lys Glu Tyr Gly Glu Gln Ile Asp Pro Ser Thr His Arg Lys Asn Tyr
65                  70                  75                  80
```

Val Arg Val Val Ser Arg Asn Gly Glu Ser Ser Glu Leu Asp Leu Gln
85 90 95

Gly Ile Arg Ile Asp Ser Asp Ile Ser Gly Thr Leu Lys Phe Ala Cys
100 105 110

Glu Ser Ile Val Glu Glu Tyr Glu Asp Glu Leu Ile Glu Phe Phe Ser
115 120 125

Arg Glu Ala Asp Asn Val Lys Asp Lys Leu Cys Ser Lys Arg Thr Asp
130 135 140

Leu Cys Asp His Ala Leu His Arg Ser His Asp Glu Leu
145 150 155

<210> 30
<211> 182
<212> PRT
<213> Mus musculus

<220>
<221> SITE
<222> (1)..(182)
<223> smERp2

<400> 30

Met Lys Gly Trp Gly Trp Leu Ala Leu Leu Leu Gly Val Leu Leu Gly
1 5 10 15

Thr Ala Trp Ala Arg Arg Ser Gln Asp Leu His Cys Gly Ala Cys Arg
20 25 30

Ala Leu Val Asp Glu Leu Glu Trp Glu Ile Ala Arg Val Asp Pro Lys
35 40 45

Lys Thr Ile Gln Met Gly Ser Phe Arg Ile Asn Pro Asp Gly Ser Gln
50 55 60

Ser Val Val Glu Val Pro Tyr Ala Arg Ser Glu Ala His Leu Thr Glu
65 70 75 80

Leu Leu Glu Glu Val Cys Asp Arg Met Lys Glu Tyr Gly Glu Gln Ile
85 90 95

Asp Pro Ser Thr His Arg Lys Asn Tyr Val Arg Val Val Ser Arg Asn
100 105 110

Gly Glu Ser Ser Glu Leu Asp Leu Gln Gly Ile Arg Ile Asp Ser Asp
115 120 125

Ile Ser Gly Thr Leu Lys Phe Ala Cys Glu Ser Ile Val Glu Glu Tyr

130 135 140

Glu Asp Glu Leu Ile Glu Phe Phe Ser Arg Glu Ala Asp Asn Val Lys
145 150 155 160

Asp Lys Leu Cys Ser Lys Arg Thr Asp Leu Cys Asp His Ala Leu His
165 170 175

Arg Ser His Asp Glu Leu
180

<210> 31
<211> 189
<212> PRT
<213> Drosophila melanogaster

<220>
<221> SITE
<222> (1)..(189)
<223> smERp2

<400> 31

Met Leu Thr Lys Ala Leu Ile Leu Phe Gly Leu Leu Ala Leu Ala Gln
1 5 10 15

Gly Tyr Ser Phe Thr Ser Arg Glu Val Lys Cys His Val Cys Lys Ala
20 25 30

Val Val Thr Glu Leu Glu Glu Ala Ile Ala Lys Glu Asp Pro His Lys
35 40 45

Met Ala Asp Val Ser Gly Phe Arg Leu Asp Ala Gln Gly Asn Ser Ile
50 55 60

Ser Lys Lys Val Arg Leu Val Lys Ser Glu Met Phe Leu Thr Glu Leu
65 70 75 80

Met Glu Lys Ile Cys Glu Lys Met Asp Asp Tyr Leu Lys Ala Thr Tyr
85 90 95

Lys Ser Asn Gly Lys Phe Thr Leu Leu Lys Met Ile Ile Asn Gly Gln
100 105 110

Met Asn Pro Asp Ser Ser Leu Val Asp Phe Val Gln Asp Gly Asp Leu
115 120 125

Asn Lys Ser Leu Gly His Phe Cys Asn Glu Val Leu Glu Asp Asn Asp
130 135 140

Glu Ile Phe Val Lys Ala Phe Gln Ala Glu Glu Leu Gly Asn Asp Leu
145 150 155 160

```
Asp Ile Lys Ile Cys Ser Glu Gln Ala Ser Tyr Cys Asp Glu Ser Pro
                165                 170                 175

Val Gln Glu Glu Tyr Asp Phe Asp Gly Lys Glu Glu Leu
            180                 185


<210>  32
<211>  182
<212>  PRT
<213>  Homo sapiens

<220>
<221>  SITE
<222>  (1)..(182)
<223>  smERp2


<400>  32

Met Lys Gly Trp Gly Trp Leu Ala Leu Leu Leu Gly Ala Leu Leu Gly
1               5                   10                  15

Thr Ala Trp Ala Arg Arg Ser Gln Asp Leu His Cys Gly Ala Cys Arg
            20                  25                  30

Ala Leu Val Asp Glu Leu Glu Trp Glu Ile Ala Gln Val Asp Pro Lys
        35                  40                  45

Lys Thr Ile Gln Met Gly Ser Phe Arg Ile Asn Pro Asp Gly Ser Gln
    50                  55                  60

Ser Val Val Glu Val Pro Tyr Ala Arg Ser Glu Ala His Leu Thr Glu
65                  70                  75                  80

Leu Leu Glu Glu Ile Cys Asp Arg Met Lys Glu Tyr Gly Glu Gln Ile
                85                  90                  95

Asp Pro Ser Thr His Arg Lys Asn Tyr Val Arg Val Val Gly Arg Asn
            100                 105                 110

Gly Glu Ser Ser Glu Leu Asp Leu Gln Gly Ile Arg Ile Asp Ser Asp
        115                 120                 125

Ile Ser Gly Thr Leu Lys Phe Ala Cys Glu Ser Ile Val Glu Glu Tyr
    130                 135                 140

Glu Asp Glu Leu Ile Glu Phe Phe Ser Arg Glu Ala Asp Asn Val Lys
145                 150                 155                 160

Asp Lys Leu Cys Ser Lys Arg Thr Asp Leu Cys Asp His Ala Leu His
                165                 170                 175

Ile Ser His Asp Glu Leu
            180
```

<210> 33
<211> 193
<212> PRT
<213> Caenorhabditis elegans

<220>
<221> SITE
<222> (1)..(193)
<223> smERp2

<400> 33

Met His Phe Val Ser Thr Leu Val Cys Leu Ile Ser Leu Ser Gly Ile
1 5 10 15

Gln Ser Ala Ser Val Ser Ser Leu Glu Cys Gly Ala Cys Ser Leu Leu
20 25 30

Val Thr His Phe Glu Leu Lys Ile Ala Ala Val Asp Pro Lys Lys Lys
35 40 45

Ile Glu Val Gly Ser Phe Arg Val Ser Pro Thr Gly Asp Gln Lys Gly
50 55 60

Leu Lys Glu Ile Gly Tyr Ala Arg Ser Glu Ser His Leu Thr Glu Ile
65 70 75 80

Ile Glu His Ala Cys Asp Asp Ala Lys His Tyr Lys Leu Val Val Asn
85 90 95

Thr Ile Thr Gly Lys Ser Val Tyr Val His Asn Asp Ala Thr His Leu
100 105 110

Lys Gly Asp Glu Ser Pro Lys Met Arg Tyr Asn Leu Gln Asn Ala Cys
115 120 125

Asn Asp Phe Ile Asp Ser His Glu Asp Glu Leu Leu Gly Phe Leu Lys
130 135 140

Thr Ala His Gln Asp Pro Val Lys Gln Phe Cys Gln Gly Glu Met Gly
145 150 155 160

Ala Cys Thr Ala Val Asp Val Ala Pro Leu Pro Ala Ala Pro Glu Glu
165 170 175

Pro Met Asp Leu Ser Asp Ile Pro Asp Asp Glu Ile Asp Arg Lys Glu
180 185 190

Leu

<210> 34
<211> 138

<212> PRT
<213> Mus musculus

<220>
<221> SITE
<222> (1)..(138)
<223> part of smERp2

<400> 34

Cys Gly Ala Cys Arg Ala Leu Val Asp Glu Leu Glu Trp Glu Ile Ala
1 5 10 15

Arg Val Asp Pro Lys Lys Thr Ile Gln Met Gly Ser Phe Arg Ile Asn
20 25 30

Pro Asp Gly Ser Gln Ser Val Val Glu Val Pro Tyr Ala Arg Ser Glu
35 40 45

Ala His Leu Thr Glu Leu Leu Glu Glu Val Cys Asp Arg Met Lys Glu
50 55 60

Tyr Gly Glu Gln Ile Asp Pro Ser Thr His Arg Lys Asn Tyr Val Arg
65 70 75 80

Val Val Ser Arg Asn Gly Glu Ser Ser Glu Leu Asp Leu Gln Gly Ile
85 90 95

Arg Ile Asp Ser Asp Ile Ser Gly Thr Leu Lys Phe Ala Cys Glu Ser
100 105 110

Ile Val Glu Glu Tyr Glu Asp Glu Leu Ile Glu Phe Phe Ser Arg Glu
115 120 125

Ala Asp Asn Val Lys Asp Lys Leu Cys Ser
130 135

<210> 35
<211> 147
<212> PRT
<213> Mus musculus

<220>
<221> SITE
<222> (1)..(147)
<223> part of smERp3

<400> 35

Cys Glu Val Cys Lys Tyr Val Ala Val Glu Leu Lys Ser Ala Phe Glu
1 5 10 15

Glu Thr Gly Lys Thr Lys Glu Val Ile Asp Thr Gly Tyr Gly Ile Leu
20 25 30

```
Asp Gly Lys Gly Ser Gly Val Lys Tyr Thr Lys Ser Asp Leu Arg Leu
        35                  40                  45

Ile Glu Val Thr Glu Thr Ile Cys Lys Arg Leu Leu Asp Tyr Ser Leu
    50                  55                  60

His Lys Glu Arg Thr Gly Ser Asn Arg Phe Ala Lys Gly Met Ser Glu
65                  70                  75                  80

Thr Phe Glu Thr Leu His Asn Leu Val His Lys Gly Val Lys Val Val
                85                  90                  95

Met Asp Ile Pro Tyr Glu Leu Trp Asn Glu Thr Ser Ala Glu Val Ala
            100                 105                 110

Asp Leu Lys Lys Gln Cys Asp Val Leu Val Glu Glu Phe Glu Glu Val
        115                 120                 125

Ile Glu Asp Trp Tyr Arg Asn His Gln Glu Glu Asp Leu Thr Glu Phe
    130                 135                 140

Leu Cys Ala
145
```

<210> 36
<211> 276
<212> PRT
<213> Mus musculus

<220>
<221> SITE
<222> (1)..(276)
<223> smERp3

<400> 36

```
Arg Leu Pro Ser Lys Cys Glu Val Cys Lys Tyr Val Ala Val Glu Leu
1               5                   10                  15

Lys Ser Ala Phe Glu Glu Thr Gly Lys Thr Lys Met Glu Ser Met Ser
            20                  25                  30

Glu Leu Ala Pro Arg Cys Leu Leu Phe Pro Leu Leu Leu Leu Leu Pro
        35                  40                  45

Leu Leu Leu Leu Pro Ala Pro Lys Leu Gly Pro Ser Pro Ala Gly Ala
    50                  55                  60

Glu Glu Thr Asp Trp Val Glu Val Ile Asp Thr Gly Tyr Gly Ile Leu
65                  70                  75                  80

Asp Gly Lys Gly Ser Gly Val Lys Tyr Thr Lys Ser Asp Leu Arg Leu
                85                  90                  95
```

```
Ile Glu Val Thr Glu Thr Ile Cys Lys Arg Leu Leu Asp Tyr Ser Leu
            100                 105                 110

His Lys Glu Arg Thr Gly Ser Asn Arg Phe Ala Lys Gly Met Ser Glu
        115                 120                 125

Thr Phe Glu Thr Leu His Asn Leu Val His Lys Gly Val Lys Val Val
    130                 135                 140

Met Asp Ile Pro Tyr Glu Leu Trp Asn Glu Thr Ser Ala Glu Val Ala
145                 150                 155                 160

Asp Leu Lys Lys Gln Cys Asp Val Leu Val Glu Glu Phe Glu Glu Val
                165                 170                 175

Ile Glu Asp Trp Tyr Arg Asn His Gln Glu Glu Asp Leu Thr Glu Phe
            180                 185                 190

Leu Cys Ala Asn His Val Leu Lys Gly Lys Asp Thr Ser Cys Leu Ala
        195                 200                 205

Glu Arg Trp Ser Gly Lys Lys Gly Asp Ile Ala Ser Leu Gly Gly Lys
    210                 215                 220

Lys Ser Lys Lys Lys Arg Ser Gly Val Lys Gly Ser Ser Ser Gly Ser
225                 230                 235                 240

Ser Lys Gln Arg Lys Glu Leu Gly Gly Leu Gly Glu Asp Ala Asn Ala
                245                 250                 255

Glu Glu Glu Glu Gly Val Gln Lys Ala Ser Pro Leu Pro His Ser Pro
            260                 265                 270

Pro Asp Glu Leu
        275
```

<210> 37
<211> 221
<212> PRT
<213> Drosophila melanogaster

<220>
<221> SITE
<222> (1)..(221)
<223> smERp3

<400> 37

```
Met Leu Leu Lys Arg Leu Pro Gly Phe Val Thr Leu Trp Val Val Leu
1               5                   10                  15

Gln Leu Ala Gly Ala Asp Ser Pro Glu Glu Glu Gln Gly Val Arg Tyr
            20                  25                  30
```

Ala Asn Arg Cys Glu Ala Cys Lys Ile Leu Ala Thr Glu Leu Glu Ala
        35                  40                  45

Arg Leu Gly Glu Thr Gly Lys Ser His Asp Val Ile Glu Ile Gly Tyr
    50                  55                  60

Ser Val Asp Asp Val Lys Pro Lys Lys Arg Thr Glu Tyr Arg Arg Ser
65                  70                  75                  80

Glu Leu Arg Leu Leu Glu Ser Leu Glu Asn Val Cys Glu Arg Val Leu
                85                  90                  95

Glu Tyr Asn Leu His Lys Glu Arg Ser Asp Ser Thr Arg Phe Ala Lys
            100                 105                 110

Gly Met Ser Gln Thr Phe Gln Thr Leu His Gly Leu Val Asp Lys Gly
        115                 120                 125

Val Lys Val Asp Leu Gly Ile Pro Tyr Glu Leu Trp Asp Lys Pro Pro
    130                 135                 140

Val Glu Val Thr Gln Met Lys Thr Gln Cys Glu Asn Leu Leu Glu Glu
145                 150                 155                 160

Tyr Glu Glu Thr Ile Ser Glu Trp Tyr Phe Lys His Gln Asp Glu Lys
                165                 170                 175

Ser Leu Lys Lys His Leu Cys Glu Asp His Val Leu Lys Lys Lys Ala
            180                 185                 190

Glu Arg Glu Cys Leu Lys Glu Gln Leu Ala Pro Pro Glu Ala Lys Lys
        195                 200                 205

Ala Lys Arg Glu Lys Ala Lys Gly Asp Lys Glu Glu Leu
    210                 215                 220

<210> 38
<211> 278
<212> PRT
<213> Homo sapiens

<220>
<221> SITE
<222> (1)..(278)
<223> smERp3

<400> 38

Met Asp Ser Met Pro Glu Pro Ala Ser Arg Cys Leu Leu Leu Leu Pro
1               5                   10                  15

Leu Leu Leu Leu Leu Leu Leu Leu Leu Pro Ala Pro Glu Leu Gly Pro

```
                20                  25                  30

Ser Gln Ala Gly Ala Glu Glu Asn Asp Trp Val Arg Leu Pro Ser Lys
        35                  40                  45

Cys Glu Val Cys Lys Tyr Val Ala Val Glu Leu Lys Ser Ala Phe Glu
    50                  55                  60

Glu Thr Gly Lys Thr Lys Glu Val Ile Gly Thr Gly Tyr Gly Ile Leu
65                  70                  75                  80

Asp Gln Lys Ala Ser Gly Val Lys Tyr Thr Lys Ser Asp Leu Arg Leu
                85                  90                  95

Ile Glu Val Thr Glu Thr Ile Cys Lys Arg Leu Leu Asp Tyr Ser Leu
            100                 105                 110

His Lys Glu Arg Thr Gly Ser Asn Arg Phe Ala Lys Gly Met Ser Glu
        115                 120                 125

Thr Phe Glu Thr Leu His Asn Leu Val His Lys Gly Val Lys Val Val
    130                 135                 140

Met Asp Ile Pro Tyr Glu Leu Trp Asn Glu Thr Ser Ala Glu Val Ala
145                 150                 155                 160

Asp Leu Lys Lys Gln Cys Asp Val Leu Val Glu Glu Phe Glu Glu Val
                165                 170                 175

Ile Glu Asp Trp Tyr Arg Asn His Gln Glu Glu Asp Leu Thr Glu Phe
            180                 185                 190

Leu Cys Ala Asn His Val Leu Lys Gly Lys Asp Thr Ser Cys Leu Ala
        195                 200                 205

Glu Gln Trp Ser Gly Lys Lys Gly Asp Thr Ala Ala Leu Gly Gly Lys
    210                 215                 220

Lys Ser Lys Lys Lys Ser Ser Arg Ala Lys Ala Ala Gly Gly Arg Ser
225                 230                 235                 240

Ser Ser Ser Lys Gln Arg Lys Glu Leu Gly Gly Leu Glu Gly Asp Pro
                245                 250                 255

Ser Pro Glu Glu Asp Glu Gly Ile Gln Lys Ala Ser Pro Leu Thr His
            260                 265                 270

Ser Pro Pro Asp Glu Leu
        275
```

<210> 39

<211> 191
<212> PRT
<213> Caenorhabditis elegans

<220>
<221> SITE
<222> (1)..(191)
<223> smERp3

<400> 39

Met Pro Lys Leu Tyr Leu Leu Ile Pro Leu Tyr Leu Leu Ala Glu Phe
1 5 10 15

Ala Lys Cys Asn Thr Asp Thr Thr Phe Met Pro Thr Lys Cys Glu Ile
20 25 30

Cys Ala Leu Thr Ser Met Glu Phe Glu Ala Lys Ser Val Lys Val His
35 40 45

Lys Gln Thr Arg Leu Lys Phe Leu Phe Ile Trp Phe Phe Leu Phe Gln
50 55 60

Arg Leu Ser Ser Glu Phe Ala Asp Ile Thr Glu Asn Ile Cys Asn Gly
65 70 75 80

Phe Asn Glu Phe Lys Ile His Lys Glu Lys Thr Gly Leu Glu Arg Phe
85 90 95

Ser Arg Ala Gln Ser Lys Thr Ile Glu Thr Leu Lys Gln Met Arg Glu
100 105 110

Lys Gly Val Lys Val Glu Leu Gly Met Pro Phe Glu Met Trp Asp Gln
115 120 125

Pro Ser Ala Glu Ile Phe Ala Leu Arg Gln Gly Cys Glu Ser Leu Leu
130 135 140

Glu Asp Tyr Glu Asp Leu Ile Glu Glu Trp Phe Ile Asn Lys Ala Ser
145 150 155 160

Leu Glu Asp Leu Phe Lys Gln Leu Cys Ala Arg Asn Ala Leu Lys Asn
165 170 175

Gln Glu Thr Ser Cys Phe Asp His Leu Asp His Lys Gln Glu Leu
180 185 190

<210> 40
<211> 233
<212> PRT
<213> Arabidopsis thaliana

<220>
<221> SITE
<222> (1)..(233)

<223> smERp

<400> 40

Met Ala Lys Leu Val Leu Phe Thr Ala Val Ile Ile Ala Ile Phe Ser
1 5 10 15

His Gly Ser Ser Val Asp Asp Lys Cys Ala Ala Cys Asn Ala Val Ala
20 25 30

Glu Glu Leu Glu Leu Gln Leu Leu Lys Glu Lys Pro Arg Asn His Leu
35 40 45

Asp Met Arg Asn Arg Leu Asn Ser Lys Gly Gln Arg Glu Gly Lys Leu
50 55 60

Gly Ala Met Thr Lys Gly Lys Glu Ser Asp Arg Leu Thr Leu Leu Arg
65 70 75 80

Val Ser Val Lys Gln Gln Pro Ile His Asn Glu Phe Leu Gly Val Ala
85 90 95

Met Leu Leu Phe Leu Gly Arg Val Val Asp Leu Leu Asp Gly Leu Cys
100 105 110

Asp Arg Met Gln Asp Tyr Thr Leu Gln Lys Val Glu Pro Lys Asn Arg
115 120 125

Gln Trp Val Lys Val Gly Asn Phe Asp Asn Leu Thr Ile Arg Leu Gly
130 135 140

Ala Ser Phe Asn Glu Ser Met Glu Ala Val Tyr Leu Ile Thr Leu Ser
145 150 155 160

Pro Tyr Lys Gln Glu Ala Lys Ala His Ala Asn Asp Ile Ser Thr Tyr
165 170 175

Cys Gly Arg Leu Leu Glu Glu Thr Glu Asp Glu Leu Gly Glu Val Ile
180 185 190

Lys Asn Gly Ser Leu Lys Ala Gly Glu Val Arg Lys Val Leu Cys Gln
195 200 205

Thr Leu Ser Asn His Cys Arg Gln Ser Ser Glu Thr Asp Ser Glu Asp
210 215 220

Glu Glu Asp Asp Asp Ala Asp Glu Leu
225 230

<210> 41
<211> 417
<212> PRT

<213> Mus musculus

<220>
<221> SITE
<222> (1)..(417)
<223> ERp43 sequence

<400> 41

```
Met Pro Pro Arg Pro Gly Arg Leu Leu Gln Pro Leu Ala Gly Leu Pro
1               5                   10                  15

Ala Leu Ala Thr Leu Leu Leu Leu Leu Gly Ala Arg Lys Gly Ala Arg
            20                  25                  30

Ala Gln Glu Val Glu Ala Asp Ser Gly Val Glu Gln Asp Pro His Ala
        35                  40                  45

Lys His Leu Tyr Thr Ala Asp Met Phe Thr His Gly Ile Gln Ser Ala
    50                  55                  60

Ala His Phe Val Met Phe Phe Ala Pro Trp Cys Gly His Cys Gln Arg
65                  70                  75                  80

Leu Gln Pro Thr Trp Asn Asp Leu Gly Asp Lys Tyr Asn Ser Met Glu
            85                  90                  95

Asp Ala Lys Val Tyr Val Ala Lys Val Asp Cys Thr Ala Asp Ser Asp
            100                 105                 110

Val Cys Ser Ala Gln Gly Val Arg Gly Tyr Pro Thr Leu Lys Phe Phe
        115                 120                 125

Lys Pro Gly Gln Glu Ala Val Lys Tyr Gln Gly Pro Arg Asp Phe Glu
    130                 135                 140

Thr Leu Glu Asn Trp Met Leu Gln Thr Leu Asn Glu Glu Pro Ala Thr
145                 150                 155                 160

Pro Glu Pro Glu Ala Glu Pro Pro Arg Ala Pro Glu Leu Lys Gln Gly
                165                 170                 175

Leu Tyr Glu Leu Ser Ala Asn Asn Phe Glu Leu His Val Ser Gln Gly
            180                 185                 190

Asn His Phe Ile Lys Phe Phe Ala Pro Trp Cys Gly His Cys Lys Ala
        195                 200                 205

Leu Ala Pro Thr Trp Glu Gln Leu Ala Leu Gly Leu Glu His Ser Glu
    210                 215                 220

Thr Val Lys Ile Gly Lys Val Asp Cys Thr Gln His Tyr Ala Val Cys
225                 230                 235                 240
```

```
Ser Glu His Gln Val Arg Gly Tyr Pro Thr Leu Leu Trp Phe Arg Asp
                245                 250                 255

Gly Lys Lys Val Asp Gln Tyr Lys Gly Lys Arg Asp Leu Glu Ser Leu
            260                 265                 270

Arg Asp Tyr Val Gln Ser Gln Leu Gln Gly Ser Glu Ala Ala Pro Glu
        275                 280                 285

Thr Val Glu Pro Ser Glu Ala Pro Val Met Ala Ala Glu Pro Thr Gly
    290                 295                 300

Asp Lys Gly Thr Val Leu Ala Leu Thr Glu Lys Ser Phe Glu Asp Thr
305                 310                 315                 320

Ile Ala Gln Gly Ile Thr Phe Val Lys Phe Tyr Ala Pro Trp Cys Gly
                325                 330                 335

His Cys Lys Asn Leu Ala Pro Thr Trp Glu Glu Leu Ser Lys Lys Glu
            340                 345                 350

Phe Pro Gly Leu Ser Asp Val Thr Ile Ala Glu Val Asp Cys Thr Ala
        355                 360                 365

Glu Arg Asn Val Cys Ser Lys Tyr Ser Val Arg Gly Tyr Pro Thr Leu
    370                 375                 380

Leu Leu Phe Arg Gly Gly Glu Lys Val Gly Glu His Asn Gly Gly Arg
385                 390                 395                 400

Asp Leu Asp Ser Leu His Ser Phe Val Leu Arg Gln Ala Lys Asp Glu
                405                 410                 415

Leu


<210>  42
<211>  432
<212>  PRT
<213>  Homo sapiens

<220>
<221>  SITE
<222>  (1)..(432)
<223>  ERp43

<400>  42

Met Pro Ala Arg Pro Gly Arg Leu Leu Pro Leu Leu Ala Arg Pro Ala
1               5                   10                  15

Ala Leu Thr Ala Leu Leu Leu Leu Leu Leu Gly His Gly Gly Gly Gly
```

20 25 30

Arg Trp Gly Ala Arg Ala Gln Glu Ala Ala Ala Ala Ala Ala Asp Gly
35 40 45

Pro Ala Ala Asp Gly Glu Asp Gly Gln Asp Pro His Ser Lys His Leu
50 55 60

Tyr Thr Ala Asp Met Phe Thr His Gly Ile Gln Ser Ala Ala His Phe
65 70 75 80

Val Met Phe Phe Ala Pro Trp Cys Gly His Cys Gln Arg Leu Gln Pro
85 90 95

Thr Trp Asn Asp Leu Gly Asp Lys Tyr Asn Ser Met Glu Asp Ala Lys
100 105 110

Val Tyr Val Ala Lys Val Asp Cys Thr Ala His Ser Asp Val Cys Ser
115 120 125

Ala Gln Gly Val Arg Gly Tyr Pro Thr Leu Lys Leu Phe Lys Pro Gly
130 135 140

Gln Glu Ala Val Lys Tyr Gln Gly Pro Arg Asp Phe Gln Thr Leu Glu
145 150 155 160

Asn Trp Met Leu Gln Thr Leu Asn Glu Glu Pro Val Thr Pro Glu Glu
165 170 175

Pro Glu Val Glu Pro Pro Ser Ala Pro Glu Leu Lys Gln Gly Leu Tyr
180 185 190

Glu Leu Ser Ala Ser Asn Phe Glu Leu His Val Ala Gln Gly Asp His
195 200 205

Phe Ile Lys Phe Phe Ala Pro Trp Cys Gly His Cys Lys Ala Leu Ala
210 215 220

Pro Thr Trp Glu Gln Leu Ala Leu Gly Leu Glu His Ser Glu Thr Val
225 230 235 240

Lys Ile Gly Lys Val Asp Cys Thr Gln His Tyr Glu Leu Cys Ser Gly
245 250 255

Asn Gln Val Arg Gly Tyr Pro Thr Leu Leu Trp Phe Arg Asp Gly Lys
260 265 270

Lys Val Asp Gln Tyr Lys Gly Lys Arg Asp Leu Glu Ser Leu Arg Glu
275 280 285

Tyr Val Glu Ser Gln Leu Gln Arg Thr Glu Thr Gly Ala Thr Glu Thr

290 . 295 300

Val Thr Pro Ser Glu Ala Pro Val Leu Ala Ala Glu Pro Glu Ala Asp
305 310 315 320

Lys Gly Thr Val Leu Ala Leu Thr Glu Asn Asn Phe Asp Asp Thr Ile
325 330 335

Ala Glu Gly Ile Thr Phe Ile Lys Phe Tyr Ala Pro Trp Cys Gly His
340 345 350

Cys Lys Thr Leu Ala Pro Thr Trp Glu Glu Leu Ser Lys Lys Glu Phe
355 360 365

Pro Gly Leu Ala Gly Val Lys Ile Ala Glu Val Asp Cys Thr Ala Glu
370 375 380

Arg Asn Ile Cys Ser Lys Tyr Ser Val Arg Gly Tyr Pro Thr Leu Leu
385 390 395 400

Leu Phe Arg Gly Gly Lys Lys Val Ser Glu His Ser Gly Gly Arg Asp
405 410 415

Leu Asp Ser Leu His Arg Phe Val Leu Ser Gln Ala Lys Asp Glu Leu
420 425 430

<210> 43
<211> 403
<212> PRT
<213> Xenopus laevis

<220>
<221> SITE
<222> (1)..(403)
<223> ERp43

<400> 43

Met Leu Arg Cys Pro Ser Ala Val Leu Leu Cys Ala Leu Leu Leu Arg
1 5 10 15

Val Ser Gly Glu Pro Trp Glu Glu Gly Ala Asp Asp Asp Pro His Lys
20 25 30

Lys Asn Leu Tyr Ser Ala Asp Met Phe Asp His Ala Val Lys Gln Glu
35 40 45

Pro His Phe Ile Met Phe Phe Ala Pro Trp Cys Gly His Cys Gln Arg
50 55 60

Leu Gln Ser Thr Trp Asn Glu Leu Gly Asp Lys Tyr Asn Thr Met Pro
65 70 75 80

```
Asn Thr Pro Ala Tyr Ile Ala Lys Val Asp Cys Thr Thr Asp Met Pro
                85                  90                  95

Thr Cys Thr Asn His Gly Val Arg Gly Tyr Pro Thr Leu Lys Leu Phe
            100                 105                 110

Lys Pro Gly Gln Glu Ala Val Lys Tyr Gln Gly Pro Arg Asp Leu Gln
        115                 120                 125

Ser Leu Glu Asn Trp Met Leu Gln Thr Leu Asn Ala Glu Ala Glu Lys
    130                 135                 140

Pro Lys Val Glu Glu Lys Ala Glu Asp Pro Ala Lys Val Pro Glu Leu
145                 150                 155                 160

Lys Gln Gly Leu Tyr Glu Leu Thr Gly Ala Asn Phe Lys Glu His Val
                165                 170                 175

Ala Glu Gly Tyr His Phe Ile Lys Phe Phe Ala Pro Trp Cys Gly His
            180                 185                 190

Cys Lys Ser Leu Ala Pro Ala Trp Glu Gln Leu Ala Ala Ser Phe Gln
        195                 200                 205

Asp Ser Lys Ser Val Lys Ile Ala Lys Val Asp Cys Thr Gln His Asn
    210                 215                 220

Glu Leu Cys Ser Glu Tyr Gln Val Arg Gly Tyr Pro Thr Leu Leu Trp
225                 230                 235                 240

Phe Arg Asn Gly Glu Lys Val Asp Gln Tyr Lys Gly Lys Arg Asp Leu
                245                 250                 255

Asp Thr Met Lys Glu Tyr Ala Glu Ser Gln Leu Lys Pro Ala Glu Glu
            260                 265                 270

Lys Lys Glu Glu Glu Gln Lys Lys Glu Ala Thr Pro Pro Gln Val Gln
        275                 280                 285

Glu Thr Val Glu Ser Lys Val Leu Ser Leu Ser Glu Ser Asn Phe Asp
    290                 295                 300

Gln Thr Val Ala Thr Gly Val Ser Phe Ile Lys Phe Tyr Ala Pro Trp
305                 310                 315                 320

Cys Gly His Cys Lys Asn Leu Ala Pro Ile Trp Glu Asp Leu Ala Lys
                325                 330                 335

Lys Glu Phe Ser Gly Met Ser Asp Val Lys Ile Ala Lys Val Asp Cys
            340                 345                 350
```

Thr Ala Glu Arg Ser Leu Cys Ser Arg Phe Ser Val Arg Gly Tyr Pro
355 360 365

Ser Leu Leu Leu Phe Arg Ala Gly Glu Arg Ile Gly Glu His Glu Gly
370 375 380

Ala Arg Asp Leu Glu Thr Leu Gln Asn Phe Val Leu Arg His Ser Arg
385 390 395 400

Asp Glu Leu

<210> 44
<211> 415
<212> PRT
<213> Drosophila melanogaster

<220>
<221> SITE
<222> (1)..(415)
<223> ERp43

<400> 44

Met Leu Thr Arg Ser Ile Leu Ser Val Ala Val Cys Gly Leu Leu Leu
1 5 10 15

Ser Pro Leu Leu Pro Ile Thr Arg Ala Ser Gln Glu Glu Asp Thr Gly
20 25 30

Lys Gln Asp Lys Gln Phe Thr Val Glu Leu Asp Pro Glu Thr Phe Asp
35 40 45

Thr Ala Ile Ala Gly Gly Asn Val Phe Val Lys Phe Phe Ala Pro Trp
50 55 60

Cys His Gln Lys Arg Ile Gln Pro Leu Trp Glu Gln Leu Ala Glu Ile
65 70 75 80

Met Asn Val Asp Asn Pro Lys Val Ile Ile Ala Lys Val Asp Cys Thr
85 90 95

Lys His Gln Gly Leu Gln Ala Thr His Gln Val Thr Gly Tyr Pro Thr
100 105 110

Leu Arg Leu Phe Lys Leu Gly Glu Glu Glu Ser Val Lys Phe Lys Gly
115 120 125

Thr Arg Asp Leu Pro Ala Ile Thr Asp Phe Ile Asn Lys Glu Leu Ser
130 135 140

Ala Pro Ala Glu Ala Asp Leu Gly Glu Val Lys Arg Glu Gln Val Glu
145 150 155 160

```
Asn Ile Asn Ile Gly Lys Val Val Asp Leu Thr Glu Asp Thr Phe Ala
                165                 170                 175

Lys His Val Ser Thr Gly Asn His Phe Val Lys Phe Phe Ala Pro Trp
            180                 185                 190

Cys Ser His Cys Gln Arg Leu Ala Pro Thr Trp Glu Asp Leu Ala Lys
        195                 200                 205

Glu Leu Ile Lys Glu Pro Thr Val Thr Ile Ser Lys Ile Asp Cys Thr
    210                 215                 220

Gln Phe Arg Ser Ile Cys Gln Asp Phe Glu Val Lys Gly Tyr Pro Thr
225                 230                 235                 240

Leu Leu Trp Ile Glu Asp Gly Lys Lys Ile Glu Lys Tyr Ser Gly Ala
                245                 250                 255

Arg Asp Leu Ser Thr Leu Lys Thr Tyr Val Glu Lys Met Val Gly Val
            260                 265                 270

Pro Leu Glu Lys Thr Ala Gly Glu Ala Gly Asp Glu Lys Val Val Ile
        275                 280                 285

Glu Glu Val Ala Gly Glu Glu Asp Ala Ala Lys Lys Leu Thr Pro Gln
    290                 295                 300

Gln Leu Thr Gly Glu Asp Glu Phe Asp Gln Ala Ile Ala Glu Gly Val
305                 310                 315                 320

Ala Phe Ile Lys Phe Tyr Ala Pro Trp Cys Gly His Cys Gln Lys Leu
                325                 330                 335

Gln Pro Thr Trp Glu Gln Leu Ala Thr Glu Thr His Gln Ala Gln Ser
            340                 345                 350

Ser Val Lys Ile Ala Lys Val Asp Cys Thr Ala Pro Glu Asn Lys Gln
        355                 360                 365

Val Cys Ile Asp Gln Gln Val Glu Gly Tyr Pro Thr Leu Phe Leu Tyr
    370                 375                 380

Lys Asn Gly Gln Arg Gln Asn Glu Tyr Glu Gly Ser Arg Ser Leu Pro
385                 390                 395                 400

Glu Leu Gln Ala Tyr Leu Lys Lys Phe Leu Gly His Asp Glu Leu
                405                 410                 415
```

<210> 45
<211> 20
<212> DNA

<213> Mus musculus

<220>
<221> misc_feature
<222> (1)..(20)
<223> intron/exon boundary

<400> 45
tcgcgccctg gtaactgcag 20

<210> 46
<211> 20
<212> DNA
<213> Mus musculus

<220>
<221> misc_feature
<222> (1)..(20)
<223> intron/exon boundary

<400> 46
ttccctgtag gtgtggacac 20

<210> 47
<211> 20
<212> DNA
<213> Mus musculus

<220>
<221> misc_feature
<222> (1)..(20)
<223> intron/exon boundary

<400> 47
gataccccac gtgagtaagg 20

<210> 48
<211> 20
<212> DNA
<213> Mus musculus

<220>
<221> misc_feature
<222> (1)..(20)
<223> intron/exon boundary

<400> 48
cttctttcag cctgaagttt 20

<210> 49
<211> 20
<212> DNA
<213> Mus musculus

<220>
<221> misc_feature
<222> (1)..(20)
<223> intron/exon boundary

<400> 49
ggagccagcc gtgagtgtcc 20

<210> 50
<211> 20
<212> DNA
<213> Mus musculus

<220>
<221> misc_feature
<222> (1)..(20)
<223> intron/exon boundary

<400> 50
cttccattag acaccggagc 20

<210> 51
<211> 20
<212> DNA
<213> Mus musculus

<220>
<221> misc_feature
<222> (1)..(20)
<223> intron/exon boundary

<400> 51
gtttctcaag gtaaggatgg 20

<210> 52
<211> 20
<212> DNA
<213> Mus musculus

<220>
<221> misc_feature
<222> (1)..(20)
<223> intron/exon boundary

<400> 52
ttccccgcag gcaaccactt 20

<210> 53
<211> 20
<212> DNA
<213> Mus musculus

<220>
<221> misc_feature
<222> (1)..(20)
<223> intron/exon boundary

<400> 53
gattggcaag gtaagtccaa 20

<210> 54
<211> 20
<212> DNA
<213> Mus musculus

<220>
<221> misc_feature
<222> (1)..(20)
<223> intron/exon boundary

<400> 54
ctgttttcag gttgactgca 20

<210> 55
<211> 20
<212> DNA
<213> Mus musculus

<220>
<221> misc_feature
<222> (1)..(20)
<223> intron/exon boundary

<400> 55
tggcaagaag gtatgtcttt 20

<210> 56
<211> 20
<212> DNA
<213> Mus musculus

<220>
<221> misc_feature
<222> (1)..(20)
<223> intron/exon boundary

<400> 56
tcttggacag gtggatcagt 20

<210> 57
<211> 20
<212> DNA
<213> Mus musculus

<220>
<221> misc_feature
<222> (1)..(20)
<223> intron/exon boundary

<400> 57
gggtgacaag gtaggtgttt 20

<210> 58
<211> 20
<212> DNA
<213> Mus musculus

<220>
<221> misc_feature
<222> (1)..(20)
<223> intron/exon boundary

<400> 58

tcccctgaag ggcactgtgc 20

<210> 59
<211> 20
<212> DNA
<213> Mus musculus

<220>
<221> misc_feature
<222> (1)..(20)
<223> intron/exon boundary

<400> 59
atgctccgtg gtaagttgcc 20

<210> 60
<211> 20
<212> DNA
<213> Mus musculus

<220>
<221> misc_feature
<222> (1)..(20)
<223> intron/exon boundary

<400> 60
tctcttctag gtgtggccac 20

<210> 61
<211> 20
<212> DNA
<213> Mus musculus

<220>
<221> misc_feature
<222> (1)..(20)
<223> intron/exon boundary

<400> 61
caagtactcg gtgggtacct 20

<210> 62
<211> 20
<212> DNA
<213> Mus musculus

<220>
<221> misc_feature
<222> (1)..(20)
<223> intron/exon boundary

<400> 62
tcctttaaag gtacgaggtt 20

<210> 63
<211> 1800
<212> DNA
<213> Mus musculus

<220>
<221> misc_feature
<222> (1)..(1800)
<223> promoter region ERp43

<400> 63
taaacattta caggtggacg aacctaacat gagaatagtt acagatgatg tgagtttccc 60
tgcccctccc tttaccacca cccccaactg ttgctaaccc aggcttttgg tttagggagg 120
ctgaggctaa taaagatcag gtgattttca gaagtgttgc tcagaagtcc aatacaagat 180
ggccatgtac tcttcttact atgcccgttg ctttttccca ataaaagatc accatatata 240
tgtcttatga tgccagcttc cttttcatcg gtacagagca tagctctagg acctctctca 300
agtgtctagt ggttgtttcg ttctccctaa cttagctcca ggttttcaag gcagggcagg 360
gagcttagat tgccacccga cttaaagttc aagagttggc gtgggtccat gcgccccagc 420
ccaggcgtta gctctcaccc agaacagctg ttgccattca ttttcctatc cagggtgagt 480
tagggcacac agggcactag ccagggcctg aaggaagggc cactttgact gcctttcctt 540
ttcaaagtga aggcagggca ggataacaga ggaggtgctc ttgggaagtg gtttcatcac 600
accccctgag gccaggccct caaatcacag ttgagctttg gctaagaatc tgacttatgc 660
aagagcaaac cccacacacg tttgcctgat ttcctccttt ctgctgacag cagctcagct 720
tggtcctaaa ctctcgggtc tcttggtgtc tgattagtta tcaacagaag ctgccactgt 780
tccatagctt ggcagcttgg cctcttcgtc cctagcaggg aaaggctgat gaggagcttg 840
ggaaggagaa tgagctcagc gggatggaag ggaaccggac tgagatagcg ctggaggtgc 900
tgcacaagga ccccaggacc taagcgacct cttcctggtg gctttcagca gaagacagca 960
agttgctagg tgagccggcg ggacaggggg cgtggctctg cgagcggggg cgtgattgag 1020
gcgtggctct gcggtcgtgg gaggagccgg ggcgggagcg gcgccgcgga ggggacccgc 1080
ggcacgagcg agagctcgcc agccccgcca cgatgccccc gcgcccagga cgcctcctcc 1140
agccgctggc cgggctgccg gccctggcca cgctcctgct gctgctcggg gcgcgcaaag 1200
gcgcccgggc ccaggaggtg gaagcggaca gcggggtcga gcaggacccg cacgccaagc 1260
acctgtatac ggccgacatg ttcacgcacg ggatccagag cgccgcgcac ttcgtcatgt 1320
tcttcgcgcc ctggtaactg cagctccagc aggccggcgg agcccgatct gggaccgggg 1380
accagggacc tggagccggg gaccggggag agggcgcgag gccgcgcagg ctgcgccggg 1440
ggcgtgggcc gcgctcgggc ctcgccgctg acgtggcgct agccgccggc ctgcgagcta 1500
ggaagggcgg gccccgaggg gagacccgga aaagcctctc caccccgcca cactgctgga 1560
gtccaggcat cccccgcttc tgtcccctgc ccagctcccc aaacttcctg tgctggccgg 1620
acgcggcggg agcccggcgt ccacgtgccc tatcctgaag ggttcttcct gctggcccat 1680
caatattggg ttggattgga aatctaaggc cccctcaaga tgtgattgcc cactttgtgt 1740
gtcgagggta ggaaaagata gcctaggtcg ggaactgaga cttccccggc ccctggagtc 1800

<210> 64
<211> 27
<212> PRT
<213> Mus musculus

<220>
<221> SITE
<222> (1)..(27)
<223> PDI a

<400> 64

Ala Pro Trp Cys Gly His Cys Lys Ala Leu Arg Leu Ala Lys Val Asp
1 5 10 15

Ala Thr Glu Glu Ser Asp Leu Ala Gln Gln Tyr
20 25

<210> 65
<211> 27
<212> PRT
<213> Mus musculus

<220>
<221> SITE
<222> (1)..(27)
<223> PDI a'

<400> 65

Ala Pro Trp Cys Gly His Cys Lys Gln Leu Ile Ile Ala Lys Met Asp
1 5 10 15

Ser Thr Ala Asn Glu Val Glu Ala Val Lys Val
20 25

<210> 66
<211> 27
<212> PRT
<213> Mus musculus

<220>
<221> SITE
<222> (1)..(27)
<223> ERP57 a

<400> 66

Ala Pro Trp Cys Gly His Cys Lys Arg Leu Pro Leu Ala Lys Val Asp
1 5 10 15

Ser Thr Ala Asn Thr Asn Thr Cys Asn Lys Tyr
20 25

<210> 67
<211> 27
<212> PRT
<213> Mus musculus

<220>
<221> SITE
<222> (1)..(27)
<223> ERP57 a'

<400> 67

Ala Pro Trp Cys Gly His Cys Lys Asn Leu Val Ile Ala Lys Met Asp
1 5 10 15

Ala Thr Ala Asn Asp Val Pro Ser Pro Tyr Glu
20 25

<210> 68
<211> 27
<212> PRT
<213> Mus musculus

<220>
<221> SITE
<222> (1)..(27)
<223> ERP43 a

<400> 68

Ala Pro Trp Cys Gly His Cys Gln Arg Leu Tyr Val Ala Lys Val Asp
1 5 10 15

Cys Thr Ala Asp Ser Asp Val Cys Ser Ala Gln
20 25

<210> 69
<211> 27
<212> PRT
<213> Mus musculus

<220>
<221> SITE
<222> (1)..(27)
<223> ERP43 a'

<400> 69

Ala Pro Trp Cys Gly His Cys Lys Ala Leu Lys Ile Gly Lys Val Asp
1 5 10 15

Cys Thr Gln His Tyr Ala Val Cys Ser Glu His
20 25

<210> 70
<211> 27
<212> PRT
<213> Mus musculus

<220>
<221> SITE
<222> (1)..(27)
<223> ERP43 a''

<400> 70

Ala Pro Trp Cys Gly His Cys Lys Asn Leu Thr Ile Ala Glu Val Asp
1               5                   10                  15

Cys Thr Ala Glu Arg Asn Val Cys Ser Lys Tyr
            20                  25

<210> 71
<211> 27
<212> PRT
<213> Mus musculus

<220>
<221> SITE
<222> (1)..(27)
<223> ERP44 a

<400> 71

Ala Asp Trp Cys Arg Phe Ser Gln Met Leu Val Phe Ala Arg Val Asp
1               5                   10                  15

Cys Asp Gln His Ser Asp Ile Ala Gln Arg Tyr
            20                  25

<210> 72
<211> 27
<212> PRT
<213> Saccharomyces cerevisiae

<220>
<221> SITE
<222> (1)..(27)
<223> Pdil a

<400> 72

Ala Pro Trp Cys Gly His Cys Lys Asn Met Thr Leu Ala Gln Ile Asp
1               5                   10                  15

Cys Thr Glu Asn Gln Asp Leu Cys Met Glu His
            20                  25

<210> 73
<211> 27
<212> PRT
<213> Saccharomyces cerevisiae

<220>
<221> SITE
<222> (1)..(27)
<223> Pdil a'

<400> 73

Ala Pro Trp Cys Gly His Cys Lys Arg Leu Leu Ile Ala Lys Leu Asp
1               5                   10                  15

```
His Thr Glu Asn Asp Val Arg Gly Val Val Ile
            20                  25


<210>  74
<211>  27
<212>  PRT
<213>  Saccharomyces cerevisiae

<220>
<221>  SITE
<222>  (1)..(27)
<223>  Eugl a


<400>  74

Ala Pro Trp Cys Leu His Ser Gln Ile Leu Pro Val Val Gln Ile Asp
1               5                   10                  15


Cys Glu Ala Asn Ser Met Val Cys Leu Gln Gln
            20                  25


<210>  75
<211>  27
<212>  PRT
<213>  Saccharomyces cerevisiae

<220>
<221>  SITE
<222>  (1)..(27)
<223>  Eugl a'


<400>  75

Ala Thr Trp Cys Ile His Ser Lys Arg Phe Leu Ile Ala Glu Val Asp
1               5                   10                  15


Ser Gly Ala Asn Asp Ile Leu Ser Phe Pro Val
            20                  25
```